(19) Europäisches Patentamt
European Patent Office
Office européen des brevets

(11) Veröffentlichungsnummer: **0 121 753**
**A2**

(12) # EUROPÄISCHE PATENTANMELDUNG

(21) Anmeldenummer: **84102344.3**

(22) Anmeldetag: **05.03.84**

(51) Int. Cl.³: **C 07 D 405/14**, C 07 D 409/14, C 07 D 495/10, C 07 D 491/10, C 07 D 471/10, A 61 K 31/475 // C07D405/12, C07D217/00, C07D221/20, C07D409/04, C07D409/06, C07D405/06, C07D401/06

(30) Priorität: **10.03.83 DE 3308554**

(43) Veröffentlichungstag der Anmeldung: **17.10.84 Patentblatt 84/42**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Schaper, Wolfgang, Dr., Rauenthaler Weg 18, D-6000 Frankfurt am Main 71 (DE)**
Erfinder: **Blume, Ernst, Dr., Rossertstrasse 20, D-6239 Kriftel (DE)**
Erfinder: **Raether, Wolfgang, Dr., Falkensteinstrasse 6, D-6072 Dreieich (DE)**
Erfinder: **Dittmar, Walter, Dr., Uhlandstrasse 10, D-6238 Hofheim am Taunus (DE)**

(54) **1-(1,3-Dioxolan-2-ylmethyl)-azole, ihre Salze, Verfahren zu ihrer Herstellung und sie enthaltende pharmazeutische Zubereitungen.**

(57) Es werden neue 1-(1,3-Dioxolan-2-ylmethyl)-azole, ihre Steroisomeren und ihre Salze beschrieben, sowie Verfahren zu ihrer Herstellung. Die neuen Verbindungen sind Chemotherapeutika. Sie wirken gegen Hautpilze, Schimmelpilze und Hefen sowie gegen Bakterien.

EP 0 121 753 A2

1-(1,3-Dioxolan-2-ylmethyl)-azole, ihre Salze, Verfahren zu ihrer Herstellung und sie enthaltende pharmazeutische Zubereitungen

Die Erfindung betrifft den in den Ansprüchen gekennzeichneten Gegenstand.

Nach US-A 3 936 470, Fr-A 2 438 046, den DE-A 2 803 870, 2 804 096, sowie den EP-A 0 006 712, 0 006 722, 0 050 298 und 0 052 905 sind 1-(1-3-Dioxolan-2-ylmethyl)-1H-imidazole und 1H-1,2,4-triazole zur Bekämpfung von Pilzen und Bakterien bekannt; ihre Wirkung und Verträglichkeit sind jedoch nicht immer ganz befriedigend. Von diesen Verbindungen unterscheiden sich die erfindungsgemäßen Verbindungen wesentlich durch die Art der Substituenten in der 4-Stellung der Dioxolangruppe und durch eine bessere und breitere antimykotische Wirksamkeit und eine bessere Verträglichkeit.

Im folgenden ist unter dem Ausdruck
"$C_1$-$C_4$-Alkyl" ein unverzweigter oder verzweigter Kohlenwasserstoffrest mit 1 - 4 Kohlenstoffatomen, wie z.B. der Methyl-, Ethyl-, Propyl-, 1-Methylethyl-, 2-Methylpropyl- oder 1,1-Dimethylethylrest,
unter dem Ausdruck
"$C_1$-$C_{12}$-Alkyl" ein unverzweigter oder verzweigter Kohlenwasserstoffrest mit 1 - 12 Kohlenstoffatomen, wie z.B. die vorstehend genannten Reste oder der Pentyl-, Hexyl-, Heptyl-, Octyl-, Nonyl-, Decyl-, Undecyl-, Dodecyl- oder 1,1-Dimethyl-3,3-dimethyl-butyl-Rest,
unter dem Ausdruck
"$C_3$-$C_8$-Cycloalkyl" ein unverzweigter cyclischer Kohlenwasserstoffrest mit 3 - 8 Kohlenstoffatomen, wie z.B. der Cyclopropyl-, Cyclopentyl-, Cyclohexyl-, Cycloheptyl- oder Cyclooctyl-Rest,
unter dem Ausdruck

"$C_1$-$C_4$-Alkoxy" eine Alkoxygruppe, deren Kohlenwasserstoffrest die unter dem Ausdruck "$C_1$-$C_4$-Alkyl" angegebene Bedeutung hat,
unter dem Ausdruck
"$C_1$-$C_4$"-Dialkylamino" eine Dialkylaminogruppe mit gleichen oder verschiedenen Kohlenwasserstoffresten, wobei diese die unter dem Ausdruck "$C_1$-$C_4$"-Alkyl angegebene Bedeutung haben, oder auch cyclische Systeme wie Pyrrolidin, Piperdin oder Hexamethylenimin,
unter dem Ausdruck
"$C_1$-$C_5$-Alkanoyl" ein unverzweigter oder verzweigter Alkanoylrest mit 1 - 5 Kohlenstoffatomen, wie die Formyl-, Acetyl-, Propanoyl-, 2-Methylpropanoyl-, Butanoyl-, Pentanoyl- und 2,2-Dimethylpropanoyl-Gruppe,
unter dem Ausdruck
"$C_3$-$C_5$-Alkenyl ein verzweigter oder unverzweigter Alkenrest mit 3 - 5 Kohlenstoffatomen, wie z.B. die 2-Propenyl-, 2-Butenyl- oder die 2-Pentenyl-Gruppe,
unter dem Ausdruck
"$C_3$-$C_5$-Alkinyl" ein unverzweigter oder verzweigter Alkinrest mit 3 - 5 Kohlenstoffatomen, wie z.B. die 2-Propinyl-, 2-Butinyl- oder die 2-Pentinyl-Gruppe,
unter dem Ausdruck
"$C_1$-$C_4$-Alkylthio" eine Alkylthiogruppe, deren Kohlenwasserstoffrest die unter dem Ausdruck "$C_1$-$C_4$-Alkyl" angegebene Bedeutung hat,
unter dem Ausdruck
"Arylgruppe" die Phenyl-, Naphtyl- oder Biphenyl-Gruppe,
unter dem Ausdruck
"Heteroaryl-Gruppe" ein heteroaromatisches System mit 5 oder 6 Ringgliedern, bevorzugt Thiophen, Furan oder Pyridin,
unter den Ausdrücken
"$C_1$-$C_5$-Arylakyl-Gruppe" und "$C_1$-$C_5$-Heteroaryl-alkyl-Gruppe" ein verzweigter oder bevorzugt unverzweigter Arylalkyl- oder Heteroarylalkylrest mit 1 - 5 Kohlenstoffatomen im Alkylteil, wobei "Aryl" und "Heteroaryl" die oben angegebenen Bedeutungen haben, wie z.B. die Benzyl-, 2-Phenylethyl-, 3-Phenylpropyl-, 4-Phenylbutyl-, 5-Phenylpentyl-, 2-Thienylmethyl-, 3-Thienylmethyl-, 4-Pyridylmethyl-, 2-(2-Thienyl)-ethyl-, 3-(2-Thienyl)-propyl-, 2-(3-Thienyl-ethyl-, 4-Biphenylmethyl-, 2-(4-Biphenyl)-ethyl, 1-Naphtylmethyl-, 2-Naphthylmethyl-Gruppe,
unter dem Ausdruck
"Halogen" ein Fluor-, Chlor-, Brom- oder Jodatom zu verstehen.

Gegenstand der Erfindung sind 1-(1,3-Dioxolan-2-ylmethyl)-azole der allgemeinen Formel (I)

$$G-CH_2-O-D \quad (I)$$

sowie ihre Steroisomeren und ihre Salze mit physiologisch verträglichen Säuren,
in welcher
G einen 1-(1,3-Dioxolan-2-ylmethyl)-azol-Rest der nachstehenden Struktur bedeutet,

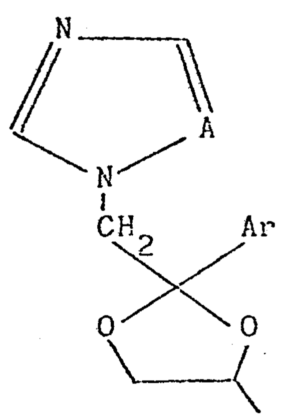

(G)

in welcher A Stickstoff oder Methin,
Ar Naphtyl, Biphenyl, Thienyl oder eine gegebenenfalls einen, zwei oder drei Substituenten tragende Phenylgruppe bedeutet, wobei die Substituenten gleich oder verschieden sind und Halogen, Trifluormethyl, $C_1$-$C_4$-Alkyl oder $C_1$-$C_4$-Alkoxy bedeuten,
und D in Formel (I) entweder

1.1. eine Isochinolingruppe der Formel D I bedeutet

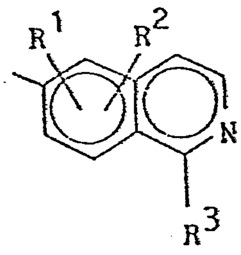

(D I)

wobei $R^1$ und $R^2$ unabhängig voneinander ein Wasserstoffatom, Halogen, $C_1$-$C_4$-Alkyl oder $C_1$-$C_4$-Alkoxy bedeuten, und $R^3$ ein Wasserstoffatom $C_1$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, Heteroaryl, $C_1$-$C_5$-Heteroaryl-alkyl oder eine gegebenenfalls eine einen, zwei oder drei Substituenten tragende Aryl- oder $C_1$-$C_5$-Aryl-alkyl-Gruppe bedeutet, wobei die Substituenten an der Arylgruppe gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten, oder

1.2. eine 3,4-Dihydro-isochinolingruppe der Formel D II bedeutet

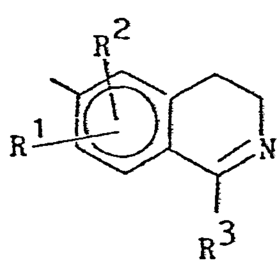

(D II)

wobei $R^1$, $R^2$ und $R^3$ die zu Formel DI angegebenen Bedeutungen haben, oder

1.3. eine 1,2,3,4-Tetrahydro-isochinolingruppe der Formel D III bedeutet

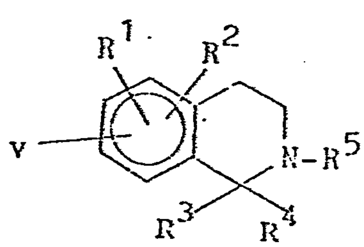

(D III)

wobei v die Position der Verknüpfung des Isochinolinsystems mit dem Rest $G-CH_2-O$ angibt, $R^1$, $R^2$ und $R^3$ die zur Formel D I angegebenen Bedeutungen haben und $R^4$ ein Wasserstoffatom oder eine $C_1-C_4$-Alkyl-Gruppe bedeutet, sowie zusätzlich, falls $R^3$ und $R^4$ Alkylgruppen sind, diese zusammen mit dem sie tragenden C-Atom ein spirocyclisches Ringsystem der allgemeinen Formel D III' bedeuten können,

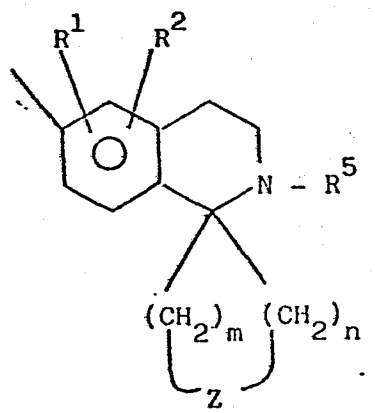

D III'

worin m und n 1, 2 oder 3 bedeuten und Z entweder eine Methylengruppe bedeutet oder, falls m = n = 2 ist, ein Heteroatom aus der Gruppe Sauerstoff, Schwefel oder Stickstoff bedeutet, wobei der Stickstoff durch einen Rest $R^6$ substituiert sein kann, der $C_1-C_4$-Alkyl, $C_1-C_5$-Alkanoyl, $C_1-C_4$-Alkoxycarbonyl oder eine gegebenenfalls mit einem oder zwei Substituenten versehene Benzoyl- oder Benzylgruppe bedeutet, wobei die Substituenten am Phenylring gleich oder verschieden sein können und jeweils Halogen, $C_1-C_4$-Alkyl, Trifluormethyl oder $C_1-C_4$-Alkoxy bedeuten, und v, falls $R^3$ und/oder $R^4$ kein Wasserstoffatom ist, die 6-Stellung bedeutet oder, falls $R^3$ und $R^4$ Wasserstoffatome sind, die 5-, 6-, 7- oder 8-Stellung bedeutet, und $R^5$, falls $R^3$ und/oder $R^4$ kein Wasserstoffatom ist,

ein Wasserstoffatom oder eine $C_1$-$C_4$-Alkylgruppe bedeutet oder, falls $R^3$ und $R^4$ Wasserstoffatome sind und v die 5-, 7- oder 8-Stellung bedeutet, $R^5$ die zur Formel D I für $R^3$ angegebenen Bedeutungen haben kann, mit der Einschränkung, daß $R^5$ keine Aryl- oder Heteroaryl-Gruppe bedeutet, oder, falls $R^3$ und $R^4$ Wasserstoffatome sind und v die 6-Stellung bedeutet, $R^5$ die zur Formel D I für $R^3$ angegebenen Bedeutungen hat, und $R^5$ weiterhin eine Acylgruppe der allgemeinen Formel Q bedeuten kann,

$$-\underset{\underset{O}{\|}}{C}-R^7 \qquad Q$$

wobei $R^7$ die zur Formel D I für $R^3$ angegebenen Bedeutungen haben kann.

Von Verbindungen der Formel (I) sind solche bevorzugt, für die in dieser Formel Ar eine mit 1 oder 2 Halogenatomen, vorzugsweise Fluor, Chlor oder Brom substituierte Phenylgruppe, eine 4-Trifluormethyl-, 4-Methyl-, 4-Methoxyhenyl- oder eine Biphenylgruppe bedeutet, $R_1$ und $R_2$ Wasserstoffatome sind und die Verknüpfung zwischen Dioxolan- und Isochinolinteil in Formel (I) vorzugsweise an $C_6$ oder $C_7$ des Isochinolinteils erfolgt.

Stärker bevorzugt sind Verbindungen der allgemeinen Formel (I) in denen Ar die 4-Chlorphenyl- oder die 2,4-Dichlorphenylgruppe bedeutet, $R_1$ und $R_2$ Wasserstoffatome bedeuten, die Verknüpfung zwischen Dioxolan- und Isochinolinteil an $C_6$ oder $C_7$ des Isochinolinteils erfolgt und es sich bei dem Rest D in Formel (I) um ein Tetrahydroisochinolinderivat der allgemeinen Formeln (D III) handelt.

Am stärksten bevorzugt sind Verbindungen der allgemeinen Formel (I), in denen A eine Methingruppe, Ar die 2,4-Dichlorphenylgruppe bedeutet und in denen es sich bei dem Rest D um ein Tetrahydroisochinolin der allgemeinen Formel (D III) handelt in dem $R_1$, $R_2$, $R_3$ und $R_4$ Wasserstoffatome bedeuten und $R_5$ eine $C_1$-$C_4$-Arylalkyl-Gruppe bedeutet und das mit dem Dioxolan-Rest in Formel (I) über $C_6$ oder $C_7$ des Isochinolin-Systems verbunden ist.

Gegenstand der Erfindung ist auch ein Verfahren zur Herstellung der Verbindungen der allgemeinen Formel (I) in Anspruch 1, dadurch gekennzeichnet, daß man

a) eine Verbindung der allgemeinen Formel (II)

$$G - CH_2 - E \qquad (II)$$

in der E Halogen oder einen reaktiven Esterrest bedeutet, mit einer Verbindung der allgemeinen Formel (III)

$$H - O - D \qquad (III)$$

umsetzt, in der D die in Anspruch 1 angegebene Bedeutung hat, und gegebenenfalls eine so erhaltene Verbindung der Formel (I) acyliert, alkyliert oder reduziert, oder

b) eine Verbindung der allgemeinen Formel (IV)

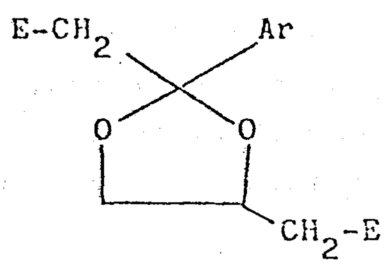

(IV)

in der Ar die in Anspruch 1 zu Formel I und E die zur Formel (II) angegebenen Bedeutungen haben, zunächst mit einer Verbindung der allgemeinen Formel III umsetzt und hierbei eine Verbindung der allgemeinen Formel (V) herstellt,

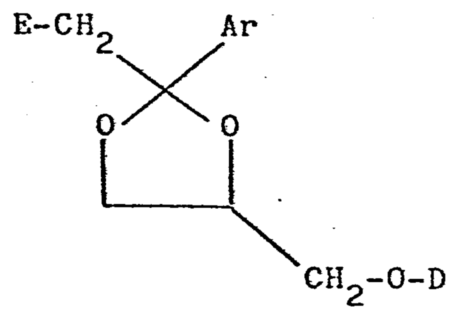

(V)

in der Ar und D die in Anspruch 1 und E die zur Formel (II) in Anspruch 2 angegebenen Bedeutungen haben, und anschließend die so erhaltene Verbindung der allgemeinen Formel (V) mit einer Verbindung der allgemeinen Formel (VI) umsetzt,

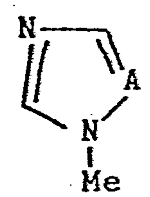

(VI)

in der A die in Anspruch 1 angegebenen Bedeutungen hat und Me Wasserstoff oder ein Metallatom bedeutet,
und gegebenenfalls eine so erhaltene Verbindung der Formel (I) acyliert, alkyliert oder reduziert
und gegebenenfalls eine nach a) oder b) erhaltene Verbindung mit einer physiologisch verträglichen Säure in das Salz überführt.

Die Verbindungen der allgemeinen Formel (I) werden nach Verfahrensvariante a) gemäß nachfolgender Formelgleichung durch Umsetzung eines entsprechend substituierten Isochinolins der allgemeinen Formel (III) mit einer Verbindung der allgemeinen Formel (II) hergestellt

$$G\text{-}CH_2\text{-}E + HOD \quad (I)$$
(II) (III)

wobei in der vorstehenden allgemeinen Formel (II) G die zur Formel (I) in Anspruch 1 angegebene Bedeutung hat und E eine reaktive Abgangsgruppe wie Chlor, Brom, Jod oder Trifluoracetyloxy oder eine Sulfonyloxygruppe wie z.B. Methyl-, 4-Methylphenyl-, 4-Chlorphenyl- oder Trifluormethylsulfonyloxy bedeutet und D die zu Formel I angegebenen Bedeutungen besitzt.

Die vorgenannte Umsetzung wird in einem Temperaturbereich von 30 bis 150°C, vorzugsweise 40 bis 100°C, zweckmäßig in Anwesenheit einer Base und in einem inerten organischen Lösungsmittel, wie N,N-Dimethylformamid, N,N-Dimethylacetamid, Dimethylsulfoxid, N-Methylpyrrolidon-2-, Dioxan, Tetrahydrofuran, 4-Methyl-2-pentanon, Methanol, Ethanol, Isopropylalkohol, Propanol, Butanol, Pentanol Tert.-butylalkohol, Methylglykol, Methylenchlorid oder einem aromatischen Kohlenwasserstoff wie Benzol, Chlorbenzol, Toluol oder Xylol oder in Wasser durchgeführt. Es können auch Gemische der beispielhaft genannten Lösungsmittel verwendet werden.

Geeignete Basen sind beispielsweise Alkalimetall- oder Erdalkalimetall-carbonate, -hydrogencarbonate, -hydroxide, -alkoholate oder -hydride wie z.B. Natriumcarbonat, Natriumhydrogencarbonat, Kaliumcarbonat, Natriumhydroxid, Natriummethylat oder Natriumhydrid.

Die Umsetzung erfolgt ebenso unter den Bedingungen einer Phasentransferreaktion, indem man die Reaktionspartner in einem aromatischen Kohlenwasserstoff wie Benzol, Chlorbenzol, Toluol oder Xylol unter kräftigem Rühren in Gegenwart eines Phasentransferkatalysators und entweder einem gepulverten Alkalihydroxid, wie z.B. Natrium- oder Kaliumhydroxid oder einer konzentrierten wäßrigen Lösung derselben, vorzugsweise in einem Temperaturbereich von 20 bis 120°C, aufeinander einwirken läßt.

Geeignete Phasentransferkatalysatoren sind z.B. Trialkylbenzylmmonium- oder Tetraalkylammonium-halogenide, -hydroxide, -hydrogensulfate mit vorzugsweise 1 bis 12 C-Atomen im Alkylrest oder Kronenether, wie z.B. 12-Crown-4-, 15-Crown-5, 18-Crown-6 oder Dibenzo-18-crown-6.

Solche Verbindungen der allgemeinen Formel (I), für die der Rest D den Rest D III bedeutet, können auch hergestellt werden, indem man zunächst eine Verbindung der allgemeinen Formel (II) mit einer Verbindung der allgemeinen Formel (III a) umsetzt, in der $R_1$, $R_2$, $R_3$ und $R_4$ die in Anspruch 1 angegebenen Bedeutungen haben und in der anstelle von $R_5$ der Formel D III in Anspruch 1.3 ein Wasserstoffatom steht, die so erhaltene Verbindung der allgemeinen Formel (I a), in der $R_1$, $R_2$, $R_3$ und $R_4$ die oben angegebenen Bedeutungen haben,

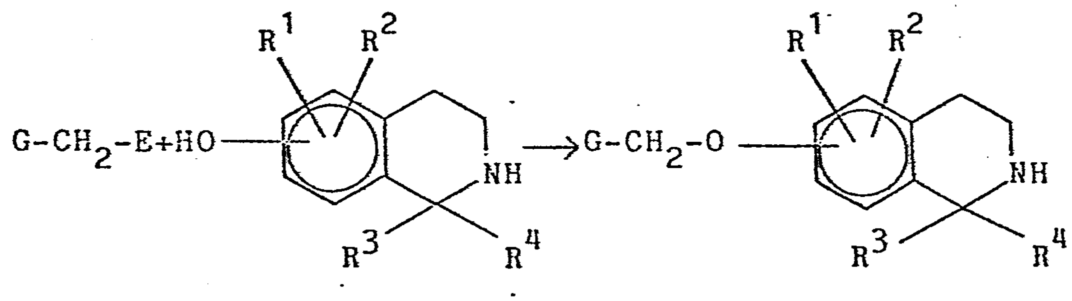

(II) (III a) (Ia)

mit einem Alkylierungsmittel Alk-E zu einer Verbindung der allgemeinen Formel (I b) alkyliert, oder mit einem Acylierungsmittel $R^7$-CO-X zu einer Verbindung der allgemeinen Formel (I c) acyliert und (I c) gegebenenfalls zu (I b) reduziert,

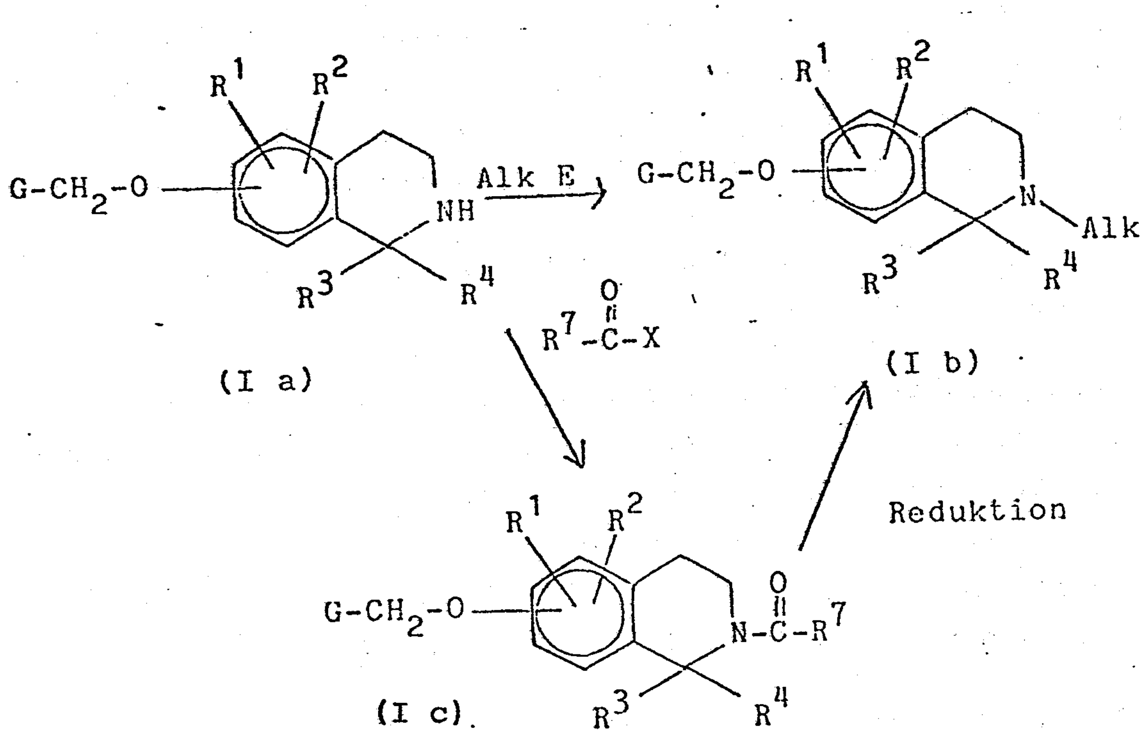


wobei Alk $C_1$-$C_{12}$-Alkyl, $C_3$-$C_8$-Cycloalkyl, $C_3$-$C_5$-Alkenyl, $C_3$-$C_5$-Alkinyl, $C_1$-$C_5$-Heteroaryl-alkyl oder $C_1$-$C_5$-Arylalkyl bedeutet, wobei die Arylgruppe einen, zwei oder drei Substituenten tragen kann und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten, und E eine reaktive Abgangsgruppe wie Chlor, Brom, Jod, Trifluoracetyloxy oder eine Sulfonyloxygruppe wie Methyl-, Phenyl-, 4-Methylphenyl-, 4-Chlorphenyl- oder Trifluormethylsulfonyloxy bedeutet, $R^7$ die zur Formel I angegebenen Bedeutungen hat und X Chlor, Brom Jod, $C_1$-$C_4$-Alkoxy, Aryloxy oder Aryl-$C_1$-$C_4$-Alkoxy oder eine Gruppe der Formel $R_7-\overset{O}{\overset{\|}{C}}-O-$ bedeutet.

Zur Umsetzung der Verbindungen (II) und (III a) wird zweckmäßig eine Verbindung (III a) in einem inerten dipolaren aprotischen organischen Lösungsmittel wie Dimethylformamid,

Dimethylacetamid, Tetramethylharnstoff, Sulfolan oder Dimethylsulfoxid mit einer geeigneten Base versetzt und anschließend mit einer Verbindung (II) bei einer Temperatur zwischen 20 und 120°C ein bis mehrere Stunden lang gerührt.

Geeignete Basen sind hier z.B. Alkali- oder Erdalkalimetallamide oder -hydride.

Die anschließende Alkylierung der Verbindungen der allgemeinen Formel (I a) mit Alk-E erfolgt zweckmäßig in einem inerten organischen Lösungsmittel in Gegenwart einer geeigneten Base, bei einer Temperatur zwischen 20 und 120°C. Geeignete Basen sind hier z.B. Alkali- oder Erdalkalimetallcarbonate oder -Hydrogencarbonate.

Die vorstehend beschriebenen stufenweise durchgeführten Alkylierungen der OH- und anschließend der NH-Gruppe werden zweckmäßig als Eintopfreaktion durchgeführt, indem man zunächst bei 20 bis 120°C eine Verbindung der allgemeinen Formel (III a) mit einer Verbindung der allgemeinen Formel (II) in Gegenwart eines Äquivalents einer zum ersten Alkylierungsschritt vorstehend genannten Base in einer der genannten inerten Lösungsmittel umsetzt und anschließend ohne Isolierung der Verbindung (I a) das Alkylierungsmittel Alk-E und ein Äquivalent einer zum zweiten Alkylierungsschritt genannten Base zugibt und erneut auf eine Temperatur zwischen 20 und 120°C erhitzt.

Die Überführung von Verbindungen der allgemeinen Formel (I a) in Alkylierungsprodukte der allgemeinen Formel (I b) kann je nach Zugänglichkeit der Ausgangsstoffe auch durch Umsetzung einer Verbindungen (I a) mit einer Carbonylverbindung in Gegenwart eines Reduktionsmittels in einem inerten Lösungsmittel erfolgen (reduktive Aminierung).

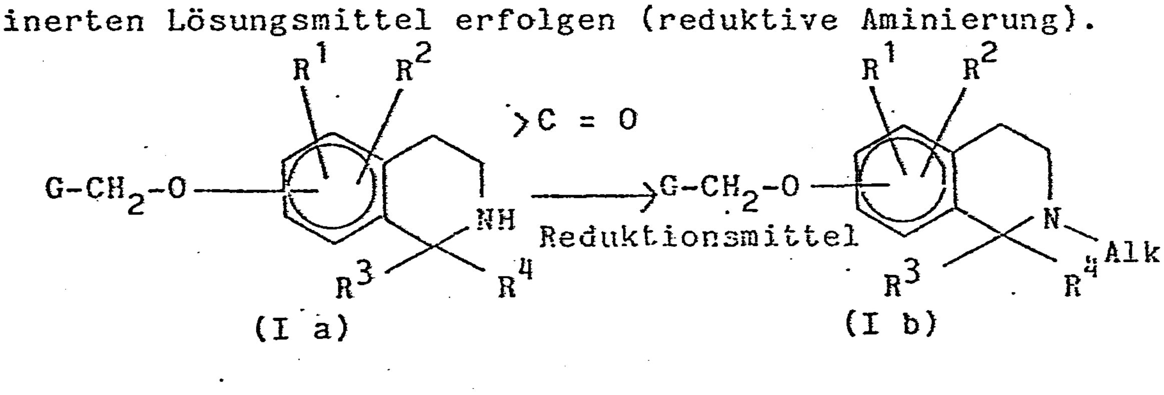


(I a) (I b)

Als Kombination von Carbonylverbindung und Reduktionsmittel kommen hierfür beispielsweise in Frage Aldehyde oder Ketone und Wasserstoff in Gegenwart eines Metallkatalysators wie z.B. Raney-Nickel unter erhöhtem Druck (Organikum, VEB Deutscher Verlag der Wissenschaften, Berlin 1977, S. 542); Aldehyde oder Ketone und Natriumcyanoborhydrid ( J. Amer. Chem. Soc. 93, 2897, (1971); oder Carbonsäuren und Natriumborhydrid (J. Org. Chem. 40, 3453 (1975) und Synthesis 1975), 650).

Zur Herstellung der Verbindungen der Formel (I c) wird eine Verbindung der allgemeinen Formel (I a) mit einem Acylierungsmittel der Formel $R^7-\underset{O}{\underset{\|}{C}}-X$ in einem inerten Lösungsmittel wie Methylenchlorid, Chloroform, Benzol, Toluol oder Pyridin umgesetzt, zweckmäßig in Gegenwart einer Base wie Alkali- oder Erdalkalimetell-carbonat oder -hydrogencarbonat, z.B. Natriumcarbonat, Natriumhydrogencarbonat oder Kaliumcarbonat oder einer organischen Base, z.B. Triethylamin, Tributylamin, N-Ethylmorpholin, Pyridin, Chinolin oder 1H-Imidazol.

Die Reduktion der Verbindungen der allgemeinen Formel (I c) zu Verbindungen der allgemeinen Formel (I b) erfolgt mit einem komplexen Metallhydrid wie Lithiumaluminiumhydrid, Diisobutylaluminiumhydrid oder Natrium-bis-(2-methoxyethoxy)-aluminiumdihydrid in einem inerten Lösungsmittel wie Diethylether, Tetrahydrofuran, Dioxan oder Dimethoxyethan in einem Temperaturbereich von 0 - 50°C.

Zur Durchführung des Verfahrens nach Verfahrensvariante b) wird eine Verbindung der allgemeinen Formel (IV)

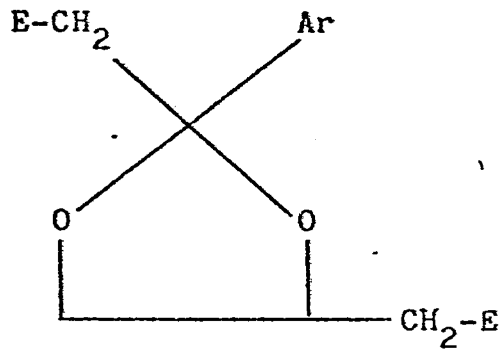

(IV)

in der Ar die in Anspruch 1 und E jeweils eine oder verschiedene der zur Formel (II) für E angegebenen Bedeutungen haben, zunächst mit einer Verbindung der allgemeinen Formel (III) in Anspruch 2 umgesetzt und hierbei eine Verbindung der allgemeinen Formel (V) hergestellt, in der Ar und D die in Anspruch 1 und E die zur Formel (II) in Anspruch 2 angegebenen Bedeutungen haben.

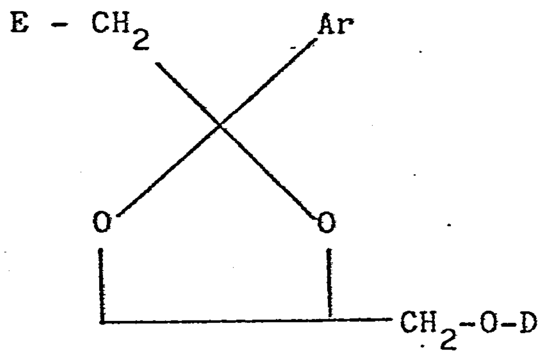

(V)

Die Reaktionsbedingungen zur Herstellung der Verbindungen der allgemeinen Formel (V) durch Umsetzung von Verbindungen der allgemeinen Formel(IV) mit Verbindungen der allgemeinen Formel (III), sind die gleichen, wie bei Verfahrensvariante a) zur Umsetzung von Verbindungen der allgemeinen Formel (II) mit (III) angegeben.

Anschließend wird die so erhaltene Verbindung der allgemeinen Formel (V) mit einer Verbindung der allgemeinen Formel (VI) umgesetzt,

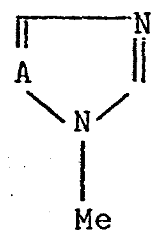

(VI)

in der A die in Anspruch 1 angegebene Bedeutung hat und Me Wasserstoff oder ein Metallatom, bevorzugt Alkali- oder Erdalkali-Metallatom, bedeutet.

Die Herstellung von Verbindungen der allgemeinen Formel (I) nach Verfahrensvariante b) durch Umsetzung einer Verbindung der allgemeinen Formel (V) mit einer Verbindung der allgemeinen Formel (VI) erfolgt zweckmäßig in einem inerten Lösungsmittel und gegebenenfalls in Gegenwart einer Base, wie zum ersten Herstellungsverfahren bei der Umsetzung von (II) mit (III) vorstehend angegeben vorzugsweise bei einer Temperatur zwischen 100 und 190°C. Die Umsetzung erfolgt gegebenenfalls in einem Autoklaven unter Druck.

Die vorstehend beschriebenen Umsetzungen von (III) mit (IV) zu (V) und anschließend mit (VI) zu (I) werden zweckmäßig als Eintopfreaktion durchgeführt, indem man zunächst bei 30 bis 150°C eine Verbindung der allgemeinen Formel (IV) mit einer Verbindung der allgemeinen Formel (III) in Gegenwart eines Äquivalents einer Base in einem inerten Lösungsmittel umsetzt. Anschließend wird ohne Isolierung der Verbindung der allgemeinen Formel (V) eine Verbindung der allgemeinen Formel (VI) und ein weiteres Äquivalent einer Base zugegeben und auf 100 bis 190°C erhitzt.

Gegenstand der Erfindung sind ferner die als Ausgangsmaterialien in den vorstehend beschriebenen Verfahren benutzten Verbindungen der allgemeinen Formel (III')

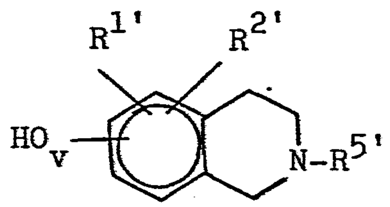

(III')

wobei v die Position der Hydroxygruppe am Isochinolinsystem angibt und die 5-, 6-, 7- oder 8-Position bedeutet, worin

$R^{1'}$ und $R^{2'}$ unabhänig voneinander ein Wasserstoffatom, Halogen oder $C_1$-$C_4$-Alkyl bedeuten,

und

$R^{5'}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl, $C_1$-$C_5$-Arylalkyl oder falls v die 6-Position bedeutet, zusätzlich Aryl oder Heteroaryl bedeutet, wobei bei den Aryl- oder $C_1$-$C_5$-Arylalkyl-Gruppen die Arylgruppen einen, zwei oder drei Substituenten tragen können und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten,

und worin

$R^{5'}$ weiterhin eine Acylgruppe der allgemeinen Formel Q' bedeutet

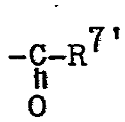

(Q')

worin $R^{7'}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl, $C_1$-$C_5$-Arylalkyl, Aryl oder Heteroaryl bedeutet, wobei bei den Aryl- oder $C_1$-$C_5$-Arylalkyl-Gruppen

die Arylgruppen einen, zwei oder drei Substituenten tragen können und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten.

Gegenstand der Erfindung ist weiterhin ein Verfahren zur Herstellung von Verbindungen der allgemeinen Formel (III'a)

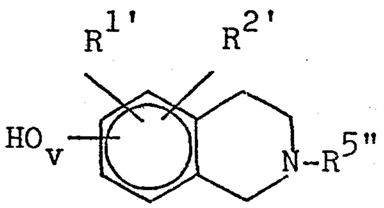

(III'a)

für die $R^{1'}$, $R^{2'}$ und v die der Formel (III') angegebenen Bedeutungen haben und $R^{5''}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl oder $C_1$-$C_5$-Arylalkyl bedeutet, wobei die Arylgruppe einen, zwei oder drei Substituenten tragen kann und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten, dadurch gekennzeichnet, daß man eine Verbindung der allgemeinen Formel (III'g)

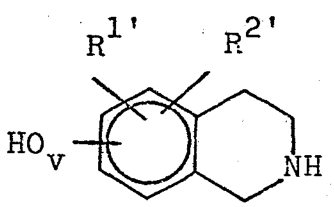

(III'g)

oder eine Verbindung der allgemeinen Formel (X)

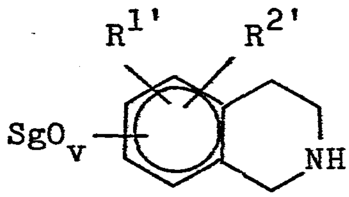

(X)

in denen v, $R^{1'}$ und $R^{2'}$ die oben angegebenen Bedeutungen haben und Sg eine Schutzgruppe wie eine $C_1$-$C_4$-Alkylgruppe, eine Benzylgruppe oder ein Gruppe der allgemeinen Formel $R^7-\underset{\underset{O}{\|}}{C}-$ bedeutet,

wobei $R^7$ die zur Formel DIII angegebenen Bedeutungen hat, mit einem Alkylierungsmittel Alk'E umsetzt, wobei Alk' $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroarylalkyl oder $C_1$-$C_5$-Arylalkyl bedeutet, wobei die Arylgruppe einen, zwei oder drei Substituenten tragen kann und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten,

und E eine reaktive Abgangsgruppe mit den oben angegebenen Bedeutungen ist, und bei den aus der Verbindung der Formel (X) erhaltenen Verbindungen die Schutzgruppe Sg abspaltet.

Die Umsetzung wird unter den Bedingungen einer N-Alkylierung durchgeführt indem man eine Verbindung (III'g) oder (X) mit dem Alkylierungsmittel Alk-E in Gegenwart einer geeigneten Base, vorzugsweise eines Alkali- oder Erdalkalimetallcarbonats oder -Hydrogencarbonats in einem inerten Lösungsmittel wie Acetonitril, Dimethylformamid, Dimethylacetamid, Dimethylsulfoxid, Tetramethylharnstoff oder Sulfolan bei einer Temperatur zwischen 20 und 120°C umsetzt.

Zur Entfernung der verschiedenen Schutzgruppen Sg aus den Zwischenprodukten der allgemeinen Formel (X')

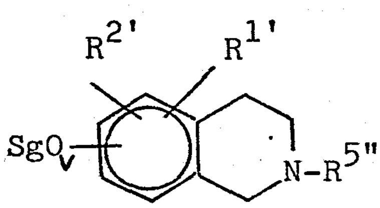

(X')

worin $R^{1'}$, $R^{2'}$, $R^{5'}$ und Sg die oben angegebenen Bedeutungen haben, steht eine Vielzahl von Methoden zur Verfügung (vgl. T.W. Greene, Protective Groups in Organic Synthesis, New York 1981, S. 87 ff.). Die Spaltung der Methylether erfolgt vorzugweise durch Erhitzen mit starken Säuren wie z.B. Bromwasserstoffsäure, die Benzylether werden bevorzugt hydrogenolytiscch in Gegenwart von Edelmetallkatalysatoren wie z.B. Palladium oder Platin gespalten. Die Abspaltung der Acyl-Schutzgruppe erfolgt vorzugsweise durch Hydrolyse mit wäßriger Natron- oder Kalilauge oder durch Erhitzen mit einem Dialkylamin, z.B. Diethylamin in einem inerten Lösungsmittel, z.B. Ethanol ., erfolgen. Die für die oben beschriebenen Synthesen benötigten Tetrahydroisochinoline der allgemeinen Formel (III'g) und (X) sind bekannt (vgl. z.B. J. Heterocyclic Chem. <u>8</u>, 665 (1971) und dort zitierte Literatur) oder können in Analogie zu bekannten Vorschriften hergestellt werden.

Gegenstand der Erfindung ist weiterhin ein

Verfahren zur Herstellung von Verbindungen der allgemeinen Formel (III'b)

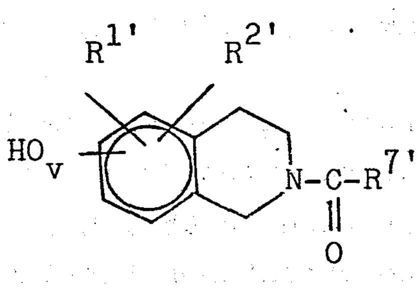

(III'b)

worin $R^{1'}$, $R^{2'}$, v und $R^{7'}$ die zur Formel (III') angegebenen Bedeutungen haben, dadurch gekennzeichnet, daß man eine Verbindung der allgemeinen Formel (III'g) oder (X ) mit einem Acylierungsmittel $R^{7'}-\overset{O}{\overset{\|}{C}}-X$ umsetzt, wobei $R^{7'}$ die oben angegebenen Bedeutungen hat und X eine reaktive Abgangsgruppe wie Halogen oder einen reaktiven Esterrest bedeutet,

und bei den aus der Verbindung der allgemeinen Formel (X) erhaltenen Verbindungen der allgemeinen Formel (X")

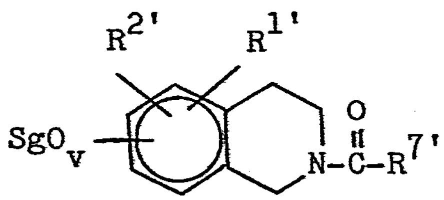

(X")

worin $R^{1'}$, $R^{2'}$, $R^{7'}$, Sg und v die oben an gegebenen Bedeutungen haben, die Schutzgruppe Sg abspaltet.

Die Umsetzung der Verbindungen (III'g) oder (X) mit den Verbindungen $R^{7'}-\underset{O}{\underset{\|}{C}}-X$ erfolgt unter den Bedingungen einer N-Acylierung gemäß der Verfahrensweise, die bereits bei der Herstellung der Verbindungen der allgemeinen Formel (I c) aus den Verbindungen der allgemeinen Formel (I a) beschrieben ist.

Als Schutzgruppe wählt man zweckmäßig entweder die Benzylgruppe, die nach vollzogener Acylierung wie oben beschrieben hydrogenolytisch entfernt wird, oder den Rest $R^{7'}-\underset{O}{\underset{\|}{C}}-$, wobei $R^{7'}$ die oben angegebenen Bedeutungen hat, und geht so vor, daß man die Verbindung der allgemeinen Formel (III'g) zunächst mit 2 Äquivalenten Acylierungsmittel $R^{7'}-COX$ in Gegenwart einer Base in üblicher Weise O- und N-acyliert, und bei der so erhaltenen Bisalkanoylverbindung (X"), für die Sg die Gruppe $R^{7'}-\overset{O}{\overset{\|}{C}}$ bedeutet, durch Erhitzen mit einem Dialkylamin, wie z.B. Diethylamin, in einem inerten Lösungsmittel, wie beispielsweise Ethanol, die Schutzgruppe abspaltet (vgl. Tetrahedron Letters 1982, 1845).

Gegenstand der Erfindung ist weiterhin ein Verfahren zur Herstellung von Verbindungen der allgemeinen Formel (III'c)

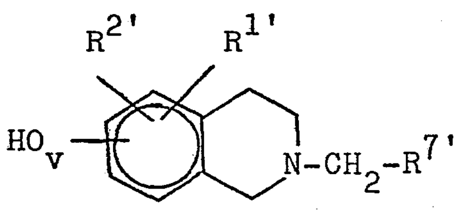

(III'c)

worin $R^{1'}$, $R^{2'}$, $R^{7"}$ und v zur Formel (III') angegebenen Bedeutungen haben, dadurch gekennzeichnet, daß man

a) eine Verbindung der allgemeinen Formel (III'b) oder eine Verbindung der allgemeinen Formel (X") mit einem komplexen Metallhydrid reduziert, und bei den aus der Verbindung (X") erhaltenen Verbindungen die Schutzgruppe Sg abspaltet, oder

b) eine Verbindung der allgemeinen Formel (III'g) oder (X) mit einem Aldehyd der allgemeinen Formel $R^{7'}$-CHO unter den Bedingungen einer reduktiven Aminierung umsetzt, wobei $R^{7'}$ die oben angegebenen Bedeutungen hat, und bei den aus der Verbindung (X) erhaltenen Verbindungen die Schutzgruppe Sg abspaltet, oder

c) eine Verbindung der allgemeinen Formel (III'd)

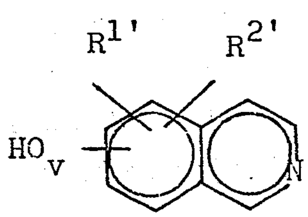

(III'd)

in der v, $R^{1'}$ und $R^{2'}$ die zur Formel (III') angegebenen Bedeutungen haben, mit einem Alkylierungsmittel Alk'E umsetzt, wobei Alk'-E die oben angegebenen Bedeutungen hat und die so erhaltene Verbindung der allgemeinen Formel (XI)

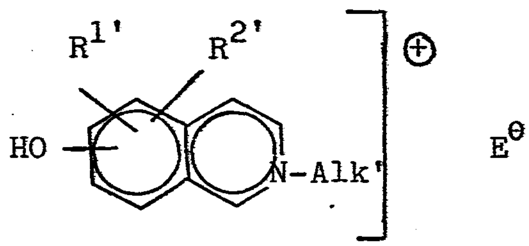

(XI)

in der v, $R^{1'}$, $R^{2'}$ und Alk' die oben angegebenen Bedeutungen haben, mit einem komplexen Hydrid reduziert.

Die Reduktion der Amide nach Verfahrensvariante a) erfolgt mit den gleichen Reagentien und unter den gleichen Reaktionsbedingungen wie bei der Umwandlung der Verbindungen der allgemeinen Formel (I c) in Verbindungen der allgemeinen Formel (I b) beschrieben.

Die Entfernung einer Benzyl- oder Alkyl-Schutzgruppe erfolgt unter den oben angegebenen Bedingungen.

Als Alkanoyl-Schutzgruppe wählt man zweckmäßig den Rest $R^{7'}-\overset{O}{\overset{\|}{C}}-$ selbst, wobei $R^{7'}$ die oben angegebenen Bedeutungen hat, und geht so vor, daß man die Verbindung der allgemeinen Formel (III'g) zunächst mit 2 Äquivalenten Acylierungsmittel $R^{7'}$-COX in Gegenwart einer geeigneten Base in üblicher Weise O- und N-acyliert und aus der so erhaltenen Bisalkanoylverbindung durch Reduktion mit einem komplexen Metallhydrid in einem inerten Lösungsmittel (wie bei der Umwandlung der Verbindungen der allgemeinen Formel (I c) in Verbindungen der allgemeinen Formel (I b) beschrieben) unter gleichzeitiger Abspaltung der Schutzgruppe die Verbindungen der allgemeinen Formel (III'c) erhält.

Zur Herstellung der Verbindungen (III'c) nach Verfahrensvariante b) setzt man ein Amin der Formel (III'g) oder (X) mit einem Aldehyd $R^{7'}$-CHO unter den Bedingungen einer reduktiven Aminierung, wie vorstehend bei Herstellung der Verbindungen der Formel (I b) aus solchen der Formel (I a) beschrieben, um. Als Reduktionsmittel kommen Wasserstoff in Gegenwart eines Metallkatalysators wie z.B. Raney-Nickel oder Natriumcyanoborhydrid zur Anwendung.

Die Abspaltung einer Schutzgruppe erfolgt nach den oben beschriebenen Methoden.

Zur Herstellung der Verbindungen (III'c) nach Verfahrensvariante c) setzte man das Hydroxychinolin (III'd) mit dem Alkylierungsmittel Alk'-E in einem inerten Lösungsmitel wie Ethanol, Aceton oder Sulfolan in einem Temperaturbereich von 20 - 150°C, bevorzugt 50 - 100°C, zu einer Verbindung der allgemeinen Formel (XI) um und reduziert letztere in einem geeigneten Lösungsmittel, wie Wasser, Methanol, Ethanol oder Gemische derselben, mit einem geeigneten Reduktionsmittel, vorzugsweise Natriumborhydrid, zu den Endprodukten (III'c).

Die Hydroxychinoline (III'd) sind in der Literatur beschrieben oder können analog zu bekannten Literaturvorschriften hergestellt werden (vgl. z.B. J. Amer. Chem. Soc. 67, 860 (1945); J. Amer. Chem. Soc. 69, 1944 (1947), J. Org. Chem. 27, 4571, (1962)).

Gegenstand der Erfindung ist weiterhin ein Verfahren zur Herstellung von Verbindungen der allgemeinen Formel (III'e)

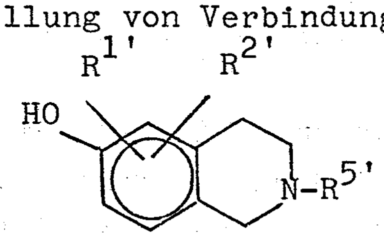

(III'e)

für die $R^{1'}$, $R^{2'}$ und $R^{5'}$ die zur Formel (III') angegegebenen Bedeutungen haben, dadurch gekennzeichnet, daß man eine Verbindung der allgemeinen Formel (VII )

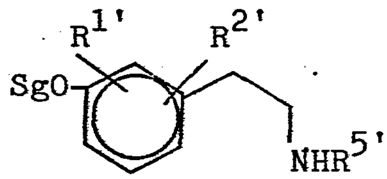

(VII)

in der $R^{1'}$, $R^{2'}$ $R^{5'}$ und Sg die zur Formel (III') angegebenen Bedeutungen haben, mit Formaldehyd in Gegenwart einer Säure umsetzt und anschließend die Gruppe Sg abspaltet.

Zur Durchführung dieses Verfahrens werden Phenylethylamine der allgemeinen Formel (XII) oder Phenylessigsäuren der allgemeinen Formel (XIII)

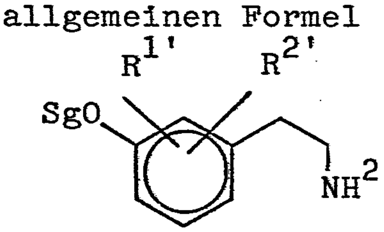

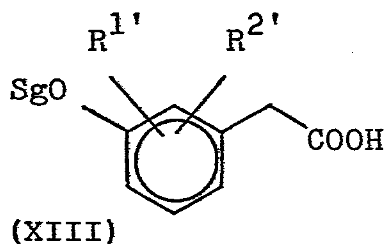

(XII) (XIII)

worin $R^{1'}$, $R^{2'}$ und Sg die oben angegebenen Bedeutungen haben, nach allgemeinen bekannten Methoden (z.B. Acylierung/Lithiumalanat-Reduktion; reduktive Aminierung) in die Phenylethylamine (VII) überführt und diese über eine Pictet-Spengler-Reaktion durch Erhitzen der Phenylethylamine (VII) mit wäßrigem Formaldehyd in Gegenwart einer Säure (vgl. J. Amer. Chem. Soc. 56, (1934) und anschliessende Entfernung der Schutzgruppe (wie weiter oben beschrieben) in die Endprodukte (III'e) überführt.

Zur Herstellung weiterer Ausgangsmaterialien, die teilweise bekannt sind und die in 1-Stellung mono- oder disubstituierte 6-Hydroxy-1,2,3,4-tetrahydroisochinoline (III f) sind, wählt man vorteilhaft die phenolische Cyclisierung (vgl. J. Chem. Soc. (C) 1971. 2632; J. Pharm.

Soc. Jap. 89, 1482 (1969)), indem man die 6-Hydroxyphenylethylamine (VIII) mit einem Aldehyd oder Keton der allgemeinen Formel (IX) in einem inerten Lösungsmittel, vorzugsweise Ethanol, erhitzt, wobei $R^1$, $R^2$, $R^3$, $R^4$ und $R^5$ die vorstehend zu Formel I angegebenen Bedeutungen haben.

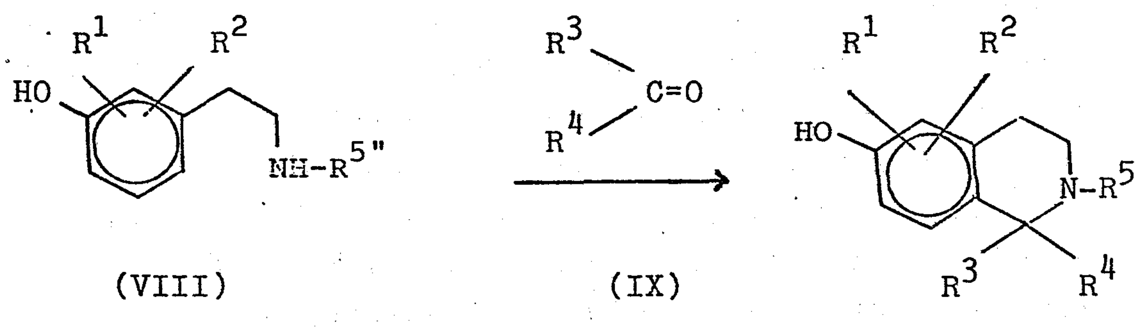

(VIII) (IX) (III f)

Verbindungen der allgemeinen Formel (III) in der D die Bedeutung D I oder D II aus Formel (I) hat, sowie weitere Verbindungen, für die D die Bedeutung D III aus Formel (I) hat, sind teilweise bekannt bzw. lassen sich in Analogie zu Literaturvorschriften herstellen (vgl. z.B. J. Heterocyclic Chem. 5, 825 (1968); Org. Reactions Vol. 6, New York (1951), S. 74 - 150; R.C. Elderfield, Heterocyclic Compounds, Vol. 4 S. 399 ff. und dort zitierte Literatur.)

Die Herstellung von Ausgangsverbindungen der allgemeinen Formel (III), in der Ar die zu Formel I angegebene Bedeutung hat und A eine Methingruppe bedeutet, ist in der BE-PS 837 831, der DE-OS 2 804 096 und in J. Med. Chem. 22, 1003 (1979) beschrieben; solche Verbindungen in denen A Stickstoff bedeutet, können in Analogie zu den oben zitierten Literaturstellen hergestellt werden.

Die Herstellung der Verbindungen der allgemeinen Formel (IV), in der Ar die zu Formel I und E die zur Formel (II) angegebenen Bedeutungen haben, ist in den EP-A 0 050 298 und 0 052 905 beschrieben oder kann in Analogie zu den oben zitierten Literaturstellen erfolgen.

Die Verbindungen der allgemeinen Formel (I) weisen ihre wesentlichen Eigenschaften auch in Form ihrer Salze auf. Zur Herstellung von Säureadditionssalzen kommen alle physiologisch verträglichen Säuren in Frage. Hierzu gehören vorzugsweise die Halogenwasserstoffsäuren, wie z.B. Chlorwasserstoff- und Bromwasserstoff-säure, sowie Salpetersäure, ferner Phosphorsäure oder Schwefelsäure. Bevorzugte organische Säuren sind mono- und bifunktionelle Carbonsäuren und Hydroxycarbonsäuren wie z.B. Essigsäure, Maleinsäure, Oxalsäure, Bernsteinsäure, Fumarsäure, Weinsäure, Zitronensäure, Salicylsäure, Sorbinsäure, Milchsäure sowie Sulfonsäuren wie z.B. p-Toluolsulfonsäure, Methyl- und Phenylsulfonsäure sowie 1,5-Naphthalindisulfonsäure.
Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure oder Salpetersäure, erhalten werden und in bekannter Weise, z.B. durch Abfiltrieren, isoliert und gegebenenfalls durch Waschen oder Umkristallisation mit einem inerten organischen Lösungsmittel gereinigt werden.

Aus der allgemeinen Formel (I) ist ersichtlich, daß die erfindungsgemäßen Verbindungen mindestens zwei asymmetrische Kohlenstoffatome aufweisen, die sich in 2- und 4-Stellung des Dioxolanringes befinden. Diese Verbindungen können demnach in Form verschiedener Stereoisomeren vorliegen.

Die diastereomeren Racemate (cis- oder trans-Form) der Verbindungen der allgemeinen Formel (I) können in üblicher Weise getrennt werden. Geeignete Methoden sind beispielsweise die selektive Kristallisation und die Chromatographie, z.B. die Säulenchromatographie.

Da die stereochemische Konfiguration bereits in den Zwischenprodukten der allgemeinen Formel (II) bzw. (V) vorgegeben ist, kann die Trennung in die cis- und trans-Form bereits auf dieser Stufe oder bevorzugt noch früher, beispielsweise auf der Stufe der Zwischenprodukte der allgemeinen Formel (IV) erfolgen. Die Trennung ist nach vorstehend beschriebenen Methoden leicht möglich. Die cis- und trans-diastereomeren Racemate können ihrerseits in üblicher Weise in ihre optischen Antipoden cis(+), cis(-) bzw. trans(+) und trans(-) getrennt werden.

Die neuen Verbindungen der Formel (I) sind Chemotherapeutika und besitzen eine einschlägig bekannten Verbindungen überlegene Wirkung und Verträglichkeit gegen Pilzinfektionen, und zwar sowohl bei lokaler als auch bei systemischer Anwendung. Sie sind in vitro sehr gut wirksam gegen Hautpilze, wie z.B. Trichophyton mentagrophytes, Microsporum canis, Epidermophytes floccosum; Schimmelpilze, wie z.B. Aspergillus niger oder Hefen, wie z.B. Candida albicans, C.tropicalis, Torulpsis glabrata und Trich osporon cutaneum oder Trichomonas vaginalis oder T.fetus oder grampositive und gramnegative Bakterien.

Auch in vivo, z.B. bei der experimentellen Nierencandidose der Maus besitzen die Verbindungen nach oraler oder parenteraler Anwendung einen sehr guten systemischen Effekt z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als geeignete Anwendungsform der erfindungsgemäßen Verbindung kommen beispielsweise in Frage, Tabletten oder Kapseln, Suspensionen, Lösungen, Gelees, Cremes oder Salben sowie Aerosole in Form von Spray.
Die Anwendungskonzentration für Lösungen, Gelees, Cremes oder Salben sowie Aerosole in Form von Spray beträgt im allgemeinen zwischen 0,1 - 3 Gewichtsprozenten.
Die orale Anwendung erfolgt in pharmazeutisch üblichen Zubereitungen, beispielsweise in Form von Tabletten oder Kapseln, die pro Tagesdosis 50 - 200 mg des Wirkstoffes in Mischung mit einem gebräuchlichen Trägerstoff und/oder Konstituents enthalten.
Für die lokale Anwendung können z.B. Suspensionen, Lösungen, Gelees, Cremes, Salben oder Suppositorien verwendet werden.
Für die parenterale Anwendung kommen geeignete Suspensionen oder Lösungen in Frage in einer Anwendungskonzentration zwischen 0,1 - 5 Gewichtsprozenten.

Die Beispiele erläutern die Erfindung.

I. Beispiele für das Herstellungsverfahren a) nach Anspruch 2.

6-/‾(2RS,4SR)-2-(2,4-Dichlorphenyl)-2-(1H-imidazol-1-ylmethyl)-1,3-dioxolan-4-ylmethyloxy_7-1,2,3,4-tetrahydro-isochinolin

Zu einer Lösung von 1,50 g 6-Hydroxy-1,2,3,4-tetrahydroisochinolin in 30 ml Dimethylsulfoxid wird 0,55 g Natriumhydrid (50 %ige Suspension in Öl) gegeben und bei 50°C bis zum Ende der Wasserstoffentwicklung gerührt. Man gibt 4,07 g (2RS, 4SR)-2-(2,4-Dichlorphenyl)-2-(1H-imidazol-1-ylmethyl)-1,3-dioxolan-4-ylmethyl-methansulfonat zu und rührt 5 Stunden bei 80°C. Das Lösungsmittel wird am Ölpumpenvakuum abgezogen, der Rückstand mit einem Gemisch aus 50 ml Wasser und 50 ml Methylenchlorid aufgenommen, die organische Phase noch zweimal mit Wasser ausgeschüttet, getrocknet und einrotiert. Der Rückstand wird an Kieselgel mit Methanol als Laufmittel chromatographiert. Man erhält 2,45 g farbloses Öl (53 % d. Th.), das allmählich durchkristallisiert. Fp. 143 - 145°C.

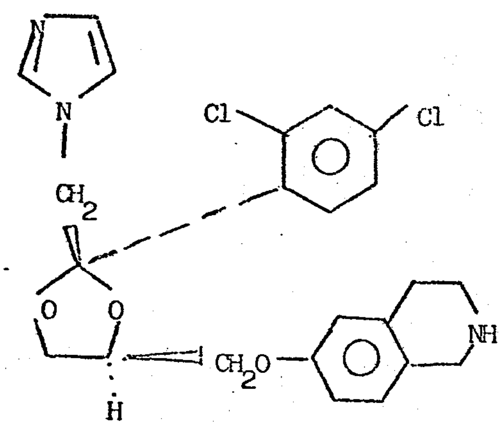

| Analyse: | Ber.: | Gef.: |
|---|---|---|
| $C_{23}H_{23}Cl_2N_3O_3$ | C 60,00 | C 59.4 |
| MG 460.36 | H 5.04 | H 5.2 |
| | N 9.13 | N 9.0 |

0121753

Beispiel 2

2-Allyl-6-⁅(2RS,4SR)-2-(2,4-dichlorphenyl)-2-(1H-imidazol-1-ylmethyl)-1.3-dioxolan-4-ylmethyloxy⁆-1,2,3,4-tetrahydro-isochinolin

Zu einer frisch hergestellten Alkoholatlösung aus 0,23 g Natrium in 30 ml trockenem Ethanol gibt man 1,89 g 2-Allyl-6-hydroxy-1,2,3,4-tetrahydro-isochinolin, rührt 15 Minuten nach, gibt 4,07 g (2RS,4SR)-2-(2,4-Dichlorphenyl)-2-(1H-imidazol-1-ylmethyl)-1,3-dioxolan-4-ylmethyl-methansulfonat zu und erhitzt anschließend 8 Stunden unter Rückfluß. Nach dem Abkühlen wird das Ethanol am Rotationsverdampfer abgezogen und der Rückstand mit einem Gemisch von 50 ml Wasser aufgenommen. Die organische Phase wird noch zweimal mit Wasser geschüttelt, über Magnesiumsulfat getrocknet und einrotiert. Der Rückstand wird säulenchromatographisch an Kieselgel mit einer Mischung von Ethylacetat/Methanol 4 : 1 gereinigt. Man erhält 1.3 g eines farblosen Harzes (26 % d.Th.) welches allmählich durchkristallisiert. Fp. 94 - 95°.

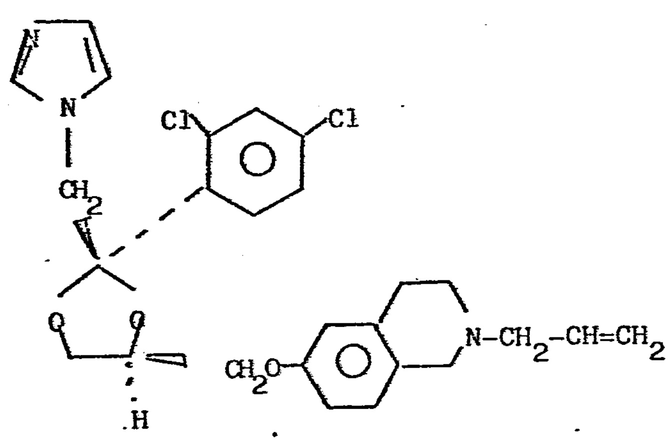

| Analyse: | Ber.: | Gef.: |
|---|---|---|
| $C_{26}H_{27}Cl_2N_3O_3$ | C 62,40 | 59.4 |
| MG 500,43 | H 5.44 | 5.5 |
| | N 8.40 | 8.3 |

Beispiel 3

6-[(2RS,4SR)-2-(2,4-Dichlorphenyl)-2-(1H-imidazol-1-ylmethyl)-1.3-dioxolan-4-ylmethyloxy]-2-(4-phenyl-benzyl)-1,2,3,4-tetrahydro-isochinolin

Zu einer Lösung von 0,75 g 6-Hydroxy-1,2,3,4-tetrahydroisochinolin in 30 ml Dimethylformamid gibt man 0,26 g Natriumhydrid (50 %ige Suspension in Öl) und rührt 30 Minuten bei 50°C. Anschließend trägt man 2,04 g (2RS,4SR)-2-(2,4-Dichlorphenyl)-2-(1H-imidazol-1-ylmethyl)-1,3-dioxolan-4-ylmethyl-methansulfonat ein und rührt 5 Stunden bei 80°C. Man läßt abkühlen, gibt 1,00 g fein pulveriertes Kaliumcarbonat und 1,00 g 4-Chlormethyl-biphenyl zu und rührt weitere 5 Stunden bei 60°. Nach dem Abkühlen wird das Lösungsmittel am Rotationsverdampfer mit der Ölpumpe abgezogen und der Rückstand mit einem Gemisch von 50 ml Wasser und 50 ml Methylenchlorid aufgenommen. Die organische Phase wird noch zweimal mit Wasser gewaschen, über Magnesiumsulfat gerocknet und einrotiert. Zur Reinigung wird das Rohprodukt am Kieselgel mit einer Mischung aus Ethylacetat und Methanol 19 : 1 chroatographiert. Man erhält 1.7 g eines zähen Öls (57 % d. Th.), welches allmählich durchkristallisiert. Fp. 103 - 105°.

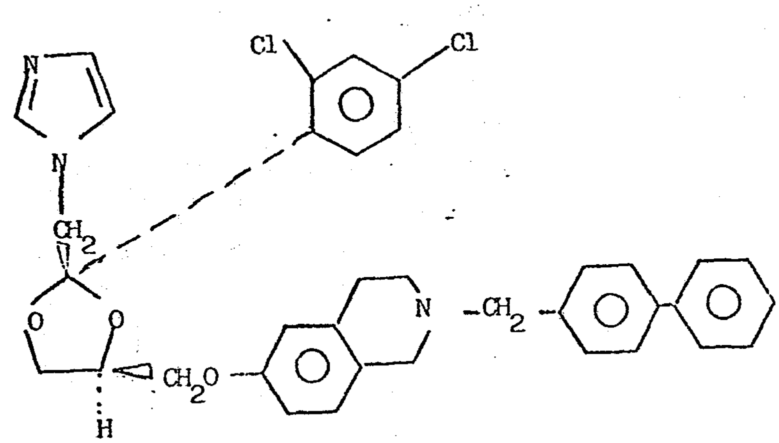

| Analyse: | Ber.: | Gef.: |
|---|---|---|
| $C_{36}H_{33}Cl_2N_3O_3$ | C 69,01 | C 68.8 |
| MH 626,59 | H 5,31 | H 5,4 |
| | N 6,71 | N 6,8 |

Beispiel 4

6'-[(2RS,4SR)-2-(2,4-Dichlorphenyl)-2-(1H-imidazol-1-ylmethyl)-1.3-dioxolan-4-ylmethyloxy]-2'-methyl-1',2',3',4'-tetrahydrospiro[tetrahydrothiopyran-4,1'-isochinolin]

Zu einer Lösung von 1,19 g 6'-Hydroxy-1',2',3',4'-tetrahydrospiro[tetrahydrothiopyran-4,1'-isochinolin] in 30 ml Dimethylformamid gibt man 0,26 g Natriumhydrid (50 %ige Suspension in Öl) und rührt 30 Minuten bei 50°C. Anschließend trägt man 2,04 g (2RS,4SR)-2-(2,4-Dichlorphenyl)-2-(1H-imidazol-1-ylmethyl)-1,3-dioxolan-4-ylmethyl-methansulfonat ein und rührt 5 Stunden bei 80°C. Nach dem Abkühlen wird mit Wasser gewaschen, getrocknet und einrotiert. Das Rohprodukt wird durch Chromatographie an Kieselgel mit einem Gemisch aus Ethylacetat/Methanol 9 : 1 gereinigt. Man erhält 2,0 g schwach gelbes Harz (71 % d. Th.), welches beim Stehen allmählich kristallisiert. Fp. 138 - 139°.

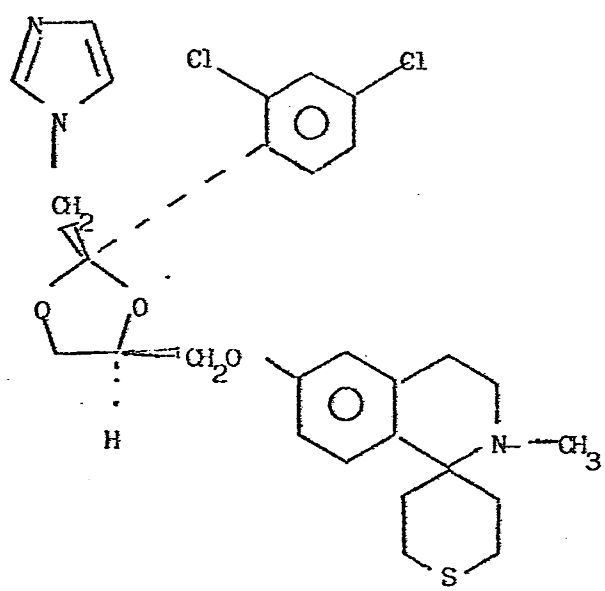

| Analyse: | Ber.: | Gef.: |
|---|---|---|
| $C_{28}H_{31}Cl_2N_3O_3S$ | C 60,00 | C 59,5 |
| MG 560,55 | H 5,57 | H 5,5 |
| | N 7,50 | N 7,5 |

Beispiel 5

6-/(2RS,4SR)-2-(2,4-Dichlorphenyl)-2-(1H-imidazol-1-ylmethyl)-1,3-dioxolan-4-ylmethyloxy/-2-(4-biphenylylacetyl)-1,2,3,4-tetrahydroisochinolin

Zu einer Lösung von 4,6 g der gemäß Beispiel 1 hergestellten Verbindung und 1,5 ml Triethylamin in 50 ml Methylenchlorid tropft man unter Rühren und Kühlung eine Lösung von 2,4 g 4-Biphenylylessigsäurechlorid in 10 ml Methylenchlorid. Nach Ausschütteln mit Wasser, 2 n-Natronlauge und nochmals Wasser wird die organische Phase über Magnesiumsulfat getrocknet und einrotiert. Das Rohprodukt wird an Kieselgel mit einem Gemisch an Ethylacetat /Methanol 19 :1 chromatographiert). Man erhält 3,2 g eines farblosen Harzes (49 % d. Th.).

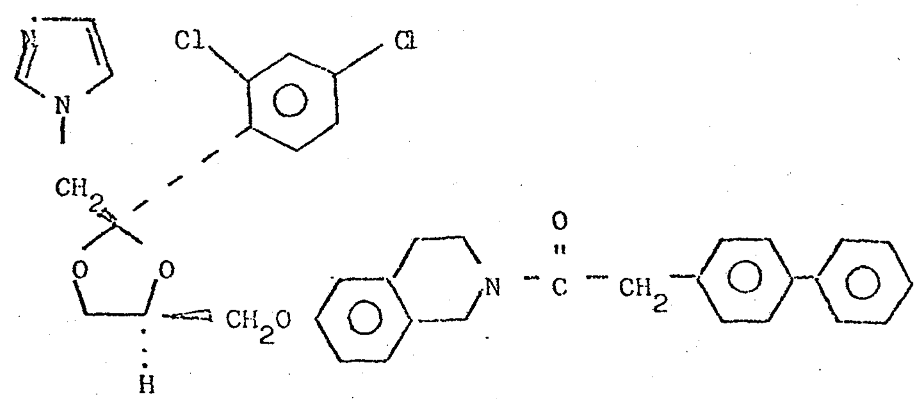

| Analyse: | Ber.: | Gef.: |
|---|---|---|
| $C_{37}H_{33}Cl_2N_3O_4$ | C 67,89 | C 67,7 |
| MG 654,60 | H 5,08 | H 5,0 |
| | N 6,42 | N 6,4 |

Beispiel 6

6-/(2RS,4SR)-2-(2,4-Dichlorphenyl)-2-(1H-imidazol-1-ylmethyl)-1,3-dioxolan-4-ylmethyloxy7-2-(4-chlor-3-trifluormethyl-benzyl)-1.2.3.4-tetrahydro-isochinolin

Zu einem Gemisch von 1,75 g der gemäß Beispiel 1 erhaltenen Verbindung, 0.80 g 4-Chlor-3-trifluormethyl-benzaldehyd und 0,7 g Toluolsulfonsäure-monohydrat in 30 ml Ethanol gibt man 0,3 g Natriumcyanoborhydrid und rührt 1 Tag bei Raumtemperatur. Nach dem Abziehen des Lösungsmittels wird mit einem Gemisch aus 2 n Natronlauge und Methylenchlorid aufgenommen, die organische Phase mit Wasser gewaschen, getrocknet und einrotiert. Der ölige Rückstand wird an Kieselgel mit Ethylacetat chromatographiert. Man erhält 1,0 g eines zähen Harzes (40 % d. Th.) das beim Verreiben mit Diethylether kristallisiert. Fp. 107 - 108°.

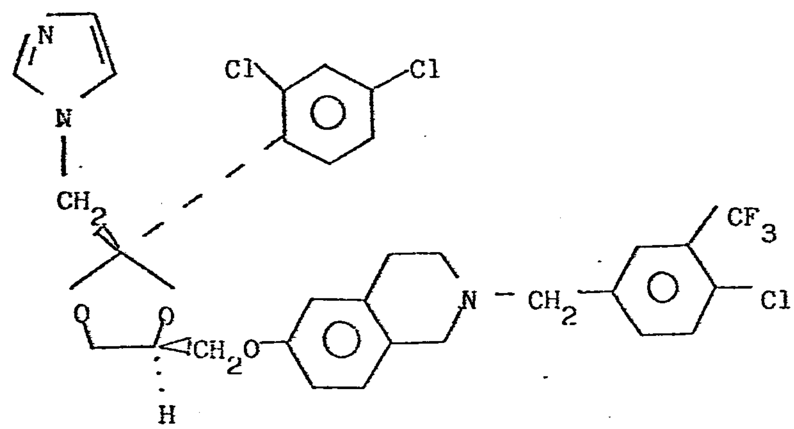

| Analyse: | Ber.: | Gef.: |
|---|---|---|
| $C_{31}H_{27}Cl_3F_3N_3O_3$ | C 57,03 | C 56,0 |
| MG: 652,93 | H 4,17 | H 4,3 |
| | N 6,44 | N 6,2 |

## Beispiel 7

6-[(2RS,4SR)-2-(2,4-Dichlorphenyl)-2-(1H-imidazol-1-ylmethyl)-1.3-dioxolan-4-ylmethyloxy]-2-[2-(4-phenylbenzyl)-ethyl]-1,2,3,4-tetrahydro-isochinolin

Zu einer Lösung von 0,1 g Lithiumaluminiumhydrid in 20 ml trockenem Tetrahydrofuran tropft man unter Rühren eine Lösung von 2,0 g der gemäß Versuch 5 hergestellten Verbindung in 10 ml trockenem Tetrahydrofuran und rührt 3 Stunden bei 50°C. Man zersetzt mit Wasser, saugt vom organischen Material ab, tocknet über Magnesiumsulfat und zieht das Lösungsmittel ab. Es bleibt ein zähes Öl zurück, das an Kieselgel mit einem Gemisch aus Ethylacetat/Methanol 9 : 1 chromatographiert wird. Man erhält 1,0 g eines zähen Öls (51 % d. Th.) das beim Stehen allmählich kristallisiert. Fp. 125 - 127 °.

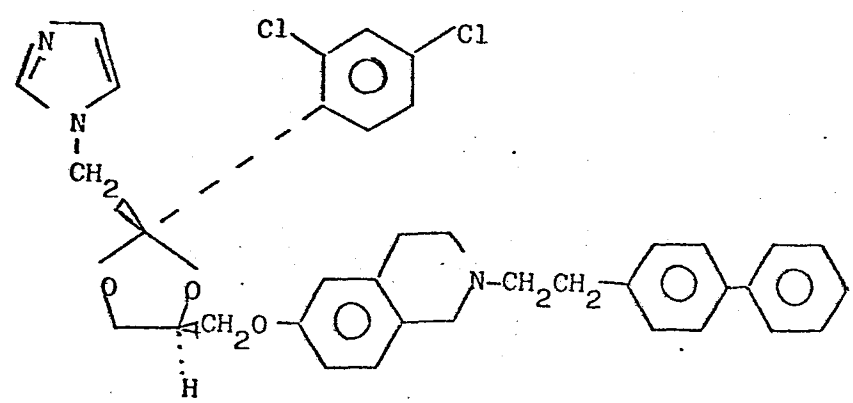

| Analyse: | Ber.: | Gef.: |
|---|---|---|
| $C_{37}H_{35}Cl_2N_3O_3$ | C 69,37 | C 70,1 |
| MG: 640,61 | H 5,50 | H 5,6 |
| | N 6,56 | N 6,8 |

Beispiel 8

6-[(2RS,4SR)-2-(2,4-Dichlorphenyl)-2-(1H-imidazol-1-ylmethyl)-1.3-dioxolan-4-ylmethyloxy]-2-(4-fluorbenzyl)-1,2,3,4-tetrahydro-isochinolin

Eine Lösung von 1,75 g der gemäß Beispiel 1 hergestellten Verbindung wird mit 0,55 g 4-Fluorbenzylchlorid und 0,60 g Kaliumcarbonat in 30 ml Dimethylformamid 5 Stunden auf 80° erwärmt. Nach dem Abkühlen wird das Lösungsmittel im Ölpumpenvakuum abgezogen, der Rückstand mit Wasser/Methylenchlorid aufgenommen, die organische Phase zweimal mit Wasser ausgeschüttelt, getrocknet und einrotiert. Das Rohprodukt wird an Kieselgel mit einem Gemisch aus Ethylacetat/ Methanol 19 : 1 chromatographiert. Man erhält 1,5 g eines farblosen Öls (69 % d. Th.) das beim Stehen kristallisiert. Fp. 119 - 120°.

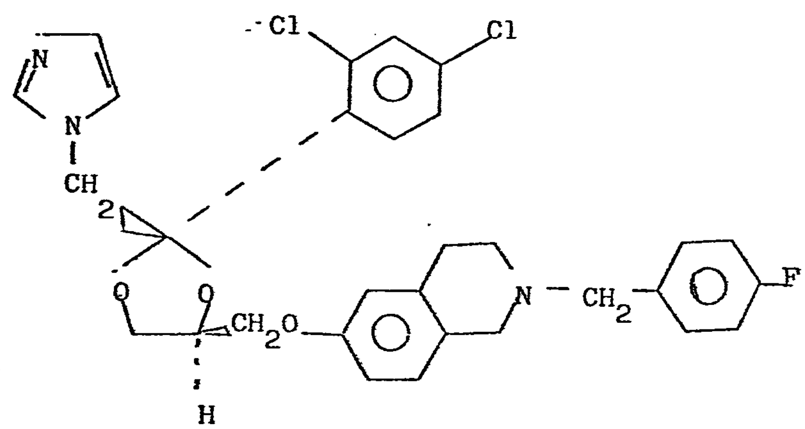

| Analyse: | Ber.: | | Gef.: | |
|---|---|---|---|---|
| $C_{30}H_{28}Cl_2FN_3O_3$ | C | 63,38 | C | 62,8 |
| MG 568,48 | H | 4,96 | H | 5,0 |
| | N | 7,39 | N | 7,4 |

0121753

Beispiel 9

6-/(2RS,4SR)-2-(2,4-Dichlorphenyl)-2-(1H-imidazol-1-ylmethyl)-1,3-dioxolan-4-ylmethyloxy_7-2-/2-(4-methoxyphenyl)-ethyl_7-1,2,3,4-tetrahydro-isochinolin

1,4 g 6-Hydroxy-2-/2-(4-methoxyphenyl)ethyl7-1,2,3,4-tetrahydroisochinolin, 2,1 g (2RS, 4SR)-2- (2,4-Dichlorphenyl)-2-(1H-imidazol-1-ylmethyl)-1,3-dioxolan-4-ylmethyl-methansulfonat und 0,3 g Tetrabutylammoniumbromid werden in einem Gemisch von 40 ml Toluol und 10 ml 50 %iger Natronlauge 9 Stunden unter heftigem Rühren unter Rückfluß erhitzt. Nach dem Abkühlen wird mit 50 ml Wasser versetzt, die organische Phase mit Wasser gewaschen, über Magnesiumsulfat getrocknet und einrotiert. Das Rohprodukt wird säulenchromatographisch an Kieselgel mit einem Gemisch aus Ethylacetat und Methanol 19 : 1 gereinigt. Man erhält 2,2 g eines farblosen Öls (76 % d. Th.) das beim Verreiben mit Diethylether kristallisiert. Fp.: 121 - 122°.

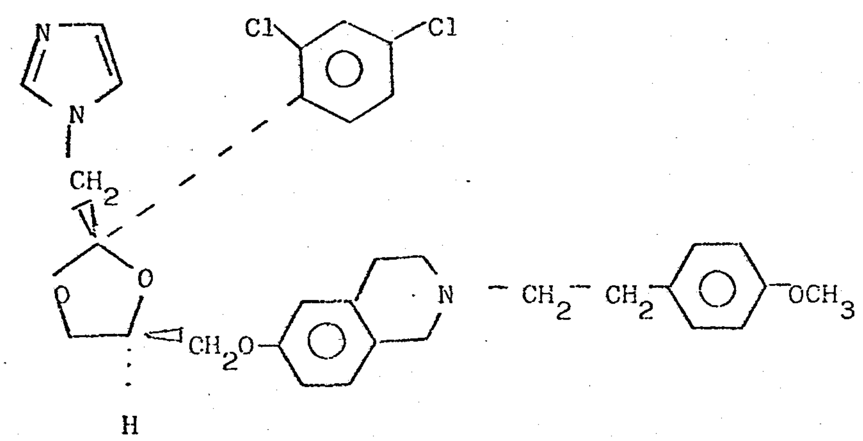

| Analyse: | Ber.: | Gef.: |
|---|---|---|
| $C_{32}H_{33}Cl_3N_3O_4$ | C 64,65 | C 64,3 |
| MG: 594,54 | H 5,59 | H 5,5 |
| | N 7,07 | N 7,1 |

Außerdem werden nach dem Verfahren a) auch die im folgenden nach Verfahren b) hergestellten Verbindungen erhalten.

II. Beispiele für das Herstellungsverfahren b) nach Anspruch 2.

Beispiel 10

a) 6-[(2RS,4SR)-2-(4-Chlorphenyl)-2-brommethyl-1,3-dioxolan-4-ylmethyloxy]-2-(4-phenylbenzyl)-1,2,3,4-tetrahydro-isochinolin

Zu einer Lösung von 6,3 g 6-Hydroxy-2-(4-phenylbenzyl)-1,2,3,4-tetrahydroisochinolin in 100 ml trockenem Dimethylformamid gibt man unter Kühlung portionsweise 1,1 g Natriumhydrid (50 %-ige Suspension in Öl). Man rührt 1 Stunde bei 50° und gibt dann 7,7 g (2RS,4SR)-2-(4-Chlorphenyl)-2-brommethyl-1,3-dioxolan-4-ylmethylmethansulfonat (Darstellung nach EP-OS 0 050 298) zu. Man rührt 5 Stunden bei 80°C, kühlt ab, zieht das Lösungsmittel im Ölvakuum ab und nimmt den Rückstand mit einem Gemisch aus Wasser und Methylenchlorid auf. Nach zweimaligem Waschen mit Wasser und Trocknen über Magnesiumsulfat wird einrotiert und der Rückstand an Kieselgel mit einem Gemisch aus Methylenchlorid und Ethylacetat im Verhältnis 3 : 1 chromatographiert. Man erhält 7,0 g eines hochviskosen Öls (58 % d. Th.).

b) 6-[(2RS,4SR)-2-(4-Chlorphenyl)-2-(1H-imidazol-1-ylmethyl)-1,3-dioxolan-4-ylmethyloxy]-2-(4-phenylbenzyl)-1,2,3,4-tetrahydro-isochinolin

Zur Suspension von 1,2 g Natriumhydrid (50 %-ige Suspension in Öl) in 50 ml trockenem Dimethylacetamid gibt man unter Rühren 1,5 g Imidazol und rührt nach bis die Wasserstoffentwicklung beendet ist. Anschließend gibt man eine Lösung von

7,0 g des unter a) erhaltenen Brommethylderivats in 10 ml Dimethylacetamid und eine Spaltspitze Kaliumjodid zu. Man erhitzt 24 Stunden auf 140°C, zieht nach dem Abkühlen das Lösungsmittel am Ölpumpenvakuum ab und nimmt den Rückstand mit Wasser/Methylenchlorid auf. Nach zweimaligem Schütteln mit Wasser wird getrocknet, einrotiert und der Rückstand an Kieselgel mit einem Gemisch aus Ethylacetat und Methanol im Verhältnis 19 : 1 chromatographiert. Man erhält 2,2 g eines hochviskosen Öls (32 % d. Th.).

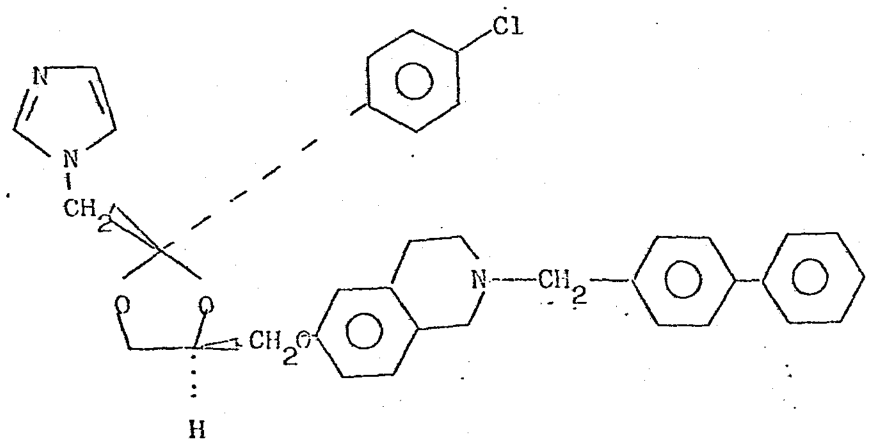

Analyse

| | | | | | |
|---|---|---|---|---|---|
| $C_{36}H_{34}ClN_3O_3$ | Ber.: | C 73,02 | Gef.: | C 72,3 |
| MG 592,14 | | H 5,79 | | H 5,7 |
| | | N 7,10 | | N 6,8 |

Beispiel 11

6-/(2RS,4SR)-2-(4-Chlorphenyl)-2-(1H-imidazol-1-ylmethyl)-1,3-dioxolan-4-ylmethyloxy/-2-/3-(4-chlorphenyl)-propyl/-1,2,3,4-tetrahydro-isochinolin

Zu einer Lösung von 3,0 g 6-Hydroxy-2-/3-(4-chlorphenyl)-propyl/-1,2,3,4-tetrahydro-isochinolin in 30 ml Dimethylacetamid gibt man 550 mg Natriumhydrid (50 %-ige Suspension Öl), rührt bis zum Ende der Wasserstoffentwicklung und gibt 3,9 g (2RS,4SR)-2-(4-Chlorphenyl)-2-brommethyl-1,3-dioxolan-4-ylmethyl-methansulfonat zu. Man erhitzt 6 Stunden auf 80° und fügt dann 0,9 g Imidazol-Natriumsalz und eine Spaltspitze Kaliumjodid zu. Die Lösung wird weitere 6 Stunden bei 100° gerührt. Nach dem Abkühlen zieht man das Lösungsmittel am Ölpumpenvakuum ab, nimmt mit Wasser/Methylenchlorid auf, wäscht die organische Phase zweimal mit Wasser, trocknet über Magnesiumsulfat und zieht das Lösungsmittel ab. Das Rohprodukt wird durch Chromatographie an Kieselgel mit einem Gemisch aus Ethylacetat/Methanol 9 : 1 gereinigt. Man erhält 1,8 g eines zähen Öls (31 % d.Th.) das beim Verreiben mit Diethylether kristallisiert.

Fp.: 101 - 103°.

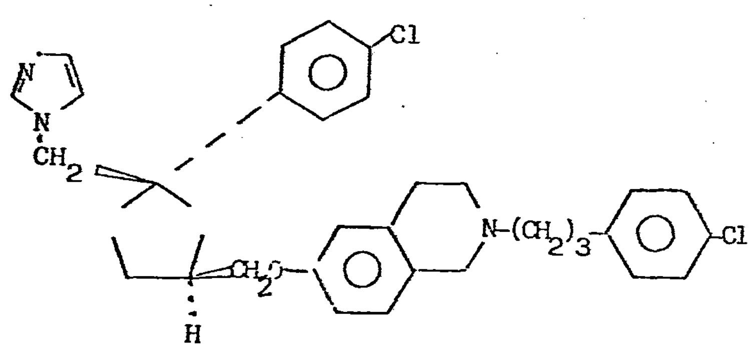

Analyse

| | | | | | |
|---|---|---|---|---|---|
| $C_{32}H_{33}Cl_2N_3O_3$ | Ber.: | C | 66,43 | Gef.: C | 66,0 |
| MG 578,54 | | H | 5,75 | H | 5,8 |
| | | N | 7,26 | N | 7,0 |

Beispiel 12

Analog den Beispielen 1 - 11, jedoch unter Verwendung äquivalenter Mengen der entsprechenden Ausgangsverbindungen können die folgenden, in Tabelle 1 aufgeführten Verbindungen der allgemeinen Formel (I) hergestellt werden.

Tabelle 1

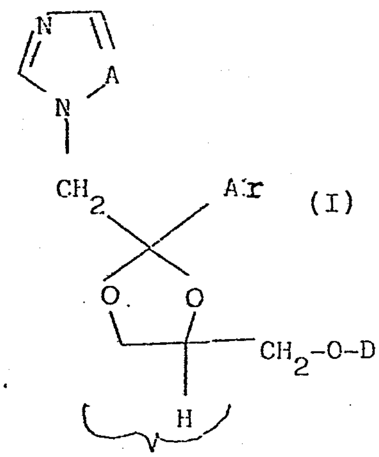

(I)

G

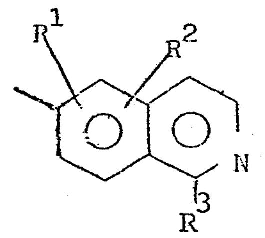

D I

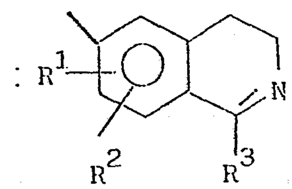

D II

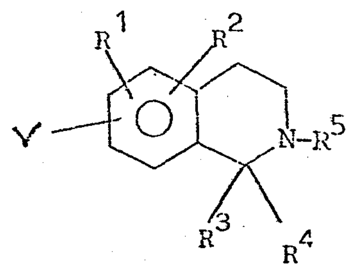

D III

a) v gibt in Tabelle 1 die Position des Restes G-$CH_2$-O- am Isochinolin-Rest D an

b) cis- und trans bezieht sich auf die relative Position des Azolyl-methyl-Restes und der Gruppe -$CH_2$-O-D am Dioxolanring.

| Verb. Nr. | A | Ar | Isochinolin vom Typ | V | $R_1$ | $R_2$ | $R_3$ | $R_4$ | $R_5$ | Isomerie am Dioxolanring | Fp. [°C] | Herstellung analog Beispiel |
|---|---|---|---|---|---|---|---|---|---|---|---|---|
| 12.1 | CH | 2,4-$C_6H_3Cl_2$ | DI | 6 | H | H | H | – | – | cis | 167 | 4 |
| 12.2 | CH | 2,4-$C_6H_3Cl_2$ | DI | 6 | H | H | $-nC_7H_{15}$ | – | – | cis | 108–109 | 4 |
| 12.3 | CH | 2,4-$C_6H_3Cl_2$ | DI | 6 | H | H | 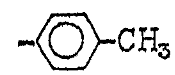 | – | – | cis | Harz | 4 |
| 12.4 | CH | 2,4-$C_6H_3Cl_2$ | DI | 6 | H | H | 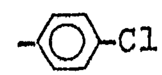 | – | – | cis | Harz | 4 |
| 12.5 | CH | 2,4-$C_6H_3Cl_2$ | DII | 6 | H | H | $-nC_7H_{15}$ | – | – | cis | 86– 88 | 4 |
| 12.6 | CH | 2,4-$C_6H_3Cl_2$ | DII | 6 | H | H | 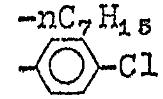 | – | – | cis | Harz | 4 |
| 12.7 | CH | 2,4-$C_6H_3Cl_2$ | DII | 6 | 7-$OCH_3$ | H | 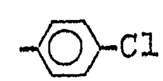 | – | – | cis | Harz | 4 |
| 12.8 | CH | 2,4-$C_6H_3Cl_2$ | DII | 6 | H | H | 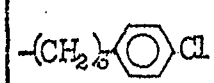 | – | – | cis | 112 | 4 |
| 12.9 | CH | 2,4-$C_6H_3Cl_2$ | DIII | 6 | 7-$OCH_3$ | H | 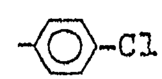 | H | H | cis | Harz | 4 |

| Verb. Nr. | A | Ar | Iso-chino-lin vom Typ | V | $R_1$ | $R_2$ | $R_3$ | $R_4$ | $R_5$ | Isomerie am Dioxolanring | Fp. [°C] | Herstellung analog Beispiel |
|---|---|---|---|---|---|---|---|---|---|---|---|---|
| 12.10 | CH | $2,4-C_6H_3Cl_3$ | DIII | 6 | H | H | $-C_2H_5$ | H | $CH_3$ | cis | Harz | 4 |
| 12.11 | CH | $2,4-C_6H_3Cl_2$ | DIII | 6 | H | H | $CH_3$ | $CH_3$ | $CH_3$ | cis | 110-112 | 4 |
| 12.12 | CH | $2,4-C_6H_3Cl_2$ | DIII | 6 | H | H | $-(CH_2)_5-$ | | $CH_3$ | cis | 127-130 | 4 |
| 12.13 | CH | $2,4-C_6H_3Cl_2$ | DIII | 6 | H | H | $-(CH_2)_2-O-(CH_2)_2-$ | | $CH_3$ | cis | | 4 |
| 12.14 | CH | $2,4-C_6H_3Cl_2$ | DIII | 6 | H | H | $-(CH_2)_2-N(CH_3)-(CH_2)_2-$ | | $CH_3$ | cis | Harz | 4 |
| 12.15 | CH | $2,4-C_6H_3Cl_2$ | DIII | 6 | H | H | $-(CH_2)_2-N(CH_2C_6H_5)-(CH_2)_2-$ | | $CH_3$ | cis | Harz | 4 |
| 12.16 | CH | $2,4-C_6H_3Cl_2$ | DIII | 6 | H | H | $-(CH_2)_2-N(COCH_3)-(CH_2)_2-$ | | $CH_3$ | cis | Harz | 4 |
| 12.17 | CH | $2,4-C_6H_3Cl_2$ | DIII | 6 | H | H | $-(CH_2)_2-N(COC_6H_5)-(CH_2)_2-$ | | $CH_3$ | cis | Harz | 4 |
| 12.18 | CH | $2,4-C_6H_3Cl_2$ | DIII | 6 | H | H | $-(CH_2)_2-N(COOC_2H_5)-(CH_2)_2-$ | | $CH_3$ | cis | Harz | 4 |
| 12.19 | CH | $2,4-C_6H_3Cl_2$ | DIII | 6 | H | H | 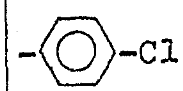 | H | $CH_3$ | cis | Harz | 4 |
| 12.20 | CH | $2,4-C_6H_3Cl_2$ | DIII | 6 | H | H | 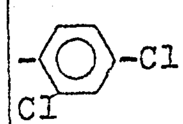 | H | $CH_3$ | cis | Harz | 4 |

| Verb. Nr. | A | Ar | Isochinolin vom Typ | V | $R_1$ | $R_2$ | $R_3$ | $R_4$ | $R_5$ | Isomerie am Dioxolanring | Fp. [°C] | Herstellung analog Beispiel |
|---|---|---|---|---|---|---|---|---|---|---|---|---|
| 12.21 | CH | 2,4-$C_6H_3Cl_2$ | DIII | 6 | H | H | 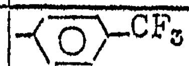 | H | $CH_3$ | cis | Harz | 4 |
| 12.22 | CH | 2,4-$C_6H_3Cl_2$ | DIII | 6 | H | H | 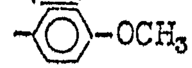 | H | $CH_3$ | cis | Harz | 4 |
| 12.23 | CH | 2,4-$C_6H_3Cl_2$ | DIII | 6 | H | H | 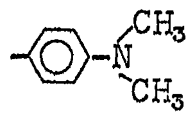 | H | $CH_3$ | cis | Harz | 4 |
| 12.24 | CH | 2,4-$C_6H_3Cl_2$ | DIII | 6 | H | H | 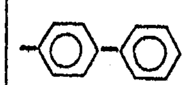 | H | $CH_3$ | cis | 130-135 | 4 |
| 12.25 | CH | 2,4-$C_6H_3Cl_2$ | DIII | 6 | H | H | 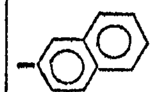 | H | $CH_3$ | cis | Harz | 4 |
| 12.26 | CH | 2,4-$C_6H_3Cl_2$ | DIII | 6 | H | H | 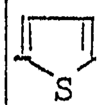 | H | $CH_3$ | cis | Harz | 4 |
| 12.27 | CH | 2,6-$C_6H_3Cl_2$ | DIII | 6 | H | H | H | H | $CH_3$ | cis | 120-121 | 4 |
| 12.28 | CH | 2,6-$C_6H_3Cl_2$ | DIII | 6 | 5$CH_3$ | 7$CH_3$ | H | H | $CH_3$ | cis | Harz | 4 |
| 12.29 | CH | 2,6-$C_6H_3Cl_2$ | DIII | 6 | H | H | H | H | -n$C_4H_9$ | cis | | 4 |

| Verb.- | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.30 | CH | $2,4-C_6H_3Cl_2$ | DIII | 6 | H | H | H | H | $-nC_8H_{17}$ | cis | 99-100 | 4 |
| 12.31 | CH | $2,4-C_6H_3Cl_2$ | DIII | 6 | H | H | H | H | $-nC_{12}H_{25}$ | cis | | 4 |
| 12.32 | CH | $2,4-C_6H_3Cl_2$ | DIII | 6 | H | H | H | H | $-CH_2-C{\equiv}CH$ | cis | 117-118 | 4 |
| 12.33 | CH | $2,4-C_6H_3Cl_2$ | DIII | 6 | H | H | H | H | 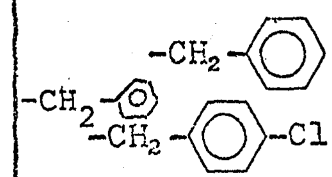 | cis | 88 | 9 |
| 12.34 | CH | $2,4-C_6H_3Cl_2$ | DIII | 7 | H | H | H | H | | cis | | 9 |
| 12.35 | CH | $2,4-C_6H_3Cl_2$ | DIII | 5 | H | H | H | H | | cis | Harz | 4 |
| 12.36 | CH | $2,4-C_6H_3Cl_2$ | DIII | 6 | H | H | H | H | 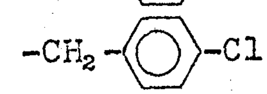 | cis | Harz | 4 |
| 12.37 | CH | $2,4-C_6H_3Cl_2$ | DIII | 7 | H | H | H | H | 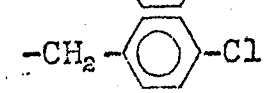 | cis | Harz | 4 |
| 12.38 | CH | 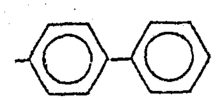 | DIII | 6 | H | H | H | H | 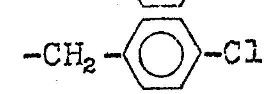 | cis | 168 | 4 |
| 12.39 | CH | $2,4-C_6H_3Cl_2$ | DIII | 6 | H | H | H | H | 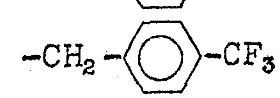 | cis | 125 | 9 |
| 12.40 | CH | $2,4-C_6H_3Cl_2$ | DIII | 7 | H | H | H | H | 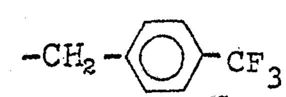 | cis | (Harz) | 9 |
| 12.41 | CH | $2,4-C_6H_3Cl_2$ | DIII | 6 | H | H | H | H | 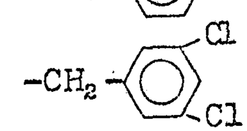 | cis | | 4 |
| 12.42 | CH | $2,4-C_6H_3Cl_2$ | DIII | 6 | H | H | H | H | 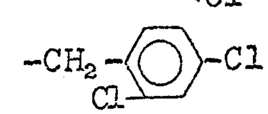 | cis | 144-145 | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.43 | CH | 2,4-$C_6H_3Cl_2$ | DIII | 6 | H | H | H | H | 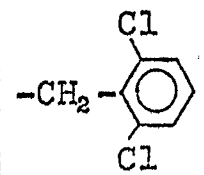 | cis | Harz | 4 |
| 12.44 | CH | 2,4-$C_6H_3Cl_2$ | DIII | 6 | H | H | H | H | 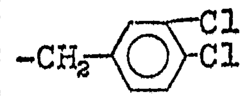 | cis | 114-115 | 4 |
| 12.45 | CH | 2,4-$C_6H_3Cl_2$ | DIII | 6 | H | H | H | H | 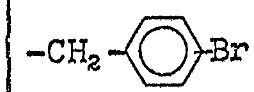 | cis | | 4 |
| 12.46 | CH | 2,4-$C_6H_3Cl_2$ | DIII | 7 | H | H | H | H | 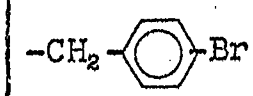 | cis | | 4 |
| 12.47 | CH | 2,4-$C_6H_3Cl_2$ | DIII | 7 | H | H | H | H | 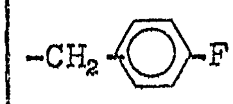 | cis | Harz | 9 |
| 12.48 | CH | 2,4-$C_6H_3Cl_2$ | DIII | 6 | H | H | H | H | 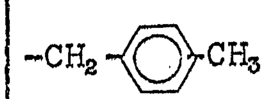 | cis | Harz | 9 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.49 | CH | 2,4-$C_6H_3Cl_2$ | DIII | 6 | H | H | H | H | 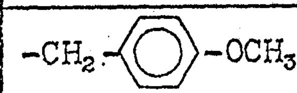 | cis | 124-126 | 9 |
| 12.50 | CH | 2,4-$C_6H_3Cl_2$ | DIII | 7 | H | H | H | H | 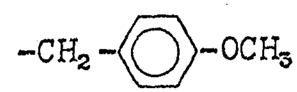 | cis | | 9 |
| 12.51 | CH | 2,4-$C_6H_3Cl_2$ | DIII | 6 | H | H | H | H | 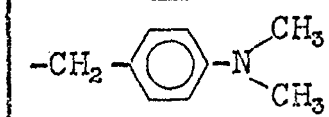 | cis | 91- 92 | 9 |
| 12.52 | CH | 2,4-$C_6H_3Cl_2$ | DIII | 7 | H | H | H | H | 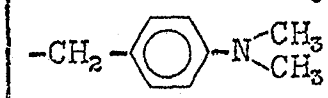 | cis | | 9 |
| 12.53 | CH | 2,4-$C_6H_3Cl_2$ | DIII | 6 | H | H | H | H | 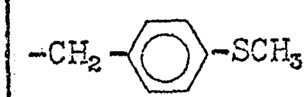 | cis | 80-82 | 4 |
| 12.54 | CH | 2,4-$C_6H_3Cl_2$ | DIII | 7 | H | H | H | H | 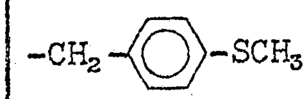 | cis | Harz | 9 |
| 12.55 | CH | 2,4-$C_6H_3Cl_2$ | DIII | 6 | H | H | H | H | 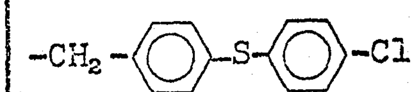 | cis | Harz | 4 |
| 12.56 | CH | 2,4-$C_6H_3Cl_2$ | DIII | 6 | H | H | H | H | 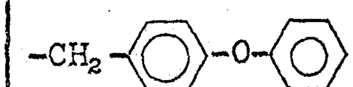 | cis | | 4 |
| 12.57 | CH | 2,4-$C_6H_3Cl_2$ | DIII | 7 | H | H | H | H | dto. | cis | | 4 |
| 12.58 | CH | 2,4-$C_6H_3Cl_2$ | DIII | 6 | H | H | H | H | 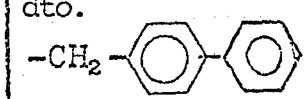 | trans | Harz | 4 |
| 12.59 | CH | 2,4-$C_6H_3Cl_2$ | DIII | 7 | H | H | H | H | 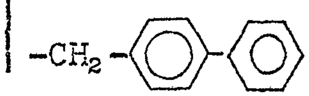 | cis | Harz | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.60 | CH | 2,4-$C_6H_3Cl_2$ | DIII | 6 | 5-$CH_3$ | 7-$CH_3$ | H | H | $-CH_2-$ 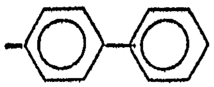 | cis | Harz | 4 |
| 12.61 | CH | 2,4-$C_6H_3Cl_2$ | DIII | 6 | 7-$OCH_3$ | H | H | H | $-CH_2-$ 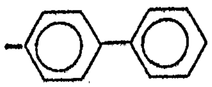 | cis | Harz | 4 |
| 12.62 | CH | 2,4-$C_6H_3Cl_2$ | DIII | 6 | H | H | H | H | $-CH_2-$ 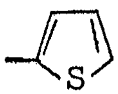 | cis | Harz | 9 |
| 12.63 | CH | 2,4-$C_6H_3Cl_2$ | DIII | 7 | H | H | H | H | $-CH_2-$ 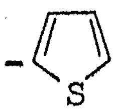 | cis | | 9 |
| 12.64 | CH | 2,4-$C_6H_3Cl_2$ | DIII | 6 | H | H | H | H | $-CH_2-$ 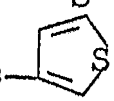 | cis | Harz | 9 |
| 12.65 | CH | 2,4-$C_6H_3Cl_2$ | DIII | 7 | H | H | H | H | $-CH_2-$ 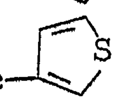 | cis | | 9 |
| 12.66 | CH | 2,4-$C_6H_3Cl_2$ | DIII | 6 | H | H | H | H | $-CH_2-$ 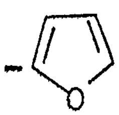 | cis | 99-101 | 4 |
| 12.67 | CH | 2,4-$C_6H_3Cl_2$ | DIII | 7 | H | H | H | H | $-CH_2-$ 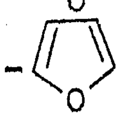 | cis | | 4 |
| 12.68 | CH | 2,4-$C_6H_3Cl_2$ | DIII | 6 | H | H | H | H | $-CH_2-$ 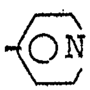 | cis | 116-118 | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.69 | CH | 2,4-$C_6H_3Cl_2$ | DIII | 6 | H | H | H | H | $-CH_2-$ 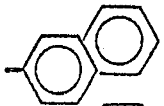 | cis | 150-152 | 4 |
| 12.70 | CH | 2,4-$C_6H_3Cl_2$ | DIII | 7 | H | H | H | H | $-CH_2-$ 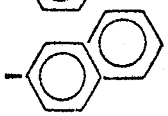 | cis | | 4 |
| 12.71 | CH | 2,4-$C_6H_3Cl_2$ | DIII | 6 | H | H | H | H | $-CH_2-$ 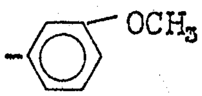 | cis | 96- 98 | 4 |
| 12.72 | CH | 2,4-$C_6H_3Cl_2$ | DIII | 6 | H | H | H | H | $-CH_2-CH_2-$ 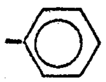 | cis | | 9 |
| 12.73 | CH | 2,4-$C_6H_3Cl_2$ | DIII | 7 | H | H | H | H | $-CH_2-CH_2-$ 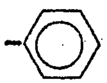 | cis | | 9 |
| 12.74 | CH | 2,4-$C_6H_3Cl_2$ | DIII | 6 | H | H | H | H | $-CH_2-CH_2-$ 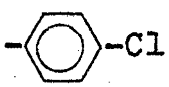 | cis | 104-106 | 4 |
| 12.75 | CH | 2,4-$C_6H_3Cl_2$ | DIII | 6 | H | H | H | H | $-CH_2-CH_2-$ 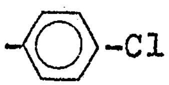 | trans | 121-123 | 4 |
| 12.76 | CH | 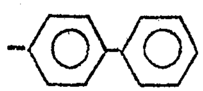 | DIII | 6 | H | H | H | H | $-CH_2-CH_2-$ 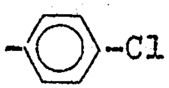 | cis | | 9 |
| 12.77 | CH | 2,4-$C_6H_3Cl_2$ | DIII | 7 | H | H | H | H | $-CH_2-CH_2-$ 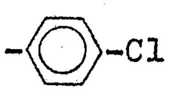 | cis | 107-109 | 9 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.78 | CH | 2,4-$C_6H_3Cl_2$ | DIII | 7 | H | H | H | H | $-CH_2-CH_2-$ 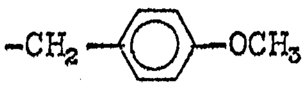 | cis | | 9 |
| 12.79 | CH | 2,4-$C_6H_3Cl_2$ | DIII | 6 | H | H | H | H | $-CH_2-CH_2-$ 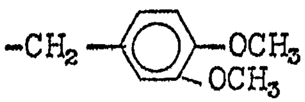 | cis | | 9 |
| 12.80 | CH | 2,4-$C_6H_3Cl_2$ | DIII | 6 | H | H | H | H | $-CH_2-CH_2-$ 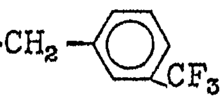 | cis | | 9 |
| 12.81 | CH | 2,4-$C_6H_3Cl_2$ | DIII | 6 | H | H | H | H | $-CH_2-CH_2-$ 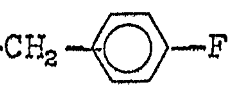 | cis | | 9 |
| 12.82 | CH | 2,4-$C_6H_3Cl_2$ | DIII | 7 | H | H | H | H | $-CH_2-CH_2-$ 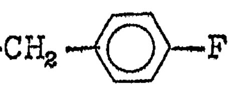 | cis | | 9 |
| 12.83 | CH | 2,4-$C_6H_3Cl_2$ | DIII | 7 | H | H | H | H | $-CH_2-CH_2-$ 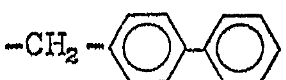 | cis | | 9 |
| 12.84 | CH | 2,4-$C_6H_3Cl_2$ | DIII | 6 | H | H | H | H | $-CH_2-CH_2-$ 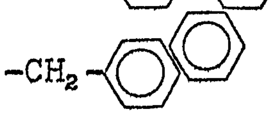 | cis | | 9 |
| 12.85 | CH | 2,4-$C_6H_3Cl_2$ | DIII | 6 | H | H | H | H | $-CH_2-CH_2-$ 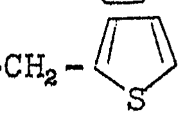 | cis | | 9 |
| 12.86 | CH | 2,4-$C_6H_3Cl_2$ | DIII | 7 | H | H | H | H | $-CH_2-CH_2-$ 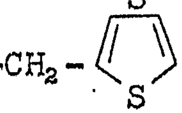 | cis | | 9 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.87 | CH | $2,4-C_6H_3Cl_2$ | DIII | 6 | H | H | H | H | $-CH_2-CH_2-$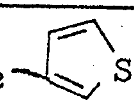 | cis | | 4 |
| 12.88 | CH | $2,4-C_6H_3Cl_2$ | DIII | 7 | H | H | H | H | $-CH_2-CH_2-$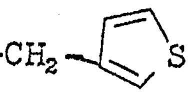 | cis | | 4 |
| 12.89 | CH | $2,4-C_6H_3Cl_2$ | DIII | 6 | H | H | H | H | $-CH_2-CH_2-$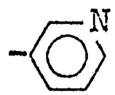 | cis | | 4 |
| 12.90 | CH | $2,4-C_6H_3Cl_2$ | DIII | 6 | H | H | H | H | $-CH_2-CH_2-CH_2-$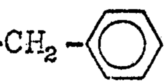 | cis | | 9 |
| 12.91 | CH | $2,4-C_6H_3Cl_2$ | DIII | 7 | H | H | H | H | dto. | cis | | 9 |
| 12.92 | CH | $2,4-C_6H_3Cl_2$ | DIII | 6 | H | H | H | H | $-CH_2-CH_2-CH_2-$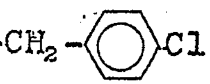 | cis | 112-114 | 4 |
| 12.93 | CH | 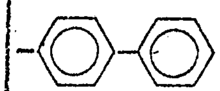 | DIII | 6 | H | H | H | H | $-CH_2-CH_2-CH_2-$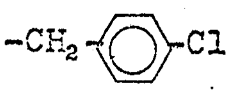 | cis | Harz | 4 |
| 12.94 | CH | $2,4-C_6H_3Cl_2$ | DIII | 7 | H | H | H | H | $-CH_2-CH_2-CH_2-$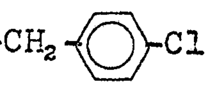 | cis | | 9 |
| 12.95 | CH | $2,4-C_6H_3Cl_2$ | DIII | 6 | H | H | H | H | $-CH_2-CH_2-CH_2-$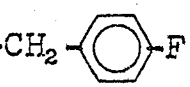 | cis | | 4 |

0121753

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.96 | CH | 2,4-$C_6H_3Cl_2$ | DIII | 7 | H | H | H | H | $-CH_2-CH_2-CH_2-$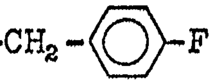 | cis | | 4 |
| 12.97 | CH | 2,4-$C_6H_3Cl_2$ | DIII | 6 | H | H | H | H | $-CH_2-CH_2-CH_2-$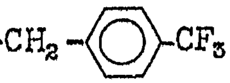 | cis | 102-103 | 4 |
| 12.98 | CH | 2,4-$C_6H_3Cl_2$ | DIII | 7 | H | H | H | H | $-CH_2-CH_2-CH_2-$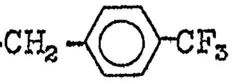 | cis | | 4 |
| 12.99 | CH | 2,4-$C_6H_3Cl_2$ | DIII | 6 | H | H | H | H | $-CH_2-CH_2-CH_2-$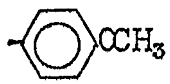 | cis | 110-111 | 4 |
| 12.100 | CH | 2,4-$C_6H_3Cl_2$ | DIII | 7 | H | H | H | H | $-CH_2-CH_2-CH_2-$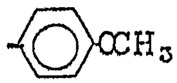 | cis | | 4 |
| 12.101 | CH | 2,4-$C_6H_3Cl_2$ | DIII | 6 | H | H | H | H | $-CH_2-CH_2-CH_2-$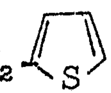 | cis | | 4 |
| 12.102 | CH | 2,4-$C_6H_3Cl_2$ | DIII | 7 | H | H | H | H | $-CH_2-CH_2-CH_2-$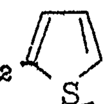 | cis | | 4 |
| 12.103 | CH | 2,4-$C_6H_3Cl_2$ | DIII | 6 | H | H | H | H | $-CH_2-CH_2-CH_2-$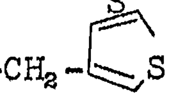 | cis | | 4 |
| 12.104 | CH | 2,4-$C_6H_3Cl_2$ | DIII | 7 | H | H | H | H | $-CH_2-CH_2-CH_2-$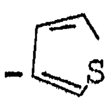 | cis | | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.105 | CH | 2,4-$C_6H_3Cl_2$ | DIII | 6 | H | H | H | H | $-CH_2-CH_2-CH_2-$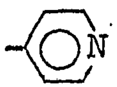 | cis | | 4 |
| 12.106 | CH | 2,4-$C_6H_3Cl_2$ | DIII | 6 | H | H | H | H | $-(CH_2)_4-$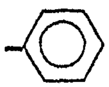 | cis | | 9 |
| 12.107 | CH | 2,4-$C_6H_3Cl_2$ | DIII | 7 | H | H | H | H | $-(CH_2)_4-$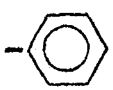 | cis | | 9 |
| 12.108 | CH | 2,4-$C_6H_3Cl_2$ | DIII | 6 | H | H | H | H | $-(CH_2)_4-$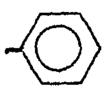$-Cl$ | cis | 101-103 | 4 |
| 12.109 | CH | 2,4-$C_6H_3Cl_2$ | DIII | 7 | H | H | H | H | $-(CH_2)_4-$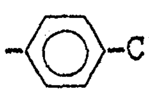$-Cl$ | cis | | 4 |
| 12.110 | CH | 2,4-$C_6H_3Cl_2$ | DIII | 6 | H | H | H | H | $-(CH_2)_4-$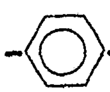$-OCH_3$ | cis | | 4 |
| 12.111 | CH | 2,4-$C_6H_3Cl_2$ | DIII | 7 | H | H | H | H | $-(CH_2)_4-$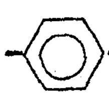$-OCH_3$ | cis | | 4 |
| 12.112 | CH | 2,4-$C_6H_3Cl_2$ | DIII | 6 | H | H | H | H | $-(CH_2)_4-$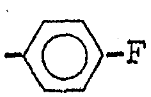$-F$ | cis | | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.113 | CH | $2,4-C_6H_3Cl_2$ | DIII | 7 | H | H | H | H | $-(CH_2)_4-$ 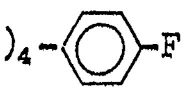 | cis | | 4 |
| 12.114 | CH | $2,4-C_6H_3Cl_2$ | DIII | 6 | H | H | H | H | $-(CH_2)_4-$ 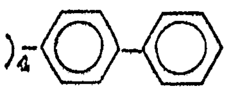 | cis | | 4 |
| 12.115 | CH | $2,4-C_6H_3Cl_2$ | DIII | 7 | H | H | H | H | $-(CH_2)_4-$ 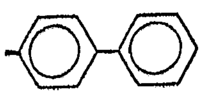 | cis | | 4 |
| 12.116 | CH | $2,4-C_6H_3Cl_2$ | DIII | 6 | H | H | H | H | $-(CH_2)_4-$ 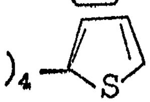 | cis | | 4 |
| 12.117 | CH | $2,4-C_6H_3Cl_2$ | DIII | 7 | H | H | H | H | $-(CH_2)_4-$ 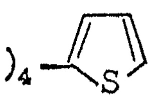 | cis | | 4 |
| 12.118 | CH | $2,4-C_6H_3Cl_2$ | DIII | 6 | H | H | H | H | $-(CH_2)_5-$ 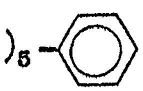 | cis | | 4 |
| 12.119 | CH | $2,4-C_6H_3Cl_2$ | DIII | 7 | H | H | H | H | $-(CH_2)_5-$ 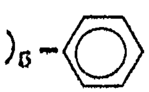 | cis | | 4 |
| 12.120 | CH | $2,4-C_6H_3Cl_2$ | DIII | 6 | H | H | H | H | $-(CH_2)_5-$ 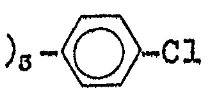 | cis | | 4 |
| 12.121 | CH | $2,4-C_6H_3Cl_2$ | DIII | 7 | H | H | H | H | $-(CH_2)_5-$ 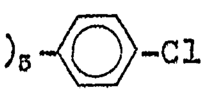 | cis | | 4 |

| Verb.-Nr. | A | Ar | Isochinolin vom Typ | V | $R_1$ | $R_2$ | $R_3$ | $R_4$ | $R_5$ | Isomerie am Dioxolanring, | Fp. /°C/ | Herstellung analog Beispiel |
|---|---|---|---|---|---|---|---|---|---|---|---|---|
| 12.122 | CH | $2,4-C_6H_3Cl_2$ | DIII | 6 | H | H | H | H | $-(CH_2)_5-$ 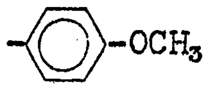 | cis | | 4 |
| 12.123 | CH | $2,4-C_6H_3Cl_2$ | DIII | 7 | H | H | H | H | $-(CH_2)_5-$ 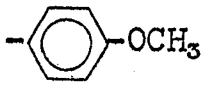 | cis | | 4 |
| 12.124 | CH | $2,4-C_6H_3Cl_2$ | DIII | 6 | H | H | H | H | $-(CH_2)_5-$ 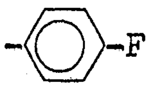 | cis | | 4 |
| 12.125 | CH | $2,4-C_6H_3Cl_2$ | DIII | 7 | H | H | H | H | $-(CH_2)_5-$ 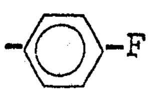 | cis | | 4 |
| 12.126 | CH | $2,4-C_6H_3Cl_2$ | DIII | 6 | H | H | H | H | $-(CH_2)_5-$ 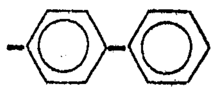 | cis | | 4 |
| 12.127 | CH | $2,4-C_6H_3Cl_2$ | DIII | 7 | H | H | H | H | $-(CH_2)_5-$ 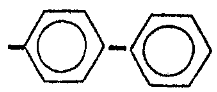 | cis | | 4 |
| 12.128 | CH | $2,4-C_6H_3Cl_2$ | DIII | 6 | H | H | H | H | $-(CH_2)_5-$ 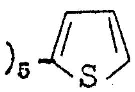 | cis | | 4 |
| 12.129 | CH | $2,4-C_6H_3Cl_2$ | DIII | 7 | H | H | H | H | $-(CH_2)_5-$ 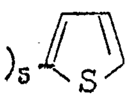 | cis | | 4 |
| 12.130 | CH | $2,4-C_6H_3Cl_2$ | DIII | 7 | H | H | H | H | H | cis | | 1 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.131 | CH | 2,4-$C_6H_3Cl_2$ | DIII | 6 | H | H | H | H | $-COCH_3$ | cis | Harz | 4 |
| 12.132 | CH | 2,4-$C_6H_3Cl_2$ | DIII | 7 | H | H | H | H | $-COCH_3$ | cis | | 4 |
| 12.133 | CH | 2,4-$C_6H_3Cl_2$ | DIII | 6 | H | H | H | H | 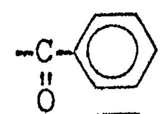 | cis | | 4 |
| 12.134 | CH | 2,4-$C_6H_3Cl_2$ | DIII | 7 | H | H | H | H | 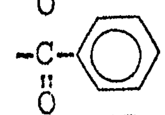 | cis | | 4 |
| 12.135 | CH | 2,4-$C_6H_3Cl_2$ | DIII | 6 | H | H | H | H | 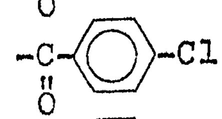 | cis | Harz | 4 |
| 12.136 | CH | 2,4-$C_6H_3Cl_2$ | DIII | 7 | H | H | H | H | 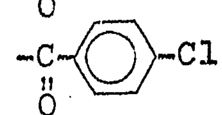 | cis | | 4 |
| 12.137 | CH | 2,4-$C_6H_3Cl_2$ | DIII | 6 | H | H | H | H | 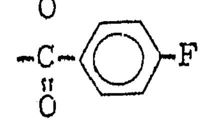 | cis | | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.138 | CH | $2,4-C_6H_3Cl_2$ | DIII | 7 | H | H | H | H | 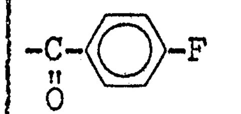 | cis | | 4 |
| 12.139 | CH | $2,4-C_6H_3Cl_2$ | DIII | 6 | H | H | H | H | 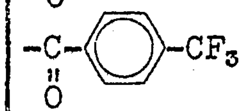 | cis | | 4 |
| 12.140 | CH | $2,4-C_6H_3Cl_2$ | DIII | 7 | H | H | H | H | 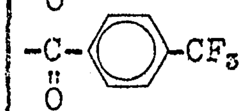 | cis | | 4 |
| 12.141 | CH | $2,4-C_6H_3Cl_2$ | DIII | 6 | H | H | H | H | 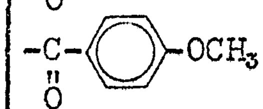 | cis | Harz | 4 |
| 12.142 | CH | $2,4-C_6H_3Cl_2$ | DIII | 7 | H | H | H | H | 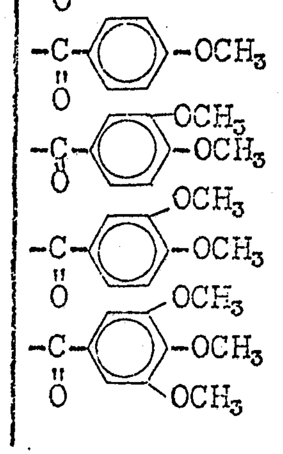 | cis | | 4 |
| 12.143 | CH | $2,4-C_6H_3Cl_2$ | DIII | 6 | H | H | H | H | | cis | | 4 |
| 12.144 | CH | $2,4-C_6H_3Cl_2$ | DIII | 7 | H | H | H | H | | cis | | 4 |
| 12.145 | CH | $2,4-C_6H_3Cl_2$ | DIII | 6 | H | H | H | H | | cis | | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. [°C] | Herstellung analog Beispiel |
|---|---|---|---|---|---|---|---|---|---|---|---|---|
| 12.146 | CH | 2,4-$C_6H_3Cl_2$ | DIII | 7 | H | H | H | H | 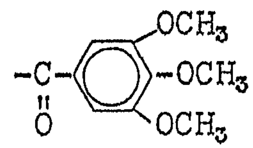 | cis | | 4 |
| 12.147 | CH | 2,4-$C_6H_3Cl_2$ | DIII | 6 | H | H | H | H | 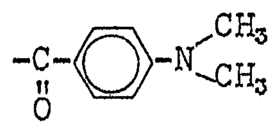 | cis | Harz | 4 |
| 12.148 | CH | 2,4-$C_6H_3Cl_2$ | DIII | 7 | H | H | H | H | 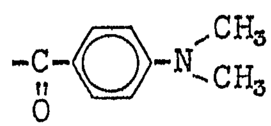 | cis | | 4 |
| 12.149 | CH | 2,4-$C_6H_3Cl_2$ | DIII | 6 | H | H | H | H | 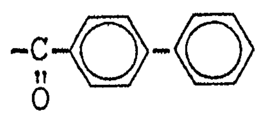 | cis | | 4 |
| 12.150 | CH | 2,4-$C_6H_3Cl_2$ | DIII | 7 | H | H | H | H | 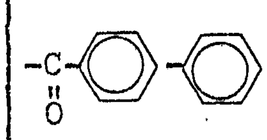 | cis | | 4 |
| 12.151 | CH | 2,4-$C_6H_3Cl_2$ | DIII | 6 | H | H | H | H | 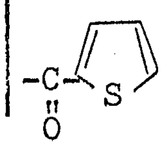 | cis | | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.152 | CH | 2,4-$C_6H_3Cl_2$ | DIII | 7 | H | H | H | H | 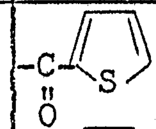 | cis | | 4 |
| 12.153 | CH | 2,4-$C_6H_3Cl_2$ | DIII | 6 | H | H | H | H | 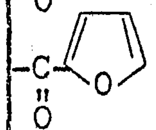 | cis | | 4 |
| 12.154 | CH | 2,4-$C_6H_3Cl_2$ | DIII | 7 | H | H | H | H | 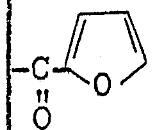 | cis | | 4 |
| 12.155 | CH | 2,4-$C_6H_3Cl_2$ | DIII | 6 | H | H | H | H | 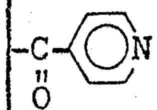 | cis | | 4 |
| 12.156 | CH | 2,4-$C_6H_3Cl_2$ | DIII | 7 | H | H | H | H | 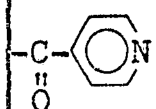 | cis | | 4 |
| 12.157 | CH | 2,4-$C_6H_3Cl_2$ | DIII | 6 | H | H | H | H | 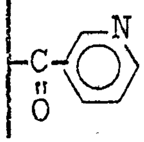 | cis | | 4 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.158 | CH | 2,4-$C_6H_3Cl_2$ | DIII | 7 | H | H | H | H | 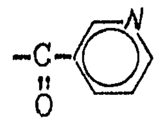 | cis | | 4 |
| 12.159 | CH | 2,4-$C_6H_3Cl_2$ | DIII | 6 | H | H | H | H | 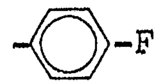 | cis | | 9 |
| 12.160 | CH | 2,4-$C_6H_3Cl_2$ | DIII | 6 | H | H | H | H | 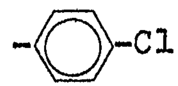 | cis | 117-119 | 9 |
| 12.161 | CH | 2,4-$C_6H_3Cl_2$ | DIII | 6 | H | H | H | H | 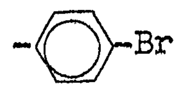 | cis | | 9 |
| 12.162 | CH | 2,4-$C_6H_3Cl_2$ | DIII | 6 | H | H | H | H | 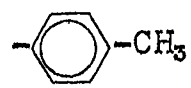 | cis | | 9 |
| 12.163 | CH | 2,4-$C_6H_3Cl_2$ | DIII | 6 | H | H | H | H | 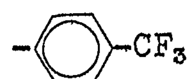 | cis | | 9 |
| 12.164 | CH | 2,4-$C_6H_3Cl_2$ | DIII | 6 | H | H | H | H | 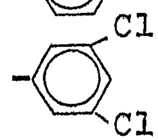 | cis | | 9 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.165 | CH | $2,4-C_6H_3Cl_2$ | DIII | 6 | H | H | H | H | 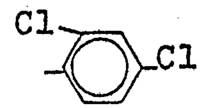 | cis | | 9 |
| 12.166 | CH | $2,4-C_6H_3Cl_2$ | DIII | 6 | H | H | H | H | 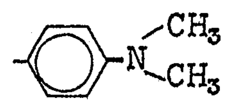 | cis | | 9 |
| 12.167 | CH | $2,4-C_6H_3Cl_2$ | DIII | 6 | H | H | H | H | 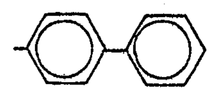 | cis | | 9 |
| 12.168 | CH | $2,4-C_6H_3Cl_2$ | DIII | 6 | H | H | H | H | 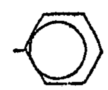 | cis | | 9 |

| Verb.-Nr. | A | Ar | Isochinolin vom Typ | V | $R_1$ | $R_2$ | $R_3$ | $R_4$ | $R_5$ | Isomerie am Dioxolanring | Fp. /°C/ | Herstellung analog Beispiel |
|---|---|---|---|---|---|---|---|---|---|---|---|---|
| 12.169 | CH | $2,4-C_6H_3Cl_2$ | DIII | 6 | H | H | H | H | 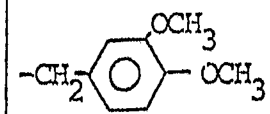 | cis | | 9 |
| 12.1 70 | CH | $2,4-C_6H_3Cl_2$ | DIII | 7 | H | H | H | H | 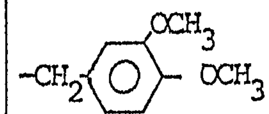 | cis | | 9 |
| 12.171 | CH | $2,4-C_6H_3Cl_2$ | DIII | 6 | H | H | H | H | 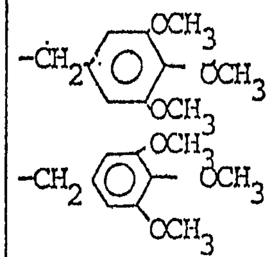 | cis | 13 1 | 9 |
| 12.172 | CH | $2,4-C_6H_3Cl_2$ | DIII | 7 | H | H | H | H | | cis | | 9 |
| 12.173 | CH | $2,4-C_6H_3Cl_2$ | DIII | 6 | H | H | H | H | 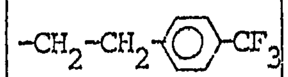 | cis | 115-116 | 9 |
| 12.174 | CH | $2,4-C_6H_3Cl_2$ | DIII | 7 | H | H | H | H | 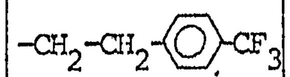 | cis | | 9 |
| 12.175 | CH | $2,4-C_6H_3Cl_2$ | DIII | 6 | H | H | H | H | 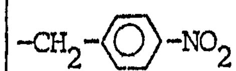 | cis | | 9 |
| 12.176 | CH | $2,4-C_6H_3Cl_2$ | DIII | 7 | H | H | H | H | 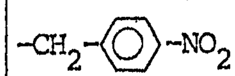 | cis | | 9 |

| Verb.Nr. | A | Ar | Iso-chino-lin vom Typ | V | $R^1$ | $R^2$ | $R^3$ | $R^4$ | $R^5$ | Isomerie am Dioxo-lanring | Fp. /°C/ | Herstellung analog Beispiel |
|---|---|---|---|---|---|---|---|---|---|---|---|---|
| 12.177 | CH | 2,4-$C_6H_3Cl_2$ | D III | 6 | H | H | H | H | 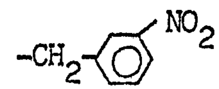 | cis | | 9 |
| 12.178 | CH | 2,4-$C_6H_3Cl_2$ | D III | 7 | H | H | H | H | 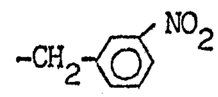 | cis | | 9 |
| 12.179 | CH | 2,4-$C_6H_3Cl_2$ | D III | 6 | H | H | H | H | 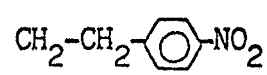 | cis | | 9 |
| 12.180 | CH | 2,4-$C_6H_3Cl_2$ | D III | 7 | H | H | H | H | 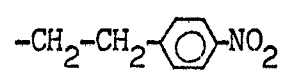 | cis | | 9 |
| 12.181 | CH | 2,4-$C_6H_3Cl_2$ | D III | 6 | H | H | H | H | 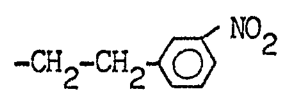 | cis | | 9 |
| 12.182 | CH | 2,4-$C_6H_3Cl_2$ | D III | 7 | H | H | H | H | 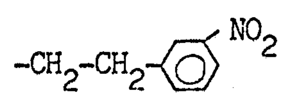 | cis | | 9 |
| 12.183 | CH | 2,4-$C_6H_3Cl_2$ | D III | 6 | H | H | H | H | 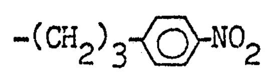 | cis | | 9 |
| 12.184 | CH | 2,5-$C_6H_3Cl_2$ | D III | 7 | H | H | H | H | 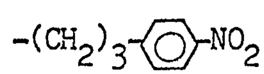 | cis | | 9 |
| 12.185 | CH | 2,4-$C_6H_3Cl_2$ | D III | 6 | H | H | H | H | 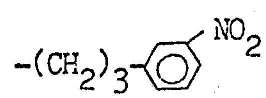 | cis | | 9 |
| 12.186 | CH | 2,4-$C_6H_2Cl_2$ | D III | 7 | H | H | H | H | 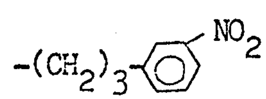 | cis | | 9 |
| 12.187 | CH | 2,4-$C_6H_3Cl_2$ | D III | 7 | H | H | H | H | 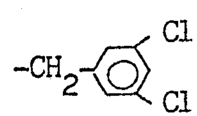 | cis | | 9 |

| Verb.Nr. | A | Ar | Iso-chino-lin vom Typ | V | $R^1$ | $R^2$ | $R^3$ | $R^4$ | $R^5$ | Isomerie am Dioxolanring | Fp. [°C] | Herstellung analog Beispiel |
|---|---|---|---|---|---|---|---|---|---|---|---|---|
| 12.188 | CH | $2,4-C_6H_3Cl_2$ | D III | 7 | H | H | H | H | 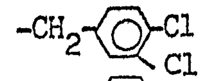 | cis | | 9 |
| 12.189 | CH | $2,4-C_6H_3Cl_2$ | D III | 7 | H | H | H | H | 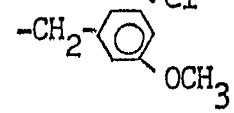 | cis | | 9 |
| 12.190 | CH | $2,4-C_6H_3Cl_2$ | D III | 7 | H | H | H | H | 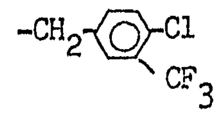 | cis | | 9 |
| 12.191 | CH | $2,4-C_6H_3Cl_2$ | D III | 7 | H | H | H | H | 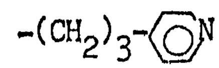 | cis | | 9 |
| 12.192 | CH | $2,4-C_6H_3Cl_2$ | D III | 6 | H | H | H | H | 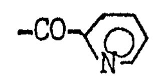 | cis | | 9 |
| 12.193 | CH | $2,4-C_6H_3Cl_2$ | D III | 7 | H | H | H | H | 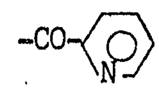 | cis | | 9 |
| 12.194 | CH | $2,4-C_6H_3Cl_2$ | D III | 7 | H | H | H | H | 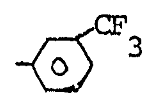 | cis | 93-95 | 9 |
| 12.195 | N | $2,4-C_6H_3Cl_2$ | D III | 6 | H | H | H | H | 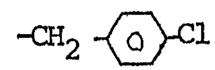 | cis | 105-107° | 9 |
| 12.196 | N | $2,4-C_6H_3Cl_2$ | D III | 6 | H | H | H | H | 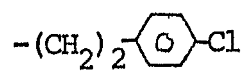 | cis | 98-100 | 9 |
| 12.197 | N | $2,4-C_6H_3Cl_2$ | D III | 6 | H | H | H | H | 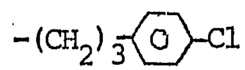 | cis | 91-93 | 9 |
| 12.198 | N | $2,4-C_6H_3Cl_2$ | D III | 6 | H | H | H | H | 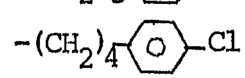 | cis | 93-95 | 9 |
| 12.199 | N | $2,4-C_6H_3Cl_2$ | D III | 6 | H | H | H | H | 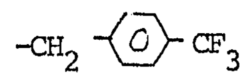 | cis | 110-112 | 9 |
| 12.200 | N | $2,4-C_6H_3Cl_2$ | D III | 6 | H | H | H | H | 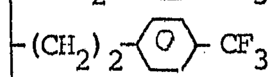 | cis | 105-107 | 9 |

III. Zur Herstellung von Verbindungen der allgemeinen Formel (III).

Beispiel 13

6-Hydroxy-2-(4-chlorbenzoyl)-1,2,3,4-tetrahydroisochinolin

Ausgangsprodukt zur Herstellung von Verbindung 12.135 in Tabelle 1

1,2 g 6-Hydroxy-1,2,3,4-tetrahydroisochinolin werden in 50 ml Methylenchlorid suspendiert, 1,2 ml Triethylamin zugegeben und eine Lösung von 1,4 g 4-Chlorbenzoylchlorid in 10 ml Methylenchlorid unter Rühren und Kühlen zugetropft. Nach Stehen über Nacht wird mit 2n Salzsäure und Wasser ausgeschüttelt, die organische Phase getrocknet und einrotiert. Es bleibt ein farbloses Öl zurück, das bei Verreiben mit Ethylacetat kristallisiert.
Ausbeute: 1,8 g (78 % d. Th.) Fp.: 170°

Beispiel 14

6-Hydroxy-2-(3-methoxybenzyl)-1,2,3,4-tetrahydroisochinolin

Ausgangsprodukt zur Herstellung von Verbindung 12.71 in Tabelle 1

4,3 g 6-Hydroxy-1,2,3,4-tetrahydro-isochinolin-Hydrobromid, 3,1 g 3-Methoxybenzylchlorid und 2,8 g fein pulverisiertes Kaliumcarbonat werden zunächst 2 Stunden bei Raumtemperatur, dann 5 Stunden bei 80°C gerührt. Nach dem Abkühlen wird das Lösungsmittel im Vakuum abgezogen, der Rückstand mit Wasser/Methylenchlorid aufgenommen, die organische Phase nochmals mit Wasser geschüttelt und über Magnesiumsulfat getrocknet. Nach dem Einrotieren verbleibt ein gelbes Öl, das nach dem Verreiben mit Ethylacetat kristallisiert.
Ausbeute: 2,6 g (52 % d. Th.) Fp.: 132 - 134°C.

Beispiel 15

6-Hydroxy-2-(thienyl-2-ylmethyl)-1,2,3,4-tetrahydro-isochinolin

Ausgangsprodukt zur Herstellung von Verbindung 12.62 in Tabelle 1

Eine Lösung von 1,5 g 6-Hydroxy-1,2,3,4-tetrahydroisochinolin, 1,2 g Thiophen-2-aldehyd und 1,8 g Toluolsulfonsäure-monohydrat in 30 ml Ethanol wird mit 0,6 g Natriumcyanoborhydrid versetzt. Man rührt 8 Stunden bei Raumtemperatur, zieht das Lösungsmittel ab und nimmt den Rückstand mit Wasser/Methylenchlorid auf. Die organische Phase wird nochmals mit Wasser geschüttelt, getrocknet und einrotiert. Das ölige Rohprodut wird durch Chromatographie an Kieselgel mit einem Gemisch von Ethylacetat/Methylenchlorid im Verhältnis 1 : 2 gereinigt. Man erhält 1,7 g eines farblosen Öls, das beim Stehen kristallisiert (69 % d. Th.).
Fp.: 110 - 112$^{o}$C

Beispiel 16

6-Hydroxy-2-(4-phenylbenzyl)-1,2,3,4-tetrahydro-isochinolin

Ausgangsprodukt zur Herstellung von Verbindung 12.58 in Tabelle 1

a) 6-Methoxy-2-(4-phenylbenzyl)-1,2,3,4-tetrahydroisochinolin

4,0 g 6-Methoxy-1,2,3,4-tetrahydro-isochinolinhydrochlorid, 4,1 g 4-Chlormethylbiphenyl und 6,0 f fein pulverisiertes Kaliumcarbonat werden in 50 ml Dimethylformamid 5 Stunden bei 80$^{o}$ gerührt. Man zieht das Lösungsmittel im Vakuum ab, nimmt mit Wasser/Methylenchlorid auf, schüttelt die organische Phase zweimal mit Wasser, trocknet die organische

Phase mit Magnesiumsulfat und rotiert ein. Das nur schwach verunreinigte Rohprodukt kann ohne weitere Reinigung weiter umgesetzt werden. Eine aus Ethylacetat umkristallisierte Probe hat den Schmelzpunkt 120°.

b) 6-Hydroxy-2-(4-phenylbenzyl)-1,2,3,4-tetrahydro-isochinolin

Die obige Methoxy-Verbindung wird 6 Stunden mit einem Gemisch aus 50 ml Eisessig und 48 %-iger wäßriger Bromwasserstoffsäure im Volumenverhältnis 1 : 1 unter Rückfluß erhitzt. Nach dem Abkühlen wird das Säuregemisch am Wasserstrahlvakuum abdestilliert, der feste Rückstand wird pulverisiert und mit wäßriger Ammoniaklösung verrührt, abgesaugt und mit Wasser nachgewaschen. Das Rohprodukt wird mit Ethanol aufgekocht und nach dem Abkühlen abgesaugt. Man erhält 5,0 g farblosen Feststoff vom Schmelzpunkt 235 - 237° (79 % d. Th.).

Beispiel 17

6-Hydroxy-2-n-octyl-1,2,3,4-tetrahydro-isochinolin-hydrobromid

Ausgangsprodukt zur Herstellung von Verbindung Nr. 12.30 in Tabelle 1

a) 6 Methoxy-2-capryloyl-1,2,3,4-tetrahydroisochinolin

4,0 g 6-Methoxy-1,2,3,4-tetrahydro-isochinolin und 3,5 ml Triethylamin werden in 50 ml Methylenchlorid vorgelegt und eine Lösung von 4,0 g Octansäurechlorid in 10 ml Methylenchlorid unter Rühren und Kühlen zugetropft. Man rührt 1 Stunde bei Raumtemperatur nach, schüttelt das Gemisch mit 1 n Salzsäure, Sodalösung und Wasser, trocknet die organische Phase über Magnesiumsulfat und rotiert ein. Das Rohprodukt (6,7 g farbloses Öl) wird ohne Reinigung weiter umgesetzt.

b) 6-Methoxy-2-n-octyl-1,2,3,4-tetrahydro-isochinolin

Das unter a) hergestellte Säureamid wird in 50 ml trockenem Diethylether gelöst zu einer Suspension von 1,0 g Lithiumaluminiumhydrid in 100 ml trockenem Diethylether getropft. Man erhitzt noch 3 Stunden unter Rückfluß, zersetzt vorsichtig unter Kühlen mit 5 ml Wasser und filtriert vom anorganischen Material ab. Das nach dem Abziehen des Lösungsmittels zurückbleibende Öl wird ohne Reinigung weiter umgesetzt.

c) 6-Hydroxy-2-n-octyl-1,2,3,4-tetrahydro-isochinolin-hydrobromid

5,8 g der unter b) hergestellten Verbindung werden mit einem Gemisch aus 100 ml Eisessig und 48 %-iger wäßriger Bromwasserstoffsäure im Valumenverhältnis 1:1 16 Stunden unter Rückfluß erhitzt. Nach dem Abkühlen wird das Säuregemisch im Wasserstrahlvakuum abdestilliert und der Rückstand mit wenig Ethanol aufgekocht. Beim Abkühlen und Anreiben kristallisieren 4,0 g farblose Kristalle aus (47 % d. Th. über alle Stufen) Fp.: 165 - 166°.

Beispiel 18

6'-Hydroxy-1',2',3',4'-tetrahydro-spiro[tetrahydrothiopyran-4,1'-isochinolin]

Ausgangsprodukt für Beispiel 4

2,0 g 6-Hydroxy-N-methyl-ß-phenethylamin und 1,6 g - Tetrahydrothiopyran-4-on werden in 30 ml Ethanol 8 Stunden unter Rückfluß erhitzt. Beim Abkühlen des Gemisches kristallisiert 1,4 g farbloses Produkt aus (45 % d. Th.) Fp. 211 - 213°.

Beispiel 19

7-Hydroxy-2-(4-chlorbenzyl)-1,2,3,4-tetrahydro-isochinolin

Ausgangsprodukt zur Herstellung von Verbindung 12.37 in Tabelle 1

2,9 g 7-Hydroxy-isochinolin werden mit 3,5 g 4-Chlorbenzylchlorid in 30 ml Ethanol 8 Stunden unter Rückfluß erhitzt. Nach Stehen über Nacht kristallisieren 5,7 g Isochinoliniumsalz aus (Fp.: 273-274°). Dieses Salz wird in 200 ml Methanol/Wasser 9 : 1 gelöst und unter Kühlung 6 g Natriumborhydrid zugegeben. Nach Ende der Wasserstoffentwicklung wird noch 15 Minuten unter Rückfluß erhitzt. Man engt auf 1/3 des Volumens ein, gießt in 200 ml Wasser, gibt Ammoniumchlorid bis zur Sättigung zu und filtriert den ausgefallenen Feststoff ab. Umkristallisation aus Ethanol ergibt 3,6 g farblose Kristalle (67 % d. Th.) Fp.: 177 - 178°.

Beispiel 20

6-Hydroxy-2-(4-chlorphenyl)-1,2,3,4-tetrahydro-isochinolin

Ausgangsprodukt zur Herstellung von Verbindung 12.159 in Tabelle 1

a) N-(4-chlorphenyl)-2-(3-methoxyphenyl)-ethylamin

22,1 g N-(4-chlorphenyl)-3-methoxyphenyl-acetamid werden mit Lithiumaluminiumhydrid in THF reduziert. Nach Zersetzen mit Wasser, Filtrieren, Trocknen und Einrotieren wird der ölige Rückstand in Diethylether aufgenommen und das gewünschte Produkt als Hydrochlorid ausgefällt.
Ausbeute: 16,6 g farbl. Pulver (70 % d. Th.)
Fp.: 149 - 150°

b) 2-(4-Chlorphenyl)-6-methoxy-1,2,3,4-tetrahydro-isochinolin

14,4 g des unter a) beschriebenen Produktes, 6 ml 35 %-ige wäßrige Formaldehydlösung und 10 ml konz. Salzsäure werden in 20 ml Methanol 1 Tag unter Rückfluß erhitzt. Beim Abdestillieren des Methanols kristallisiert das Produkt aus. Nach Abkühlen wird abgesaugt und das erhaltene Produkt (13,5 g, 91 % d. Th.) ohne weitere Reinigung unter c) weiter umgesetzt.

c) 6-Hydroxy-2-(4-chlorphenyl)-1,2,3,4-tetrahydro-isochinolin

7,0 g der unter b) hergestellten Verbindung werden mit einem Gemisch aus 50 ml Eisessig/Bromwasserstoffsäure (48 %-ig) im Volumenverhältnis 1:1 8 Stunden unter Rückfluß erhitzt. Nach Abrotieren der Säure wird mit Methylenchlorid/verd. Ammoniak aufgenommen und die organische Phase getrocknet und einrotiert. Der ölige Rückstand wird an Kieselgel mit einem Gemisch aus Methylenchlorid/Essigester im Vehältnis 19 : 1 chromatographiert. Man erhält 2,2 g schwach gelbe Kristalle (38 % d.Th.) Fp.: 122 - 123°.

Beispiel 21

6-Hydroxy-2-acetyl-1,2,3,4-tetrahydro-isochinolin

Ausgangsprodukt zur Herstellung von Verbindung 12.131 in Tabelle 1

5,0 g 6-Hydroxy-1,2,3,4-tetrahydro-isochinolin werden mit Acetanhydrid oder Acetylchlorid/Triethylamin in die Bisacetylverbindung überführt und letztere ohne Reinigung in 50 ml Ethanol mit 10 ml Diethylamin 6 Stunden unter Rückfluß erhitzt. Man rotiert den Ansatz im Vakuum ein und chromatographiert den Rückstand an Kieselgel mit Essigester/

Methanol im Verhältnis 9 : 1. Man erhält ein gelbes Öl das beim Verreiben mit Diethylether kristallisiert. Ausbeute: 5,2 g (78 % d. Th.) Fp.: 129 - 130°.

Beispiel 22

6-Hydroxy-2-[3-(4-methoxyphenyl)-propyl]-1,2,3,4-tetrahydro-isochinolin

5,4 g 6-Hydroxy-1,2,3,4-tetrahydro-isochinolin werden mit 14,2 g 4-Methoxy-dihydrozimtsäurechlorid in Methylenchlorid in Gegenwart von 7,5 g Triethylamin zweifach acyliert, der Ansatz mit Wasser ausgeschüttelt, getrocknet und einrotiert. Das Rohprodukt wird ohne weitere Reinigung in Tetrahydrofuran gelöst und unter Kühlung zu einer Lösung von 1,5 g Lithiumaluminiumhydrid in trockenem Tetrahydrofuran getropft. Nach Zersetzung mit Wasser wird vom anorganischen Material abfiltriert und das Filtrat getrocknet. Der Filterrückstand wird nach Trocknen zweimal mit Methanol ausgekocht und Methanol und Tetrahydrofuran zusammen einrotiert. Man erhält nach Verreiben des öligen Rückstands mit Essigester 3,5 g farbloses Produkt (33 % d. Th.).
Fp.: 163 - 164°.

Beispiel 23

Analog den Beispielen 13 - 22, jedoch unter Verwendung äquivalenter Mengen der entsprechenden Ausgangsverbindungen, können die folgenden in Tabelle 2 aufgeführten Verbindungen der allgemeinen Formel (III) hergestellt werden.

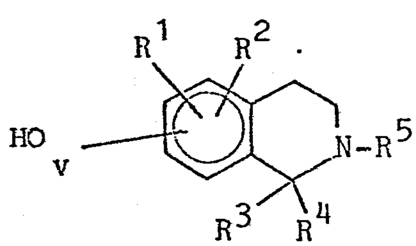

(III)

v = Position der Hydroxygruppe am Isochinolin.

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.1 | 6 | H | H | $C_2H_5$ | H | $CH_3$ | 98-100 | 18 | 12.10 |
| 23.2 | 6 | H | H | $CH_3$ | $CH_3$ | $CH_3$ | | | 12.11 |
| 23.3 | 6 | H | H | $-(CH_2)_5-$ | | $CH_3$ | 175-177 | 18 | 12.12 |
| 23.4 | 6 | H | H | $-(CH_2)_2-O-(CH_2)_2-$ | | $CH_3$ | | | 12.13 |
| 23.5 | 6 | H | H | $-(CH_2)_2-N(CH_3)-(CH_2)_2$ | | $CH_3$ | Harz | 18 | 12.14 |
| 23.6 | 6 | H | H | $-(CH_2)_2-N_3(CH_2C_6H_5)-(CH_2)_2-$ | | $CH_3$ | Harz | 18 | 12.15 |
| 23.7 | 6 | H | H | $-(CH_2)_2-N(COCH_3)-(CH_2)_2-$ | | $CH_3$ | 270° Zers. | 18 | 12.16 |
| 23.8 | 6 | H | H | $-(CH_2)_2-N(COC_6H_5)-(CH_2)_2-$ | | $CH_3$ | 237-238 | 18 | 12.17 |
| 23.9 | 6 | H | H | $-(CH_2)_2-N(COOC_2H_5)-(CH_2)_2-$ | | $CH_3$ | Harz | 18 | 12.18 |
| 23.10 | 6 | H | H | 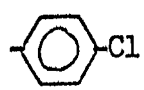 | H | $CH_3$ | 175 | 18 | 12.19 |
| 23.11 | 6 | H | H | 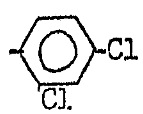 | H | $CH_3$ | Harz | 18 | 12.20 |
| 23.12 | 6 | H | H | 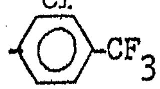 | H | $CH_3$ | | 18 | 12.21 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.13 | 6 | H | H | 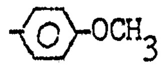 | H | $CH_3$ | Harz | 18 | 12.22 |
| 23.14 | 6 | H | H | 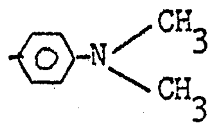 | H | $CH_3$ | Harz | 18 | 12.23 |
| 23.15 | 6 | H | H | 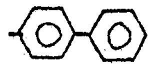 | H | $CH_3$ | 230°C Zers. | 18 | 12.24 |
| 23.16 | 6 | H | H | 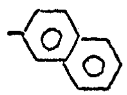 | H | $CH_3$ | 160 - 162 | 18 | 12.25 |
| 23.17 | 6 | H | H | 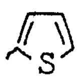 | H | $CH_3$ | 163 - 165 | 18 | 12.26 |
| 23.18 | 6 | 5-$CH_3$ | 7-$CH_3$ | H | H | $CH_3$ | 115 - 117 | 14 | 12.28 |
| 23.19 | 6 | H | H | H | | $-nC_4H_9$ | | 17 | 12.29 |
| 23.20 | 6 | H | H | H | H | $-nC_{12}H_{15}$ | | 17 | 12.31 |
| 23.21 | 6 | H | H | H | H | $-CH_2-CH=CH_2$ | 142 - 145 | 14 | 2 |
| 23.22 | 6 | H | H | H | H | $-CH_2-C\equiv CH$ | 173 - 174 | 14 | 12.32 |
| 23.23 | 6 | H | H | H | H | $-CH_2-$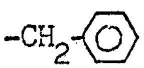 | 172 - 174 | 14 | 12.33 |
| 23.24 | 7 | H | H | H | H | $-CH_2-$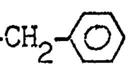 | | 19 | 13.34 |
| 23.25 | 5 | H | H | H | H | $-CH_2-$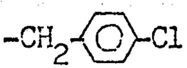 | 181 - 182 | 19 | 12.35 |
| 23.26 | 6 | H | H | H | H | $-CH_2-$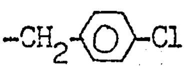 | 181 - 183 | 14 | 12.36, 12.38 |
| 23.27 | 6 | H | H | H | H | $-CH_2-$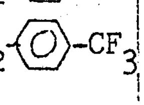 | 253 - 255 | 15 | 12.39 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.28 | 7 | H | H | H | H | 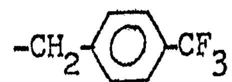 | | 15 | 12.40 |
| 23.29 | 6 | H | H | H | H | 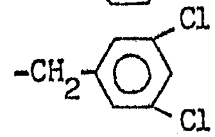 | | 14 | 12.41 |
| 23.30 | 6 | H | H | H | H | 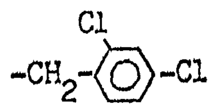 | 160 - 162 | 16 | 12.42 |
| 23.31 | 6 | H | H | H | H | 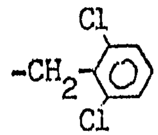 | | 16 | 12.43 |
| 23.32 | 6 | H | H | H | H | 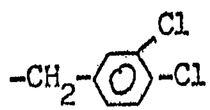 | | 14 | 12.44 |
| 23.33 | 6 | H | H | H | H | 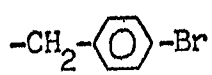 | | 14 | 12.45 |
| 23.34 | 7 | H | H | H | H | 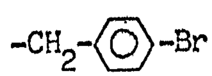 | | 19 | 12.46 |
| 23.35 | 7 | H | H | H | H | 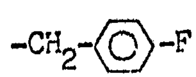 | 141-142 | 19 | 12.47 |
| 23.36 | 6 | H | H | H | H | 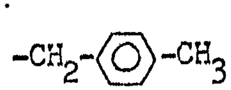 | 156-158 | 16 | 12.48 |

0121753

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.37 | 6 | H | H | H | H | 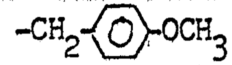 | | 15 | 12.49 |
| 23.38 | 7 | H | H | H | H | 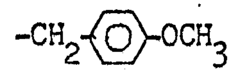 | | 15 | 12.50 |
| 23.39 | 6 | H | H | H | H | 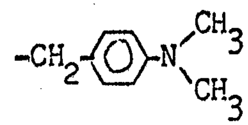 | 194-195 | 15 | 12.51 |
| 23.40 | 7 | H | H | H | H | 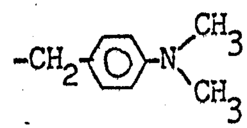 | | 15 | 12.52 |
| 23.41 | 6 | H | H | H | H | 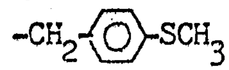 | 203-204 | 15 | 12.53 |
| 23.42 | 7 | H | H | H | H | 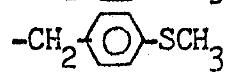 | | 15 | 12.54 |
| 23.43 | 6 | H | H | H | H | 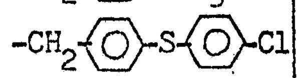 | 196-198 | 15 | 12.55 |
| 23.44 | 6 | H | H | H | H | 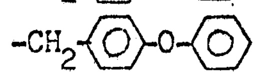 | | 15 | 12.56 |
| 23.45 | 7 | H | H | H | H | 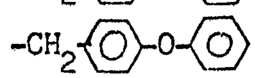 | | 15 | 12.57 |
| 23.46 | 6 | 5-$CH_3$ | 7-$CH_3$ | H | H | 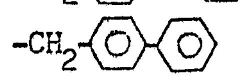 | 178-180 | 14 | 12.60 |
| 23.47 | 6 | 7-$OCH_3$ | H | H | H | 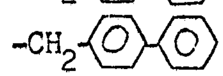 | 173-175 | 14 | 12.61 |
| 23.48 | 7 | H | H | H | H | 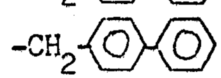 | 217-219 | 19 | 12.59 |
| 23.49 | 7 | H | H | H | H | 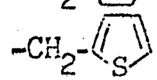 | | 15 | 12.63 |
| 23.50 | 6 | H | H | H | H | 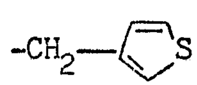 | 162 | 15 | 12.64 |
| 23.51 | 7 | H | H | H | H | 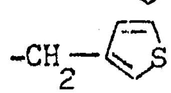 | | 15 | 12.65 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.52 | 6 | H | H | H | H | 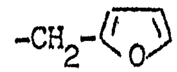 | Harz | 15 | 12.66 |
| 23.53 | 7 | H | H | H | H | 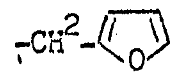 | | 15 | 12.67 |
| 23.54 | 6 | H | H | H | H | 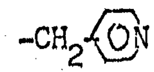 | 200-203 | 14 | 12.68 |
| 23.55 | 6 | H | H | H | H | 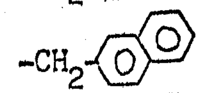 | 217-218 | 14 | 12.69 |
| 23.56 | 7 | H | H | H | H | 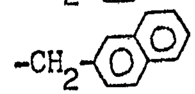 | | 19 | 12.70 |
| 23.57 | 6 | H | H | H | H | 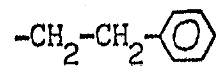 | | 17 | 12.72 |
| 23.58 | 7 | H | H | H | H | 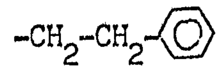 | | 17 | 12.73 |
| 23.59 | 6 | H | H | H | H | 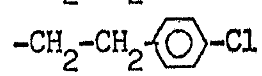 | 181-182 | 17 | 12.74, 11.75, 12.76 |
| 23.60 | 7 | H | H | H | H | 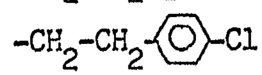 | 191-192 | 17 | 12.77 |
| 23.61 | 6 | H | H | H | H | 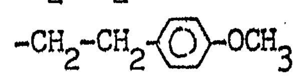 | 180-183 | 22 | 9 |
| 23.62 | 7 | H | H | H | H | 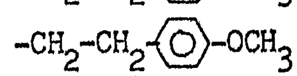 | | 22 | 12.78 |
| 23.63 | 6 | H | H | H | H | 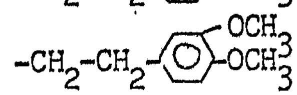 | | 22 | 12.79 |
| 23.64 | 6 | H | H | H | H | 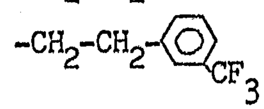 | | 17 | 12.80 |
| 23.65 | 6 | H | H | H | H | 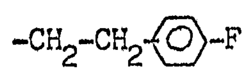 | | 17 | 12.81 |
| 23.66 | 7 | H | H | H | H | 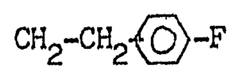 | | 17 | 12.82 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.67 | 7 | H | H | H | H | $-CH_2-CH_2-$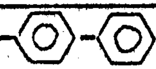 | | 17 | 12.83 |
| 23.68 | 6 | H | H | H | H | $-CH_2-CH_2-$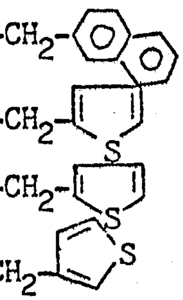 | | 17 | 12.84 |
| 23.69 | 6 | H | H | H | H | $-CH_2-CH_2-$ | | 17 | 12.85 |
| 23.70 | 7 | H | H | H | H | $-CH_2-CH_2-$ | | 17 | 12.86 |
| 23.71 | 6 | H | H | H | H | $-CH_2-CH_2-$ | | 17 | 12.87 |
| 23.72 | 7 | H | H | H | H | $-CH_2-CH_2-$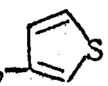 | | 17 | 12.88 |
| 23.73 | 6 | H | H | H | H | $-CH_2-CH_2-$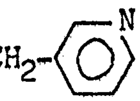 | | 17 | 12.89 |
| 23.74 | 6 | H | H | H | H | $-(CH_2)_3-$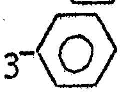 | | 17 | 12.90 |
| 23.75 | 7 | H | H | H | H | $-(CH_2)_3-$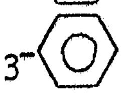 | | 17 | 12.91 |
| 23.76 | 6 | H | H | H | H | $-(CH_2)_3-$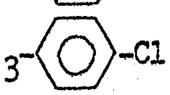-Cl | 164-166 | 17 | 12.92, 12.93 |
| 23.77 | 7 | H | H | H | H | $-(CH_2)_3-$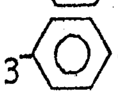-Cl | | 17 | 12.94 |
| 23.78 | 6 | H | H | H | H | $-(CH_2)_3-$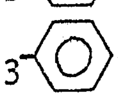-F | | 17 | 12.95 |
| 23.79 | 7 | H | H | H | H | $-(CH_2)_3-$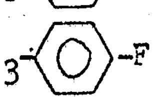-F | | 17 | 12.96 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.80 | 6 | H | H | H | H | 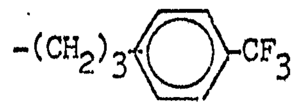 | | 17 | 12.97 |
| 23.81 | 7 | H | H | H | H | 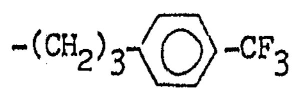 | | 17 | 12.098 |
| 23.82 | 7 | H | H | H | H | 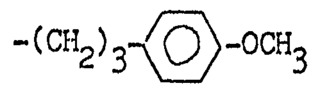 | | 22 | 12.100 |
| 23.83 | 6 | H | H | H | H | 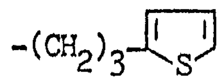 | | 17 | 12.101 |
| 23.84 | 7 | H | H | H | H | 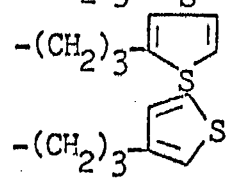 | | 17 | 12.102 |
| 23.85 | 6 | H | H | H | H | | | 17 | 12.103 |
| 23.86 | 7 | H | H | H | H | 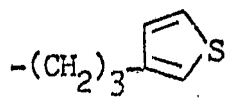 | | 17 | 12.104 |
| 23.87 | 6 | H | H | H | H | 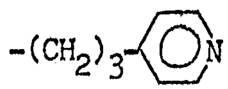 | | 17 | 12.105 |
| 23.88 | 6 | H | H | H | H | 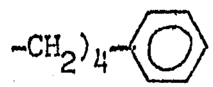 | | 17 | 12.106 |
| 23.89 | 7 | H | H | H | H | 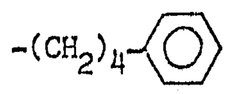 | | 17 | 12.107 |
| 23.90 | 6 | H | H | H | H | 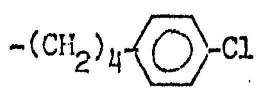 | 164-166 | 17 | 12.108 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.91 | 7 | H | H | H | H | 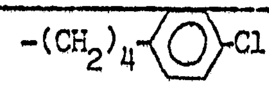 | | 17 | 12.109 |
| 23.92 | 6 | H | H | H | H | 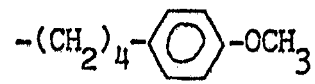 | | 22 | 12.110 |
| 23.93 | 7 | H | H | H | H | 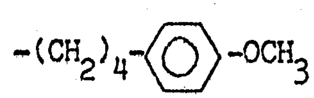 | | 22 | 12.111 |
| 23.94 | 6 | H | H | H | H | 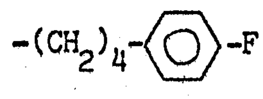 | | 17 | 12.112 |
| 23.95 | 7 | H | H | H | H | 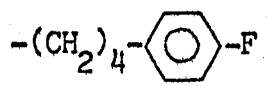 | | 17 | 12.113 |
| 23.96 | 6 | H | H | H | H | 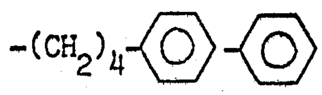 | | 17 | 12.114 |
| 23.97 | 7 | H | H | H | H | 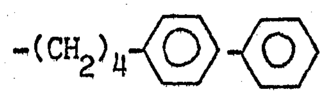 | | 17 | 12.115 |
| 23.98 | 6 | H | H | H | H | 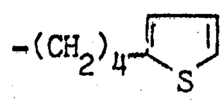 | | 17 | 12.116 |
| 23.99 | 7 | H | H | H | H | 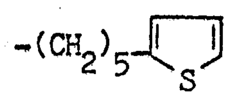 | | 17 | 12.117 |
| 23.100 | 6 | H | H | H | H | 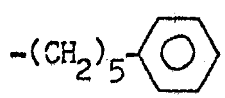 | | 17 | 12.118 |
| 23.101 | 7 | H | H | H | H | 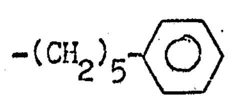 | | 17 | 12.119 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.102 | 6 | H | H | H | H | $-(CH_2)_5-$ 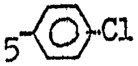 | | 17 | 12.120 |
| 23.103 | 7 | H | H | H | H | $-(CH_2)_5-$ 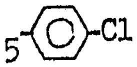 | | 17 | 12.121 |
| 23.104 | 6 | H | H | H | H | $-(CH_2)_5-$ 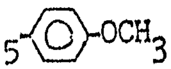 | | 22 | 12.122 |
| 23.105 | 7 | H | H | H | H | $-(CH_2)_5-$ 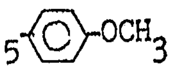 | | 22 | 12.123 |
| 23.106 | 6 | H | H | H | H | $-(CH_2)_5-$ 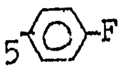 | | 17 | 12.124 |
| 23.107 | 7 | H | H | H | H | $-(CH_2)_5-$ 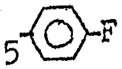 | | 17 | 12.125 |
| 23.108 | 6 | H | H | H | H | $-(CH_2)_5-$ 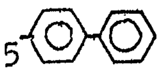 | | 17 | 12.126 |
| 23.109 | 7 | H | H | H | H | $-(CH_2)_5-$ 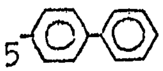 | | 17 | 12.127 |
| 23.110 | 6 | H | H | H | H | $-(CH_2)_5-$ 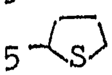 | | 17 | 12.128 |
| 23.111 | 7 | H | H | H | H | $-(CH_2)_5-$ 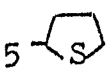 | | 17 | 12.129 |
| 23.112 | 7 | H | H | H | H | $-COCH_3$ | | 21 | 12.132 |
| 23.113 | 6 | H | H | H | H | -CO- 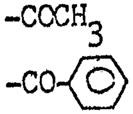 | | 13 | 12.133 |
| 23.114 | 7 | H | H | H | H | -CO- 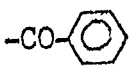 | | 13 | 12.134 |
| 23.115 | 7 | H | H | H | H | -CO- 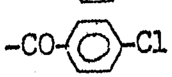 | | 13 | 12.136 |
| 23.116 | 6 | H | H | H | H | -CO- 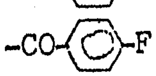 | | 13 | 12.137 |
| 23.117 | 7 | H | H | H | H | -CO- 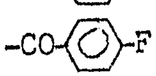 | | 13 | 12.138 |

| Verb. Nr. | V | $R^1$ | $R^2$ | $R^3$ | $R^4$ | $R^5$ | Fp. /°C/ | Herstellung analog Beispiel | Ausgangsprodukt für Beispiel bzw. Verb. Nr. in Tab. 1 |
|---|---|---|---|---|---|---|---|---|---|
| 23.118 | 6 | H | H | H | H | 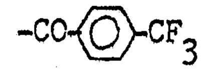 | | 13 | 12.139 |
| 23.119 | 7 | H | H | H | H | 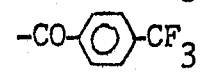 | | 13 | 12.140 |
| 23.120 | 6 | H | H | H | H | 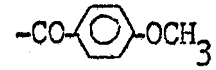 | | 13 | 12.141 |
| 23.121 | 7 | H | H | H | H | 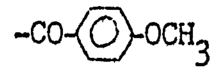 | | 13 | 12.142 |
| 23.122 | 6 | H | H | H | H | 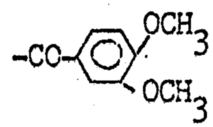 | | 13 | 12.143 |
| 23.123 | 7 | H | H | H | H | 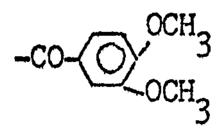 | | 13 | 12.144 |
| 23.124 | 6 | H | H | H | H | 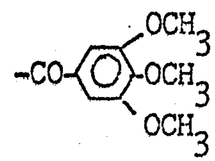 | | 13 | 12.145 |
| 23.125 | 7 | H | H | H | H | 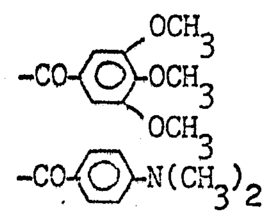 | | 13 | 12.146 |
| 23.126 | 6 | H | H | H | H | | 244-247 | 13 | 12.147 |
| 23.127 | 7 | H | H | H | H | 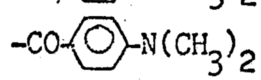 | | 13 | 12.148 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.128 | 6 | H | H | H | H | 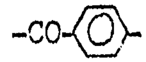 | | 13 | 12.149 |
| 23.129 | 7 | H | H | H | H | 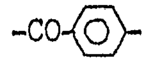 | | 13 | 12.150 |
| 23.130 | 6 | H | H | H | H | 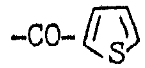 | | 13 | 12.151 |
| 23.131 | 7 | H | H | H | H | 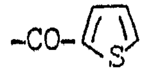 | | 13 | 12.152 |
| 23.132 | 6 | H | H | H | H | 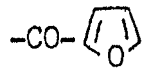 | | 13 | 12.153 |
| 23.133 | 7 | H | H | H | H | 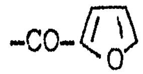 | | 13 | 12.154 |
| 23.134 | 6 | H | H | H | H | 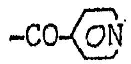 | | 13 | 12.155 |
| 23.135 | 7 | H | H | H | H | 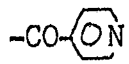 | | 13 | 12.156 |
| 23.136 | 6 | H | H | H | H | 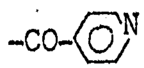 | | 13 | 12.157 |
| 23.137 | 7 | H | H | H | H | 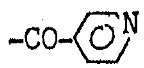 | | 13 | 12.158 |
| 23.138 | 6 | H | H | H | H | 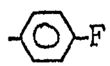 | | 20 | 12.159 |
| 23.139 | 6 | H | H | H | H | 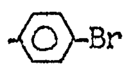 | | 20 | 12.161 |
| 23.140 | 6 | H | H | H | H | 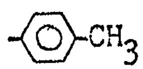 | | 20 | 12.162 |
| 23.141 | 6 | H | H | H | H | 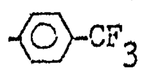 | | 20 | 12.163 |

| Verb. Nr. | V | $R^1$ | $R^2$ | $R^3$ | $R^4$ | $R^5$ | Fp. /°C/ | Herstellung analog Beispiel | Ausgangsprodukt für Beispiel bzw. Verb. Nr. in Tab. 1 |
|---|---|---|---|---|---|---|---|---|---|
| 23.142 | 6 | H | H | H | H | 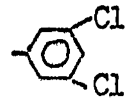 | | 20 | 12.164 |
| 23.143 | 6 | H | H | H | H | 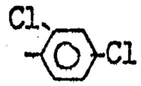 | | 20 | 12.165 |
| 23.144 | 6 | H | H | H | H | 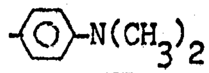 | | 20 | 12.166 |
| 23.145 | 6 | H | H | H | H | 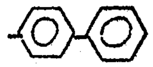 | | 20 | 12.167 |
| 23.146 | 6 | H | H | H | H | 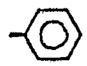 | | 20 | 12.168 |
| 23.147 | 6 | H | H | H | H | 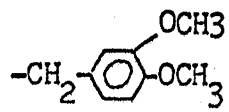 | | 15 | 12.169 |
| 23.148 | 7 | H | H | H | H | 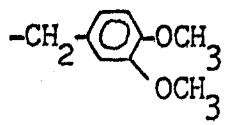 | | 15 | 12.170 |
| 23.149 | 6 | H | H | H | H | 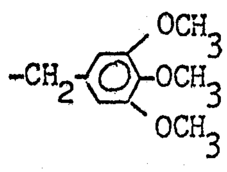 | 215-217 | 15 | 12.171 |
| 23.150 | 7 | H | H | H | H | 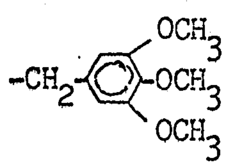 | | 15 | 12.172 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.151 | 6 | H | H | H | H | 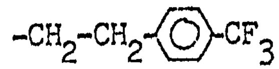 | | 17 | 12.173 |
| 23.152 | 7 | H | H | H | H | 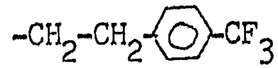 | | 17 | 12.174 |
| 23.153 | 6 | H | H | H | H | 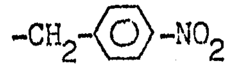 | | 14 | 12.175 |
| 23.154 | 7 | H | H | H | H | 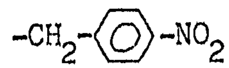 | | 14 | 12.176 |
| 23.155 | 6 | H | H | H | H | 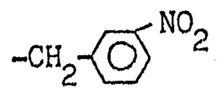 | | 14 | 12.177 |
| 23.156 | 7 | H | H | H | H | 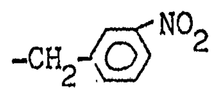 | | 14 | 12.178 |
| 23.157 | 6 | H | H | H | H | 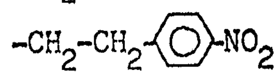 | | 16 | 12.179 |
| 23.158 | 7 | H | H | H | H | 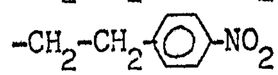 | | 16 | 12.180 |
| 23.159 | 6 | H | H | H | H | 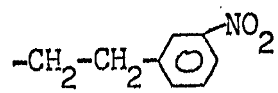 | | 16 | 12.181 |
| 23.160 | 7 | H | H | H | H | 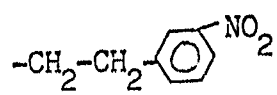 | | 16 | 12.182 |
| 23.161 | 6 | H | H | H | H | 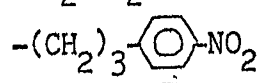 | | 16 | 12.183 |
| 23.162 | 7 | H | H | H | H | 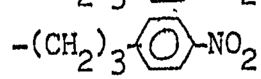 | | 16 | 12.184 |
| 23.163 | 6 | H | H | H | H | 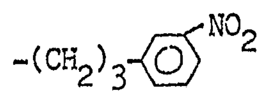 | | 16 | 12.185 |

| Verb. Nr. | V | $R^1$ | $R^2$ | $R^3$ | $R^4$ | $R^5$ | Fp. [°C] | Herstellung analog Beispiel | Ausgangsprodukt für Beispiel bzw. Verb. Nr. in Tab. 1 |
|---|---|---|---|---|---|---|---|---|---|
| 23.164 | 7 | H | H | H | H | 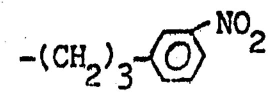 | | 16 | 12.186 |
| 23.165 | 7 | H | H | H | H | 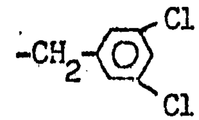 | | 14 | 12.187 |
| 23.166 | 7 | H | H | H | H | 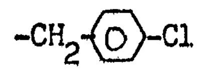 | | 14 | 12.188 |
| 23.167 | 7 | H | H | H | H | 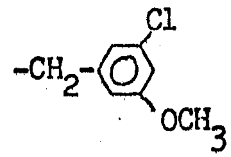 | | 14 | 12.189 |
| 23.168 | 7 | H | H | H | H | 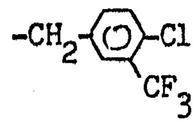 | | | 12.190 |
| 23.169 | 7 | H | H | H | H | 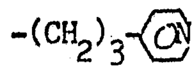 | | 17 | 12.191 |
| 23.170 | 6 | H | H | H | H | 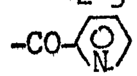 | | 13 | 12.192 |
| 23.171 | 7 | H | H | H | H | 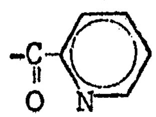 | | 13 | 12.193 |

PATENTANSPRÜCHE

1) 1-(1,3-Dioxolan-2-ylmethyl)-azole der allgemeinen Formel (I)

$$G-CH_2-O-D \qquad (I)$$

sowie ihre Steroisomeren und ihre Salze mit physiologisch verträglichen Säuren, in welcher G einen 1-(1,3-Dioxolan-2-ylmethyl)-azol-Rest der nachstehenden Struktur bedeutet,

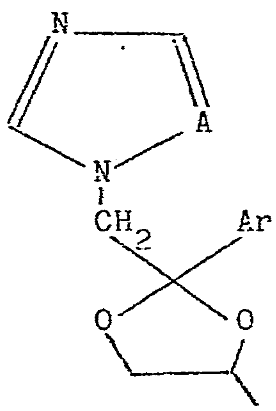

(G)

in welcher A Stickstoff oder Methin, Ar Naphtyl, Biphenyl, Thienyl oder eine gegebenenfalls einen, zwei oder drei Substituenten tragende Phenylgruppe bedeutet, wobei die Substituenten gleich oder verschieden sind und Halogen, Trifluormethyl, $C_1$-$C_4$-Alkyl oder $C_1$-$C_4$-Alkoxy bedeuten, und D in Formel (I) entweder

1.1. eine Isochinolingruppe der Formel D I bedeutet

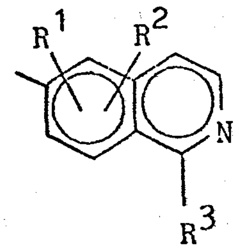

(D I)

wobei $R^1$ und $R^2$ unabhängig voneinander ein Wasserstoffatom, Halogen, $C_1$-$C_4$-Alkyl oder $C_1$-$C_4$-Alkoxy bedeuten, und $R^3$ ein Wasserstoffatom $C_1$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, Heteroaryl, $C_1$-$C_5$-Heteroaryl-alkyl oder eine gegebenenfalls eine einen, zwei oder drei Substituenten tragende Aryl- oder $C_1$-$C_5$-Aryl-alkyl-Gruppe bedeutet, wobei die Substituenten an der Arylgruppe gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten, oder

1.2. eine 3,4-Dihydro-isochinolingruppe der Formel D II bedeutet

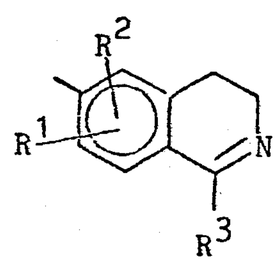

(D II)

wobei $R^1$, $R^2$ und $R^3$ die zu Formel DI angegebenen Bedeutungen haben, oder

1.3. eine 1,2,3,4-Tetrahydro-isochinolingruppe der Formel D III bedeutet

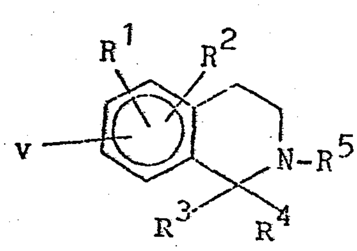

(D III)

wobei v die Position der Verknüpfung des Isochinolinsystems mit dem Rest G-$CH_2$-O angibt, $R^1$, $R^2$ und $R^3$ die zur Formel D I angegebenen Bedeutungen haben und $R^4$ ein Wasserstoffatom oder eine $C_1$-$C_4$-Alkyl-Gruppe bedeutet, sowie zusätzlich, falls $R^3$ und $R^4$ Alkylgruppen sind, diese zusammen mit dem sie tragenden C-Atom ein spirocyclisches Ringsystem der allgemeinen Formel D III' bedeuten können,

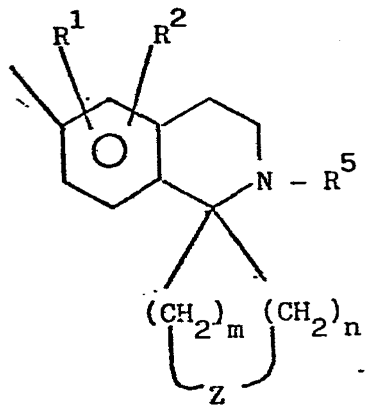

( D III')

worin m und n 1, 2 oder 3 bedeuten und Z entweder eine Methylengruppe bedeutet oder, falls m = n = 2 ist, ein Heteroatom aus der Gruppe Sauerstoff, Schwefel oder Stickstoff bedeutet, wobei der Stickstoff durch einen Rest $R^6$ substituiert sein kann, der $C_1$-$C_4$-Alkyl, $C_1$-$C_5$-Alkanoyl, $C_1$-$C_4$-Alkoxycarbonyl oder eine gegebenenfalls mit einem oder zwei Substituenten versehene Benzoyl- oder Benzylgruppe bedeutet, wobei die Substituenten am Phenylring gleich oder verschieden sein können und jeweils Halogen, $C_1$-$C_4$-Alkyl, Trifluormethyl oder $C_1$-$C_4$-Alkoxy bedeuten, und v, falls $R^3$ und/oder $R^4$ kein Wasserstoffatom ist, die 6-Stellung bedeutet oder, falls $R^3$ und $R^4$ Wasserstoffatome sind, die 5-, 6-, 7- oder 8-Stellung bedeutet, und $R^5$, falls $R^3$ und/oder $R^4$ kein Wasserstoffatom ist,

ein Wasserstoffatom oder eine $C_1$-$C_4$-Alkylgruppe bedeutet oder, falls $R^3$ und $R^4$ Wasserstoffatome sind und v die 5-, 7- oder 8-Stellung bedeutet, $R^5$ die zur Formel D I für $R^3$ angegebenen Bedeutungen haben kann, mit der Einschränkung, daß $R^5$ keine Aryl- oder Heteroaryl-Gruppe bedeutet, oder, falls $R^3$ und $R^4$ Wasserstoffatome sind und v die 6-Stellung bedeutet, $R^5$ die zur Formel D I für $R^3$ angegebenen Bedeutungen hat, und $R^5$ weiterhin eine Acylgruppe der allgemeinen Formel Q bedeuten kann,

$$-\underset{\underset{O}{\|}}{C}-R^7 \qquad (Q)$$

wobei $R^7$ die zur Formel D I für $R^3$ angegebenen Bedeutungen haben kann.

2. Verfahren zur Herstellung der Verbindungen der allgemeinen Formel (I) in Anspruch 1, dadurch gekennzeichnet, daß man

a) eine Verbindung der allgemeinen Formel (II)

$$G-CH_2-E \qquad (II)$$

in der E Halogen oder einen reaktiven Esterrest bedeutet, mit einer Verbindung der allgemeinen Formel (III)

$$H-O-D \qquad (III)$$

umsetzt, in der D die in Anspruch 1 angegebene Bedeutung hat, und gegebenenfalls eine so erhaltene Verbindung der Formel (I) acyliert, alkyliert oder reduziert, oder

b) eine Verbindung der allgemeinen Formel (IV)

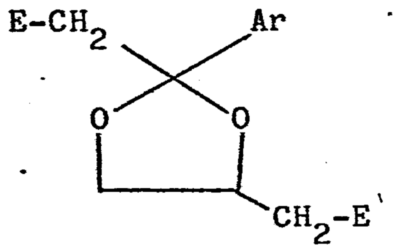

(IV)

in der Ar die in Anspruch 1 zu Formel I und E die zur Formel (II) angegebenen Bedeutungen haben, zunächst mit einer Verbindung der allgemeinen Formel III umsetzt und hierbei eine Verbindung der allgemeinen Formel (V) herstellt,

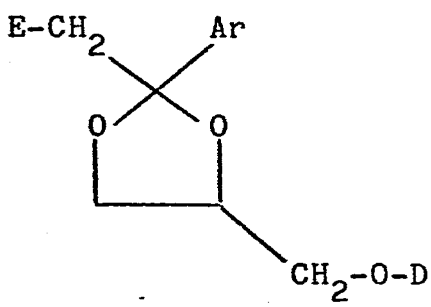

(V)

in der Ar und D die in Anspruch 1 und E die zur Formel (II) in Anspruch 2 angegebenen Bedeutungen haben, und anschließend die so erhaltene Verbindung der allgemeinen Formel (V) mit einer Verbindung der allgemeinen Formel (VI) umsetzt,

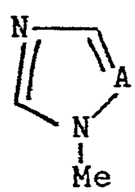

(VI)

in der A die in Anspruch 1 angegebenen Bedeutungen hat und Me Wasserstoff oder ein Metallatom bedeutet, und gegebenenfalls eine so erhaltene Verbindung der Formel (I) acyliert, alkyliert oder reduziert und gegebenenfalls mit einer physiologisch verträglichen Säure in das Salz überführt.

3. Pharmazeutische Zubereitung, dadurch gekennzeichnet, daß sie eine Verbindung gemäß Anspruch 1 enthält oder aus ihr besteht.

4. Verfahren zur Herstellung einer pharmazeutischen Zubereitung nach Anspruch 3, dadurch gekennzeichnet, daß man eine Verbindung der Formel (I) oder eines ihrer Salze mit einer physiologisch verträglichen Säure, gegebenenfalls zusammen mit einem pharmazeutischen Trägerstoff und/oder Hilfsstoff in eine für pharmazeutische Zwecke geeignete Darreichungsform bringt.

5. Verwendung einer Verbindung gemäß Anspruch 1 zur Bekämpfung von Mykosen, Protozoen und grampositiver und gramnegativer Bakterien.

6. Verbindungen der allgemeinen Formel (III')

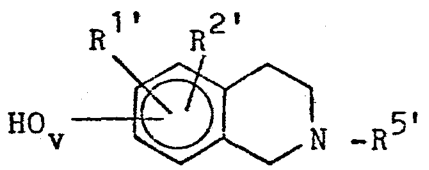

(III')

wobei v die Position der Hydroxygruppe am Isochinolinsystem angibt und die 5-, 6-, 7- oder 8-Position bedeutet, worin
$R^{1'}$ und $R^{2'}$ unabhängig voneinander ein Wasserstoffatom, Halogen oder $C_1$-$C_4$-Alkyl bedeuten,
und
$R^{5'}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl, $C_1$-$C_5$-Arylalkyl oder falls v die 6-Position bedeutet, zusätzlich Aryl oder Heteroaryl bedeutet, wobei bei den Aryl- oder $C_1$-$C_5$-Arylalkyl-Gruppen die Arylgruppen einen, zwei oder drei Substituenten tragen können und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio,

$C_1$-$C_4$-Dialkylamino, Phenylthio, Halogenphenylthio, Phenoxy oder Halogenphenoxy bedeuten,
und worin
$R^{5'}$ weiterhin eine Acylgruppe der allgemeinen Formel Q' bedeutet

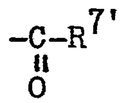 (Q')

worin $R^{7'}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl, $C_1$-$C_5$-Arylalkyl, Aryl oder Heteroaryl bedeutet, wobei bei den Aryl- oder $C_1$-$C_5$-Arylalkyl-Gruppen die Arylgruppen einen, zwei oder drei Substituenten tragen können und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten.

7. Verfahren zur Herstellung von Verbindungen der allgemeinen Formel (III'a)

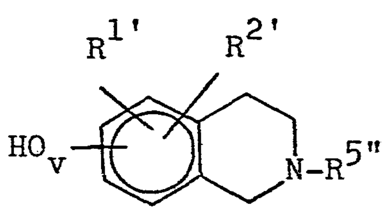 (III'a)

für die $R^{1'}$, $R^{2'}$ und v die in Anspruch 6 angegebenen Bedeutungen haben und $R^{5''}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl oder $C_1$-$C_5$-Arylalkyl bedeutet, wobei die Arylgruppe einen, zwei oder drei Substituenten tragen kann und diese Substituenten gleich oder verschieden sind und jeweils Halogen, Nitro, $C_1$-$C_4$-Alkyl, Trifluormethyl, $C_1$-$C_4$-Alkoxy,

$C_1$-$C_4$-Alkylthio, $C_1$-$C_4$-Dialkylamino, Phenylthio, Halogenphenylthio, Phenoxy oder Halogenphenoxy bedeuten, dadurch gekennzeichnet, daß man eine Verbindung der allgemeinen Formel (III'g)

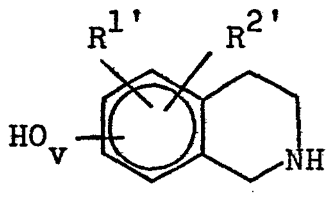

(III'g)

oder eine Verbindung der allgemeinen Formel (X)

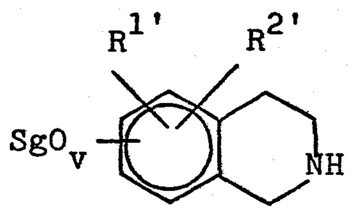

(X)

in denen v, $R^{1'}$ und $R^{2'}$ die oben angegebenen Bedeutungen haben und Sg eine Schutzgruppe wie eine $C_1$-$C_4$-Alkylgruppe, eine Benzylgruppe oder eine Gruppe der allgemeinen Formel $R^7-\underset{\underset{O}{\|}}{C}-$ bedeutet,

wobei $R^7$ die in Anspruch 1.3 angegebenen Bedeutungen hat, mit einem Alkylierungsmittel Alk'E umsetzt, wobei Alk' $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroarylalkyl oder $C_1$-$C_5$Arylalkyl bedeutet, wobei die Arylgruppe einen, zwei oder drei Substituenten tragen kann und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten,

und E eine reaktive Abgangsgruppe mit den in Anspruch 2 angegebenen Bedeutungen ist, und bei den aus der Ver-

bindung der Formel (X) erhaltenen Verbindungen die Schutzgruppe Sg abspaltet.

8. Verfahren zur Herstellung von Verbindungen der allgemeinen Formel (III'b)

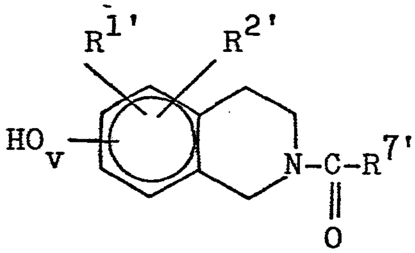

(III'b)

worin $R^{1'}$, $R^{2'}$, v und $R^{7'}$ die in Anspruch 6 angegebenen Bedeutungen haben, dadurch gekennzeichnet, daß man eine Verbindung der allgemeinen Formel (III'g) oder (X) aus Anspruch 7 mit einem Acylierungsmittel $R^{7'}-\overset{O}{\overset{\|}{C}}-X$ umsetzt, wobei $R^{7'}$ die in Anspruch 6 für $R^{7'}$ angegebenen Bedeutungen hat und X eine reaktive Abgangsgruppe wie Halogen oder einen reaktiven Esterrest bedeutet,

und bei den aus der Verbindung der allgemeinen Formel (X) erhaltenen Verbindungen der allgemeinen Formel (X")

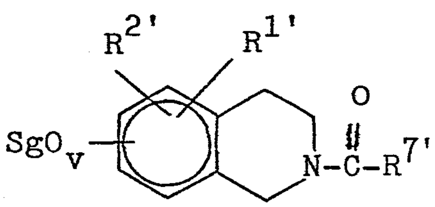

(X")

worin $R^{1'}$, $R^{2'}$, $R^{7'}$, Sg und v die in Anspruch 6 angegebenen Bedeutungen haben, die Schutzgruppe Sg abspaltet.

9. Verfahren zur Herstellung von Verbindungen der allgemeinen Formel (III'c)

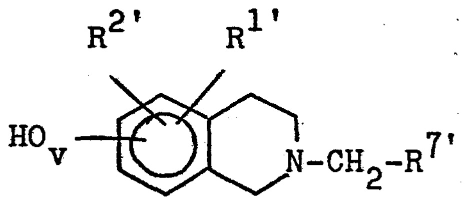

(III'c)

worin $R^{1'}$, $R^{2'}$, $R^{7'}$ und v die in Anspruch 6 angegebenen Bedeutungen haben, dadurch gekennzeichnet, daß man

a) eine Verbindung der allgemeinen Formel (III'b) aus Anspruch 8 oder eine Verbindung der allgemeinen Formel (X") aus Anspruch 8 mit einem komplexen Metallhydrid reduziert, und bei den aus der Verbindung (X") erhaltenen Verbindungen die Schutzgruppe Sg abspaltet, oder

b) eine Verbindung der allgemeinen Formel (III'a) oder (X) aus Anspruch 7 mit einem Aldehyd der allgemeinen Formel $R^{7'}$-CHO unter den Bedingungen einer reduktiven Aminierung umsetzt, wobei $R^{7'}$ die in Anspruch 6 angegebenen Bedeutungen hat, und bei den aus den aus der Verbindung (X) erhaltenen Verbindungen die Schutzgruppe Sg abspaltet, oder

c) eine Verbindung der allgemeinen Formel (III'd)

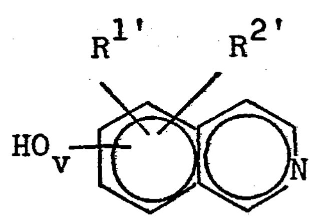

(III'd)

in der v, $R^{1'}$ und $R^{2'}$ die in Anspruch 6 angegebenen Bedeutungen haben, mit einem Alkylierungsmittel Alk'E umsetzt, wobei Alk'-E die in Anspruch 7 angegebenen Bedeutungen hat, und die so erhaltene Verbindung der allgemeinen Formel (XI)

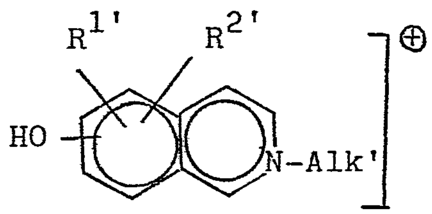

$E^{\ominus}$ (XI)

in der v, $R^{1'}$ und $R^{2'}$ die in Anspruch 6 angegebenen Bedeutungen haben, mit einem komplexen Hydrid reduziert.

10. Verfahren zur Herstellung von Verbindungen der allgemeinen Formel (III'e)

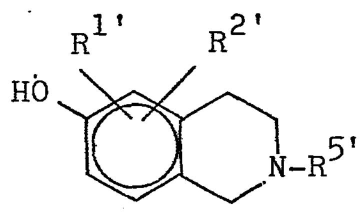

(III'e)

für die $R^{1'}$, $R^{2'}$ und $R^{5'}$ die in Anspruch 6 angegebenen Bedeutungen haben, dadurch gekennzeichnet, daß man eine Verbindung der allgemeinen Formel (VII )

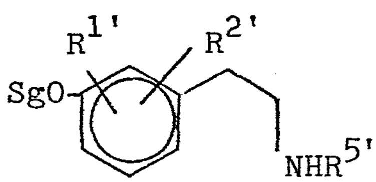

(VII )

in der $R^{1'}$, $R^{2'}$ $R^{5'}$ und Sg die in Anspruch 6 angegebenen Bedeutungen haben, mit Formaldehyd in Gegenwart einer Säure umsetzt und anschließend die Gruppe Sg abspaltet.

Patentansprüche für Österreich:

1. Verfahren zur Herstellung von 1-(1,3-Dioxolan-2-ylmethyl)-azolen der allgemeinen Formel (I)

$$G-CH_2-O-D \qquad (I)$$

sowie ihrer Stereoisomeren und ihrer Salze mit physiologisch verträglichen Säuren, in welcher G einen 1-(1,3-Dioxolan-2-ylmethyl)-azol-Rest der nachstehenden Struktur bedeutet,

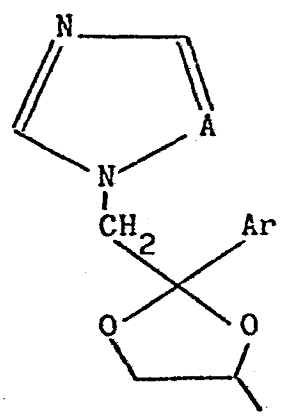

(G)

in welcher A Stickstoff oder Methin, Ar Naphtyl, Biphenyl, Thienyl oder eine gegebenenfalls einen, zwei oder drei Substituenten tragende Phenylgruppe bedeutet, wobei die Substituenten gleich oder verschieden sind und Halogen, Trifluormethyl, $C_1$-$C_4$-Alkyl oder $C_1$-$C_4$-Alkoxy bedeuten, und D in Formel (I) entweder

1.1. eine Isochinolingruppe der Formel D I bedeutet

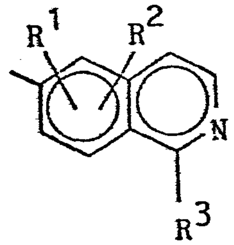

(D I)

wobei $R^1$ und $R^2$ unabhängig voneinander ein Wasserstoffatom, Halogen, $C_1$-$C_4$-Alkyl oder $C_1$-$C_4$-Alkoxy bedeuten, und $R^3$ ein Wasserstoffatom $C_1$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, Heteroaryl, $C_1$-$C_5$-Heteroaryl-alkyl oder eine gegebenenfalls eine einen, zwei oder drei Substituenten tragende Aryl- oder $C_1$-$C_5$-Aryl-alkyl-Gruppe bedeutet, wobei die Substituenten an der Arylgruppe gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten, oder

1.2. eine 3,4-Dihydro-isochinolingruppe der Formel D II bedeutet

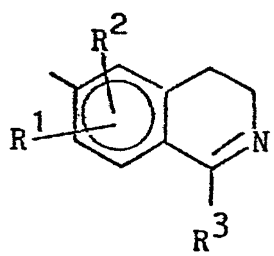

(D II)

wobei $R^1$, $R^2$ und $R^3$ die zu Formel DI angegebenen Bedeutungen haben, oder

1.3. eine 1,2,3,4-Tetrahydro-isochinolingruppe der Formel D III bedeutet

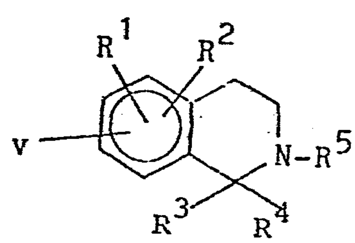

(D III)

wobei v die Position der Verknüpfung des Isochinolinsystems mit dem Rest G-$CH_2$-O angibt,
$R^1$, $R^2$ und $R^3$ die zur Formel D I angegebenen Bedeutungen haben und
$R^4$ ein Wasserstoffatom oder eine $C_1$-$C_4$-Alkyl-Gruppe bedeutet, sowie zusätzlich,
falls $R^3$ und $R^4$ Alkylgruppen sind, diese zusammen mit dem sie tragenden C-Atom ein spirocyclisches Ringsystem der allgemeinen Formel D III' bedeuten können,

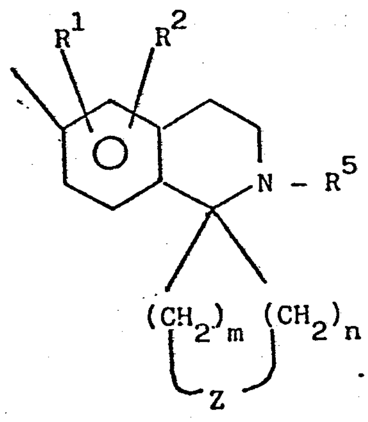

( D III')

worin m und n 1, 2 oder 3 bedeuten und
Z entweder eine Methylengruppe bedeutet oder, falls m = n = 2 ist, ein Heteroatom aus der Gruppe Sauerstoff, Schwefel oder Stickstoff bedeutet,
wobei der Stickstoff durch einen Rest $R^6$ substituiert sein kann, der $C_1$-$C_4$-Alkyl, $C_1$-$C_5$-Alkanoyl, $C_1$-$C_4$-Alkoxycarbonyl oder eine gegebenenfalls mit einem oder zwei Substituenten versehene Benzoyl- oder Benzylgruppe bedeutet, wobei die Substituenten am Phenylring gleich oder verschieden sein können und jeweils Halogen, $C_1$-$C_4$-Alkyl, Trifluormethyl oder $C_1$-$C_4$-Alkoxy bedeuten, und
v, falls $R^3$ und/oder $R^4$ kein Wasserstoffatom ist, die 6-Stellung bedeutet oder, falls $R^3$ und $R^4$ Wasserstoffatome sind, die 5-, 6-, 7- oder 8-Stellung bedeutet, und
$R^5$, falls $R^3$ und/oder $R^4$ kein Wasserstoffatom ist,

ein Wasserstoffatom oder eine $C_1$-$C_4$-Alkylgruppe bedeutet oder, falls $R^3$ und $R^4$ Wasserstoffatome sind und v die 5-, 7- oder 8-Stellung bedeutet, $R^5$ die zur Formel D I für $R^3$ angegebenen Bedeutungen haben kann, mit der Einschränkung, daß $R^5$ keine Aryl- oder Heteroaryl-Gruppe bedeutet, oder, falls $R^3$ und $R^4$ Wasserstoffatome sind und v die 6-Stellung bedeutet, $R^5$ die zur Formel D I für $R^3$ angegebenen Bedeutungen hat, und $R^5$ weiterhin eine Acylgruppe der allgemeinen Formel Q bedeuten kann,

$$-\underset{\underset{O}{\|}}{C}-R^7 \qquad (Q)$$

wobei $R^7$ die zur Formel D I für $R^3$ angegebenen Bedeutungen haben kann, dadurch gekennzeichnet, daß man

a) eine Verbindung der allgemeinen Formel (II)

$$G-CH_2-E \qquad (II)$$

in der E Halogen oder einen reaktiven Esterrest bedeutet, mit einer Verbindung der allgemeinen Formel (III)

$$H-O-D \qquad (III)$$

umsetzt, in der D die vorstehend angegebene Bedeutung hat, und gegebenenfalls eine so erhaltene Verbindung der Formel (I) acyliert, alkyliert oder reduziert, oder

b) eine Verbindung der allgemeinen Formel (IV)

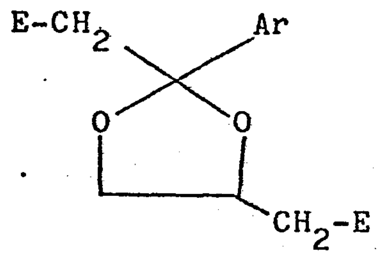

(IV)

in der Ar die vorstehend zu Formel I und E die zur Formel (II) angegebenen Bedeutungen haben, zunächst mit einer Verbindung der allgemeinen Formel III umsetzt und hierbei eine Verbindung der allgemeinen Formel (V) herstellt,

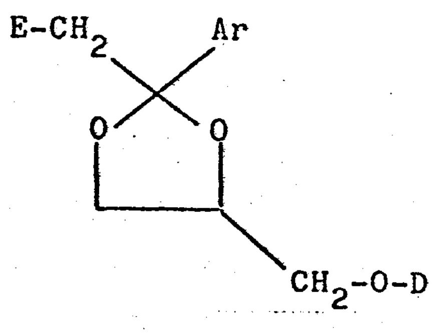

(V)

in der Ar, D und E die vorstehend angegebenen Bedeutungen haben, und anschließend die so erhaltende Verbindung der allgemeinen Formel (V) mit einer Verbindung der allgemeinen Formel (VI) umsetzt,

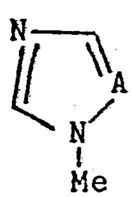

(VI)

in der A die vorstehend angegebenen Bedeutungen hat und Me Wasserstoff oder ein Metallatom bedeutet, und gegebenenfalls eine so erhaltene Verbindung der Formel (I) acyliert, alkyliert oder reduziert und gegebenenfalls mit einer physiologisch verträglichen Säure in das Salz überführt.

2. Verfahren zur Herstellung von Verbindungen der allgemeinen Formel (III'),

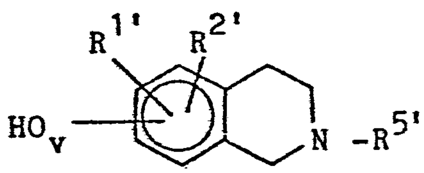

(III')

wobei v die Position der Hydroxygruppe am Isochinolinsystem angibt und die 5-, 6-, 7- oder 8-Position bedeutet, worin

$R^{1'}$ und $R^{2'}$ unabhängig voneinander ein Wasserstoffatom, Halogen oder $C_1$-$C_4$-Alkyl bedeuten,

und

$R^{5'}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl, $C_1$-$C_5$-Arylalkyl oder falls v die 6-Position bedeutet, zusätzlich Aryl oder Heteroaryl bedeutet, wobei bei den Aryl- oder $C_1$-$C_5$-Arylalkyl-Gruppen die Arylgruppen einen, zwei oder drei Substituenten tragen können und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio,

$C_1$-$C_4$-Dialkylamino, Phenylthio, Halogenphenylthio, Phenoxy oder Halogenphenoxy bedeuten,

und worin

$R^{5'}$ weiterhin eine Acylgruppe der allgemeinen Formel Q' bedeutet

$$-\underset{\underset{O}{\|}}{C}-R^{7'} \quad (Q')$$

worin $R^{7'}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl, $C_1$-$C_5$-Arylalkyl, Aryl oder Heteroaryl bedeutet, wobei bei den Aryl- oder $C_1$-$C_5$-Arylalkyl-Gruppen die Arylgruppen einen, zwei oder drei Substituenten tragen können und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten, dadurch gekennzeichnet, daß man entweder zur Herstellung von Verbindungen der allgemeinen Formel (III'a)

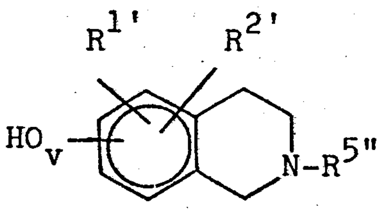

(III'a)

für die $R^{1'}$, $R^{2'}$ und v die zu Formel III' angegebenen Bedeutungen haben und $R^{5''}$ $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroaryl-alkyl oder $C_1$-$C_5$-Arylalkyl bedeutet, wobei die Arylgruppe einen, zwei oder drei Substituenten tragen kann und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten, eine Verbindung der allgemeinen Formel (III'g)

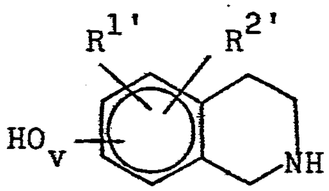

(III'g)

oder eine Verbindung der allgemeinen Formel (X)

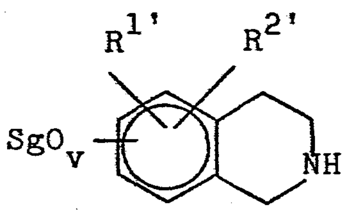

(X)

in denen v, $R^{1'}$ und $R^{2'}$ die oben angegebenen Bedeutungen haben und Sg eine Schutzgruppe wie eine $C_1$-$C_4$-Alkylgruppe, eine Benzylgruppe oder eine Gruppe der allgemeinen Formel $R^7-\underset{\underset{O}{\|}}{C}-$ bedeutet,

wobei $R^7$ die in Anspruch 1.3 angegebenen Bedeutungen hat, mit einem Alkylierungsmittel Alk'E umsetzt, wobei Alk' $C_5$-$C_{12}$-Alkyl, $C_3$-$C_8$-Cycloalkyl, $C_3$-$C_5$-Alkenyl, $C_3$-$C_5$-Alkinyl, Trifluormethyl, Trichlormethyl, $C_1$-$C_5$-Heteroarylalkyl oder $C_1$-$C_5$Arylalkyl bedeutet, wobei die Arylgruppe einen, zwei oder drei Substituenten tragen kann und diese Substituenten gleich oder verschieden sind und jeweils Halogen, Nitro, $C_1$-$C_4$-Alkyl, Trifluormethyl, $C_1$-$C_4$-Alkoxy, $C_1$-$C_4$-Alkylthio, $C_1$-$C_4$-Dialkylamino, Phenylthio, Halogenphenylthio, Phenoxy oder Halogenphenoxy bedeuten,

und E eine reaktive Abgangsgruppe mit den in Anspruch 1 angegebenen Bedeutungen ist, und bei den aus der Verbindung der Formel (X) erhaltenen Verbindungen die Schutzgruppe Sg abspaltet, oder daß man zur Herstellung von Verbindungen der allgemeinen Formel (III'b)

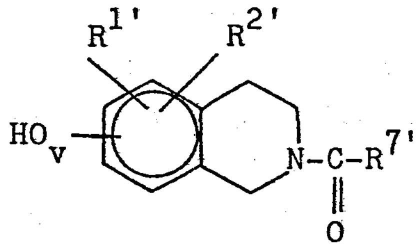

(III'b)

worin $R^{1'}$, $R^{2'}$, v und $R^{7'}$ die vorstehend angegebenen Bedeutungen haben, eine Verbindung der allgemeinen Formel (III'g) oder (X) aus Anspruch 7 mit einem Acylierungsmittel $R^{7'}-\overset{O}{\overset{\|}{C}}-X$ umsetzt, wobei $R^{7'}$ die in Anspruch 6 für $R^{7'}$ angegebenen Bedeutungen hat und X eine reaktive Abgangsgruppe wie Halogen oder einen reaktiven Esterrest bedeutet,

und bei den aus der Verbindung der allgemeinen Formel (X) erhaltenen Verbindungen der allgemeinen Formel (X")

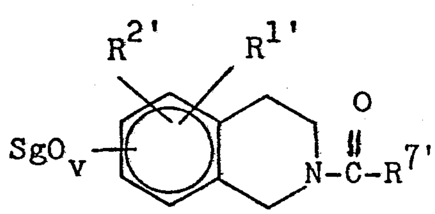

(X")

worin $R^{1'}$, $R^{2'}$, $R^{7'}$, Sg und v die in Anspruch 6 angegebenen Bedeutungen haben, die Schutzgruppe Sg abspaltet, oder daß man zur Herstellung von Verbindungen der allgemeinen Formel (III'c)

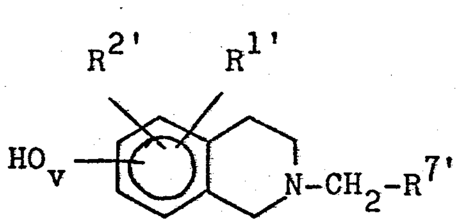

(III'c)

worin $R^{1'}$, $R^{2'}$, $R^{7'}$ und v die vorstehend angegebenen Bedeutungen haben,

a) eine Verbindung der allgemeinen Formel (III'b) oder eine Verbindung der allgemeinen Formel (X") wie vorstehend genannt mit einem komplexen Metallhydrid reduziert, und bei den aus der Verbindung (X") erhaltenen Verbindungen die Schutzgruppe Sg abspaltet, oder

b) eine Verbindung der allgemeinen Formel (III'g) oder (X) wie vorstehend genannt mit einem Aldehyd der allgemeinen Formel $R^{7'}$-CHO unter den Bedingungen einer reduktiven Aminierung umsetzt, wobei $R^{7'}$ die vorstehend angegebenen Bedeutungen hat, und bei den aus der Verbindung (X) erhaltenen Verbindungen die Schutzgruppe Sg abspaltet, oder daß man

c) eine Verbindung der allgemeinen Formel (III'd)

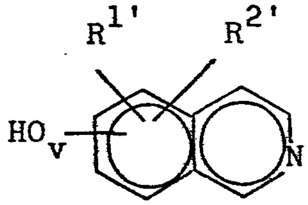

(III'd)

in der v, $R^{1'}$ und $R^{2'}$ die vorstehend angegebenen Bedeutungen haben, mit einem Alkylierungsmittel Alk'E umsetzt, wobei Alk'-E die vorstehend angegebenen Bedeutungen hat,
und die so erhaltene Verbindung der allgemeinen Formel (XI)

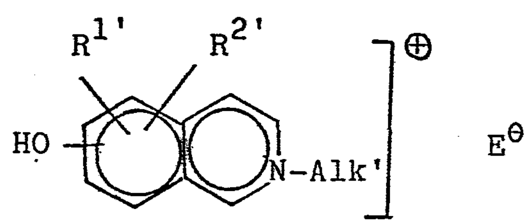

(XI)

in der v, $R^{1'}$ und $R^{2'}$ die vorstehend angegebenen Bedeutungen haben, mit einem komplexen Hydrid reduziert, oder daß man zur Herstellung von Verbindungen der allgemeinen Formel (III'e)

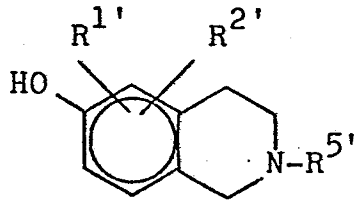

(III'e)

für die $R^{1'}$, $R^{2'}$ und $R^{5'}$ die vorstehend angegebenen Bedeutungen haben,
eine Verbindung der allgemeinen Formel (VII )

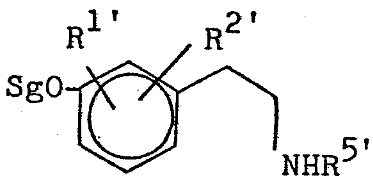

(VII )

in der $R^{1'}$, $R^{2'}$ $R^{5'}$ und Sg die vorstehend angegebenen Bedeutungen haben, mit Formaldehyd in Gegenwart einer Säure umsetzt und anschließend die Gruppe Sg abspaltet.